# EUROPEAN PATENT SPECIFICATION

(11) **EP 0 983 054 B1**
(45) Date of publication and mention of the grant of the patent: **15.09.2004**
(21) Application number: 98925148.3
(22) Date of filing: 03.06.1998
(51) Int. Cl.: A61K 7/48

(54) **HAIR GROWTH COMPOSITIONS AND USES**
HAARWUCHSZUSAMMENSETZUNGEN UND DEREN VERWENDUNGEN
COMPOSITIONS POUR LA REPOUSSE DES CHEVEUX ET LEURS UTILISATIONS

(30) Priority: 04.06.1997 US 869426
(43) Date of publication of application: 08.03.2000
(73) Proprietor: GPI NIL Holdings, Inc., Wilmington, DE 19899 (US)
(72) Inventor: HAMILTON, Gregory, S., Catonsville, MD 21228 (US); STEINER, Joseph, P., Hampstead, MD 21074 (US)
(74) Representative: Bausch, Thorsten
(86) International application number: PCT/US1998/011237
(87) International publication number: WO 1998/055090

(56) References cited:
- WO-A-95/02684
- WO-A-97/31898
- US-A- 5 614 547

## Description

### BACKGROUND OF THE INVENTION

### 1. Field of Invention

This invention relates to pharmaceutical compositions methods for treating alopecia and promoting hair growth using non-immunosuppressive neuroimmunophilin FKBP ligands.

### 2. Description of Related Art

Hair loss occurs in a variety of situations. These situations include male pattern alopecia, alopecia senilis, alopecia areata, diseases accompanied by basic skin lesions or tumors, and systematic disorders such as nutritional disorders and internal secretion disorders. The mechanisms causing hair loss are very complicated, but in some instances can be attributed to aging, genetic disposition, the activation of male hormones, the loss of blood supply to hair follicles, and scalp abnormalities.

The immunosuppressant drugs FK506, rapamycin and cyclosporin are well known as potent T-cell specific immunosuppressants, and are effective against graft rejection after organ transplantation. It has been reported that topical, but not oral, application of FK506 (Yamamoto et al., J. Invest. Dermatol., 1994, 102, 160-164; Jiang et al., J. Invest. Dermatol. 1995, 104, 523-525) and cyclosporin (Iwabuchi et al., J. Dermatol. Sci. 1995, 9, 64-69) stimulates hair growth in a dose-dependent manner. One form of hair loss, alopecia areata, is known to be associated with autoimmune activities; hence, topically administered immunomodulatory compounds are expected to demonstrate efficacy for treating that type of hair loss. The hair growth stimulating effects of FK506 have been the subject of an international patent filing covering FK506 and structures related thereto for hair growth stimulation (Honbo et al., EP 0 423 714 A2). Honbo et al. discloses the use of relatively large tricyclic compounds, known for their immunosuppressive effects, as hair revitalizing agents.

The hair growth and revitalization effects of FK506 and related agents are disclosed in many U.S. patents (Goulet et al., U.S. Patent No. 5,258,389; Luly et al., U.S. Patent No. 5,457,111; Goulet et al., U.S. Patent No. 5,532,248; Goulet et al., U.S. Patent No. 5,189,042; and Ok et al., U.S. Patent No. 5,208,241; Rupprecht et al., U.S. Patent No. 5,284,840; Organ et al., U.S. Patent No. 5,284,877). These patents claim FK506 related compounds. Although they do not claim methods of hair revitalization, they disclose the known use of FK506 for effecting hair growth. Similar to FK506 (and the claimed variations in the Honbo et al. patent), the compounds claimed in these patents are relatively large. Further, the cited patents relate to immunomodulatory compounds for use in autoimmune related diseases, for which FK506's efficacy is well known.

Other U.S. patents disclose the use of cyclosporin and related compounds for hair revitalization (Hauer et al., U.S. Patent No. 5,342,625; Eberle, U.S. Patent No. 5,284,826; Hewitt et al., U.S. Patent No. 4,996,193). These patents also relate to compounds useful for treating autoimmune diseases and cite the known use of cyclosporin and related immunosuppressive compounds for hair growth.

However, immunosuppressive compounds by definition suppress the immune system and also exhibit other toxic side effects. Accordingly, there is a need for non-immunosuppressant, small molecule compounds which are useful as hair revitalizing compounds.

Hamilton and Steiner disclose in U.S. Patent No. 5,614,547 novel pyrrolidine carboxylate compounds which bind to the immunophilin FKBP12 and stimulate nerve growth, but which lack immunosuppressive effects. Unexpectedly, it has been discovered that these non-immunosuppressant compounds promote hair growth with an efficacy similar to FK506. Yet their novel small molecule structure and non-immunosuppressive properties differentiate them from FK506 and related immunosuppressive compounds found in the prior art.

### SUMMARY OF THE INVENTION

The present invention relates to a method for treating alopecia or promoting hair growth in an animal, which comprises administering to said animal an effective amount of a non-immunosuppressive neuroimmunophilin FKBP ligand.

As its name suggests, the non-immunosuppressive neuroimmunophilin FKBP ligand used in the inventive method and pharmaceutical composition has an affinity for FKBP-type immunophilins and does not exert any significant immunosuppressive activity. In a preferred embodiment, the non-immunosuppressive neuroimmunophilin FKBP ligand is selected from the group consisting of: a heterocyclic thioester or ketone; a heterocyclic ester or amide; an N-oxide of a heterocyclic ester, amide, thioester, or ketone; an N-linked urea or carbamate of a heterocyclic thioester; an N-linked sulfonamide of a heterocyclic thioester; and a pyrrolidine derivative.

### BRIEF DESCRIPTION OF THE DRAWINGS

FIG. 1 is a photograph of C57 Black 6 mice before being shaved for the experiment. FIG. 1 shows the condition of the mice prior to the experiment.
FIG. 2 is a photograph of mice treated with a vehicle after six weeks. FIG. 2 shows that less than 3% of the shaved area is covered with new hair growth when the vehicle (control) is administered.
FIG. 3 is a photograph of mice treated with 10 µM of GPI 1046 after six weeks. FIG. 3 shows the remarkable effects of non-immunosuppressive neuroimmunophilin FKBP ligands wherein 90% of the shaved area is covered with new hair growth.
FIG. 4 is a photograph of mice treated with 30 µM of GPI 1046 after six weeks. FIG. 4 shows the remarkable ability of non-immunosuppressive neuroimmunophilin FKBP ligands to achieve, essentially, complete hair regrowth in the shaved area.
FIG. 5 is a bar graph depicting the relative hair growth indices of mice treated with a vehicle, FK506, and various non-immunosuppressive neuroimmunophilin FKBP ligands 14 days after treatment with each identified compound. FIG. 5 demonstrates the remarkable early hair growth promoted by non-immunosuppressive neuroimmunophilin FKBP ligands.

### DETAILED DESCRIPTION OF THE INVENTION

### Definitions

"Alopecia" refers to deficient hair growth and partial or complete loss of hair, including without limitation androgenic alopecia (male pattern baldness), toxic alopecia, alopecia senilis, alopecia areata, alopecia pelada and trichotillomania. Alopecia results when the pilar cycle is disturbed. The most frequent phenomenon is a shortening of the hair growth or anagen phase due to cessation of cell proliferation. This results in an early onset of the catagen phase, and consequently a large number of hairs in the telogen phase during which the follicles are detached from the dermal papillae, and the hairs fall out. Alopecia has a number of etiologies, including genetic factors, aging, local and systemic diseases, febrile conditions, mental stresses, hormonal problems, and secondary effects of drugs.

"GPI 1605" refers to a compound of formula

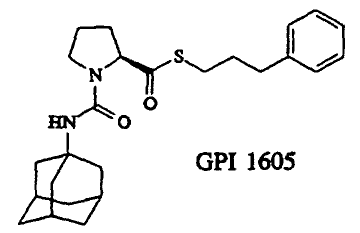

"GPI 1046" refers to 3-(3-pyridyl)-1-propyl (2s)-1-(3,3-dimethyl-1,2-dioxopentyl)-2-pyrrolidinecarboxylate, a compound of formula

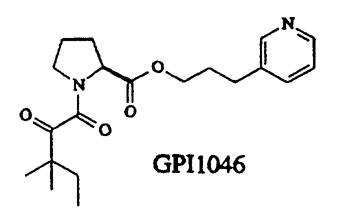

"GPI 1312" refers to a compound of formula

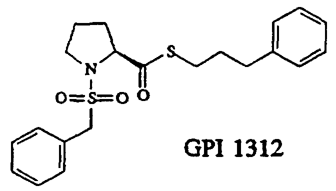

"GPI 1572" refers to a compound of formula

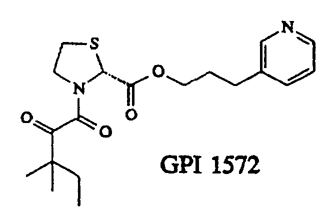

"GPI 1389" refers to a compound of formula

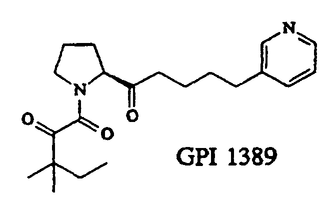

"GPI 1511" refers to a compound of formula

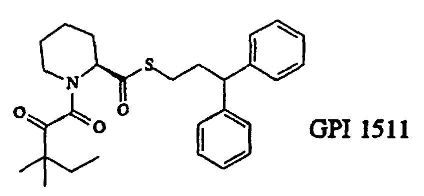

"GPI 1234" refers to a compound of formula

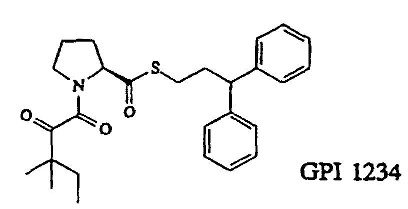

"Isomers" refer to different compounds that have the same molecular formula. "Stereoisomers" are isomers that differ only in the way the atoms are arranged in space. "Enantiomers" are a pair of stereoisomers that are non-superimposable mirror images of each other. "Diastereoisomers" are stereoisomers which are not mirror images of each other. "Racemic mixture" means a mixture containing equal parts of individual enantiomers. "Non-racemic mixture" is a mixture containing unequal parts of individual enantiomers or stereoisomers.

"Pharmaceutically acceptable salt" refers to a salt of the inventive compounds which possesses the desired pharmacological activity and which is neither biologically nor otherwise undesirable. The salt can be formed with inorganic acids such as acetate, adipate, alginate, aspartate, benzoate, benzenesulfonate, bisulfate butyrate, citrate, camphorate, camphorsulfonate, cyclopentanepropionate, digluconate, dodecylsulfate, ethanesulfonate, fumarate, glucoheptanoate, glycerophosphate, hemisulfate heptanoate, hexanoate, hydrochloride hydrobromide, hydroiodide, 2-hydroxyethanesulfonate, lactate, maleate, methanesulfonate, 2-naphthalenesulfonate, nicotinate, oxalate, thiocyanate, tosylate and undecanoate. Examples of a base salt include ammonium salts, alkali metal salts such as sodium and potassium salts, alkaline earth metal salts such as calcium and magnesium salts, salts with organic bases such as dicyclohexylamine salts, N-methyl-D-glucamine, and salts with amino acids such as arginine and lysine. Also, the basic nitrogen-containing groups can be quarternized with agents including: lower alkyl halides such as methyl, ethyl, propyl, and butyl chlorides, bromides and iodides; dialkyl sulfates such as dimethyl, diethyl, dibutyl and diamyl sulfates; long chain halides such as decyl, lauryl, myristyl and stearyl chlorides, bromides and iodides; and aralkyl halides such as benzyl and phenethyl bromides.

"Pilar cycle" refers to the life cycle of hair follicles, and includes three phases:
(1) the anagen phase, the period of active hair growth which, insofar as scalp hair is concerned, lasts about three to five years;
(2) the catagen phase, the period when growth stops and the follicle atrophies which, insofar as scalp hair is concerned, lasts about one to two weeks; and
(3) the telogen phase, the rest period when hair progressively separates and finally falls out which, insofar as scalp hair is concerned, lasts about three to four months.
Normally 80 to 90 percent of the follicles are in the anagen phase, less than 1 percent being in the catagen phase, and the rest being in the telogen phase. In the telogen phase, hair is uniform in diameter with a slightly bulbous, non-pigmented root. By contrast, in the anagen phase, hair has a large colored bulb at its root.

"Promoting hair growth" refers to maintaining, inducing, stimulating, accelerating, or revitalizing the germination of hair.

"Treating alopecia" refers to:
(i) preventing alopecia in an animal which may be predisposed to alopecia; and/or
(ii) inhibiting, retarding or reducing alopecia; and/or
(iii) promoting hair growth; and/or
(iv) prolonging the anagen phase of the hair cycle; and/or
(v) converting vellus hair to growth as terminal hair. Terminal hair is coarse, pigmented, long hair in which the bulb of the hair follicle is seated deep in the dermis. Vellus hair, on the other hand, is fine, thin, non-pigmented short hair in which the hair bulb is located superficially in the dermis. As alopecia progresses, the hairs change from the terminal to the vellus type.

### Methods of the Present Invention

The present invention relates to a method for treating alopecia or promoting hair growth in an animal, which comprises administering to said animal an effective amount of a non-immunosuppressive neuroimmunophilin FKBP ligand.

The inventive method is particularly useful for treating male pattern alopecia, alopecia senilis, alopecia areata, alopecia resulting from skin lesions or tumors, alopecia resulting from cancer therapy such as chemotherapy and radiation, and alopecia resulting from systematic disorders such as nutritional disorders and internal secretion disorders.

### Non-Immunosuppressive Neuroimmunophilin FKBP Ligands

The non-immunosuppressive neuroimmunophilin FKBP ligand used in the method and pharmaceutical composition of the present invention is a low molecular weight, small molecule compound having an affinity for an FKBP-type immunophilin, such as FKBP12. When the compound binds to an FKBP-type immunophilin, it has been found to inhibit the prolylpeptidyl *cis-trans* isomerase activity, or rotamase, activity of the binding protein. Unexpectedly, the compound has also been found to stimulate hair growth. As its name suggests, the compound is devoid of any significant immunosuppressive activity.

Examples of a non-immunosuppressive neuroimmunophilin FKBP ligand that may be used in the inventive method and pharmaceutical composition are set forth below.

### I. HETEROCYCLIC THIOESTERS AND KETONES

### FORMULA I

The non-immunosuppressive neuroimmunophilin FKBP ligand may be a compound of formula I 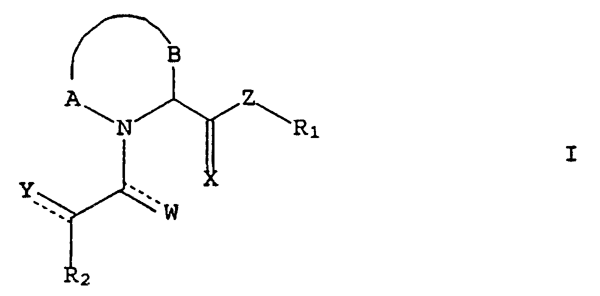 or a pharmaceutically acceptable salt, ester, or solvate thereof, wherein:
A and B, together with the nitrogen and carbon atoms to which they are respectively attached, form a 5-7 membered saturated or unsaturated heterocyclic ring containing one or more heteroatom(s) independently selected from the group consisting of O, S, SO, SO₂, N, NH, and NR₂;
X is either O or S;
Z is either S, CH₂, CHR₁ or CR₁R₃;
W and Y are independently O, S, CH₂ or H₂;
R₁ and R₃ are independently C₁-C₆ straight or branched chain alkyl or C₂-C₆ straight or branched chain alkenyl, wherein said alkyl or alkenyl is substituted with one or more substituent(s) independently selected from the group consisting of (Ar₁)ₙ, C₁-C₆ straight or branched chain alkyl or C₂-C₆ straight or branched chain alkenyl substituted with (Ar₁)ₙ, C₃-C₈ cycloalkyl, C₁-C₆ straight or branched chain alkyl or C₂-C₆ straight or branched chain alkenyl substituted with C₃-C₈ cycloalkyl, and Ar₂;
n is 1 or 2;
R₂ is either C₁-C₉ straight or branched chain alkyl, C₂-C₉ straight or branched chain alkenyl, C₃-C₈ cycloalkyl, C₅-C₇ cycloalkenyl, or Ar₁, wherein said alkyl, alkenyl, cycloalkyl or cycloalkenyl is either unsubstituted or substituted with one or more substituent(s) independently selected from the group consisting of C₁-C₄ straight or branched chain alkyl, C₂-C₄ straight or branched chain alkenyl, and hydroxy; and
Ar₁ and Ar₂ are independently an alicyclic or aromatic, mono-, bi- or tricyclic, carbo- or heterocyclic ring, wherein said ring is either unsubstituted or substituted with one or more substituent(s) independently selected from the group consisting of halo, hydroxyl, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino; wherein the individual ring size is 5-8 members; and wherein the heterocyclic ring contains 1-6 heteroatom(s) independently selected from the group consisting of O, N, and S.

Useful carbo- and heterocyclic rings include without limitation phenyl, benzyl, naphthyl, indenyl, azulenyl, fluorenyl, anthracenyl, indolyl, isoindolyl, indolinyl, benzofuranyl, benzothiophenyl, indazolyl, benzimidazolyl, benzthiazolyl, tetrahydrofuranyl, tetrahydropyranyl, pyridyl, pyrrolyl, pyrrolidinyl, pyridinyl, pyrimidinyl, purinyl, quinolinyl, isoquinolinyl, tetrahydroquinolinyl, quinolizinyl, furyl, thiophenyl, imidazolyl, oxazolyl, benzoxazolyl, thiazolyl, isoxazolyl, isotriazolyl, oxadiazolyl, triazolyl, thiadiazolyl, pyridazinyl, pyrimidinyl, pyrazinyl, triazinyl, trithianyl, indolizinyl, pyrazolyl, pyrazolinyl, pyrazolidinyl, thienyl, tetrahydroisoquinolinyl, cinnolinyl, phthalazinyl, quinazolinyl, quinoxalinyl, naphthyridinyl, pteridinyl, carbazolyl, acridinyl, phenazinyl, phenothiazinyl, and phenoxazinyl.

### FORMULA II

The non-immunosuppressive neuroimmunophilin FKBP ligand may also be a compound of formula II 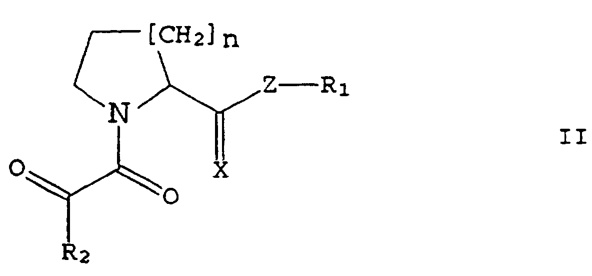 or a pharmaceutically acceptable salt, ester, or solvate thereof, wherein:
n is 1 or 2;
X is O or S;
Z is selected from the group consisting of S, CH₂, CHR₁, and CR₁R₃;
R₁ and R₃ are independently selected from the group consisting of C₁-C₅ straight or branched chain alkyl, C₂-C₅ straight or branched chain alkenyl, and Ar₁, wherein said alkyl, alkenyl or Ar₁ is unsubstituted or substituted with one or more substituent(s) independently selected from the group consisting of halo, nitro, C₁-C₆ straight or branched chain alkyl, C₂-C₆ straight or branched chain alkenyl, hydroxy, C₁-C₄ alkoxy, C₂-C₄ alkenyloxy, phenoxy, benzyloxy, amino, and Ar₁;
R₂ is selected from the group consisting of C₁-C₉ straight or branched chain alkyl, C₂-C₉ straight or branched chain alkenyl, C₃-C₈ cycloalkyl, C₅-C₇ cycloalkenyl, and Ar₁; and
Ar₁ is phenyl, benzyl, pyridyl, fluorenyl, thioindolyl or naphthyl, wherein said Ar₁ is unsubstituted or substituted with one or more substituent(s) independently selected from the group consisting of halo, trifluoromethyl, hydroxy, nitro, C₁-C₆ straight or branched chain alkyl, C₂-C₆ straight or branched chain alkenyl, C₁-C₄ alkoxy, C₂-C₄ alkenyloxy, phenoxy, benzyloxy, and amino.

Preferred compounds of formula II are presented in TABLE I.

**TABLE I**

| No. | n | X | Z | R, | R₂ |
|---|---|---|---|---|---|
| 1 | 1 | O | CH₂ | 3-Phenylpropyl | 1,1-Dimethylpropyl |
| 2 | 1 | O | CH₂ | 3-(3-Pyridyl)propyl | 1,1-Dimethylpropyl |
| 3 | 1 | 0 | CH₂ | 3-Phenylpropyl | *tert*-Butyl |
| 4 | 1 | O | CH₂ | 3-(3-Pyridyl)propyl | *tert*-Butyl |
| 5 | 1 | O | CH₂ | 3-(3-Pyridyl)propyl | Cyclohexyl |
| 6 | 1 | O | CH₂ | 3-(3-Pyridyl)propyl | Cyclopentyl |
| 7 | 1 | O | CH₂ | 3-(3-Pyridyl)propyl | Cycloheptyl |
| 8 | 1 | O | CH₂ | 2-(9-Fluorenyl)ethyl | 1,1-Dimethylpropyl |
| 9 | 1 | O | S | 2-Phenethyl | 1,1-Dimethylpropyl |
| 10 | 2 | O | S | 2-Phenethyl | 1,1-Dimethylpropyl |
| 11 | 1 | O | S | Methyl(2-thioindole) | 1,1-Dimethylpropyl |
| 12 | 1 | O | S | 2-Phenethyl | Cyclohexyl |
| 13 | 2 | O | S | 2-Phenethyl | *tert*-Butyl |
| 14 | 2 | O | S | 2-Phenethyl | Phenyl |
| 15 | 1 | O | CH₂ | 3-(4-Methoxyphenyl)propyl | 1,1-Dimethylpropyl |
| 16 | 2 | O | CH₂ | 4-(4-Methoxyphenyl)butyl | 1,1-Dimethylpropyl |
| 17 | 2 | O | CH₂ | 4-Phenylbutyl | 1,1-Dimethylpropyl |
| 18 | 2 | O | CH₂ | 4-Phenylbutyl | Phenyl |
| 19 | 2 | O | CH₂ | 4-Phenylbutyl | Cyclohexyl |
| 20 | 1 | S | CH₂ | 3-Phenylpropyl | 1,1-Dimethylpropyl |
| 21 | 1 | S | S | 2-Phenethyl | 1,1-Dimethylpropyl |
| 22 | 2 | S | CH₂ | 3-Phenylpropyl | 1,1-Dimethylpropyl |
| 23 | 2 | S | S | 2-Phenethyl | 1,1-Dimethylpropyl |
| 24 | 2 | O | CHR₁ | 3-Phenylpropyl | 1,1-Dimethylpropyl |
| 25 | 2 | O | CHR₁ | 3-Phenylpropyl | Cyclohexyl |
| 26 | 2 | O | CHR₁ | 3-Phenylpropyl | Phenyl |
| 27 | 2 | O | CHR₁ | 3-Phenylpropyl | 3,4,5-Trimethoxyphenyl |
| 28 | 1 | O | S | 2-Phenethyl | Cyclopentyl |
| 29 | 2 | O | S | 3-Phenylpropyl | *tert*-Butyl |
| 30 | 1 | O | S | 3-Phenylpropyl | 1,1-Dimethylpropyl |
| 31 | 1 | O | S | 3-(3-Pyridyl)propyl | 1,1-Dimethylpropyl |
| 32 | 1 | O | S | 3-Phenylpropyl | Cyclohexyl |
| 33 | 1 | O | S | 4-Phenylbutyl | Cyclohexyl |
| 34 | 1 | O | S | 4-Phenylbutyl | 1,1-Dimethylpropyl |
| 35 | 1 | O | S | 3-(3-Pyridyl)propyl | Cyclohexyl |
| 36 | 1 | O | S | 3,3-Diphenylpropyl | 1,1-Dimethylpropyl |
| 37 | 1 | O | S | 3,3-Diphenylpropyl | Cyclohexyl |
| 38 | 1 | O | S | 3-(4-Methoxyphenyl) propyl | 1,1-Dimethylpropyl |
| 39 | 2 | O | S | 4-Phenylbutyl | *tert*-Butyl |
| 40 | 2 | O | S | 1,5-Diphenylpentyl | 1,1-Dimethylpropyl |
| 41 | 2 | O | S | 1,5-Diphenylpentyl | Phenyl |
| 42 | 2 | O | S | 3-(4-Methoxyphenyl) propyl | 1,1-Dimethylpropyl |
| 43 | 2 | O | S | 3-(4-Methoxyphenyl) propyl | Phenyl |
| 44 | 2 | O | S | 3-(1-Naphthyl)propyl | 1,1-Dimethylpropyl |
| 45 | 1 | O | S | 3,3-Di(4-fluoro)phenylpropyl | 1,1-Dimethylpropyl |
| 46 | 1 | O | S | 4,4-Di(4-fluoro)phenylbutyl | 1.1-Dimethylpropyl |
| 47 | 1 | O | S | 3-(1-Naphthyl)propyl | 1,1-Dimethylpropyl |
| 48 | 1 | O | S | 2,2-Diphenylethyl | 1,1-Dimethylpropyl |
| 49 | 2 | O | S | 2,2-Diphenylethyl | 1,1-Dimethyipropyl |
| 50 | 2 | O | S | 3,3-Diphenylpropyl | 1,1-Dimethylpropyl |
| 51 | 1 | O | S | 3-(4-{Trifluoromethyl}phenyl)propyl | 1,1-Dimethylpropyl |
| 52 | 1 | O | S | 3-(2-Naphthyl)propyl | 1,1-Dimethylpropyl |
| 53 | 2 | O | S | 3-(1-Naphthyl)propyl | 1,1-Dimethylpropyl |
| 54 | 1 | O | S | 3-(3-Chloro)phenylpropyl | 1,1-Dimethylpropyl |
| 55 | 1 | O | S | 3-(3-{Trifluoromethyl}phenyl)propyl | 1,1-Dimethylpropyl |
| 56 | 1 | O | S | 3-(2-Biphenyl)propyl | 1,1-Dimethylpropyl |
| 57 | 1 | O | S | 3-(2-Fluorophenyl)propyl | 1,1-Dimethylpropyl |
| 58 | 1 | O | S | 3-(3-Fluorophenyl)propyl | 1,1-Dimethylpropyl |
| 59 | 2 | O | S | 4-Phenylbutyl | 1,1-Dimethylpropyl |
| 60 | 2 | O | S | 3-Phenylpropyl | 1,1-Dimethylpropyl |
| 61 | 1 | O | S | 3-(2-Chloro)phenylpropyl | 1,1-Dimethylpropyl |
| 62 | 2 | O | S | 3-(3-Chloro)phenylpropyl | 1,1-Dimethylpropyl |
| 63 | 2 | O | S | 3-(2-Fluoro)phenylpropyl | 1,1-Dimethylpropyl |
| 64 | 2 | O | S | 3-(3-Fluoro)phenylpropyl | 1,1-Dimethylpropyl |
| 65 | 1 | O | S | 3-(2,5-Dimethoxyphenyl)propyl | 1,1-Dimethylpropyl |
| 66 | 1 | O | CH₂ | 3-Phenylpropyl | Cyclohexyl |
| 67 | 1 | O | CH₂ | 3-Phenylethyl | *tert*-Butyl |
| 68 | 2 | O | CH₂ | 4-Phenylbutyl | Cyclohexyl |
| 69 | 2 | O | CHR₁ | 2-Phenylethyl | *tert*-Butyl |
| 70 | 1 | O | CH₂ | 3,3-Di(4-fluorophenyl)propyl | 1,1-Dimethylpropyl |
| 71 | 2 | O | CH₂ | 3-Phenylpropyl | 1,1-Dimethylpropyl |

Preferred compounds of TABLE I are named as follows:
1 (2*S*)-2-({1-Oxo-5-phenyl}-pentyl-1-(3,3-dimethyl-1,2-dioxopentyl)pyrrolidine
2 3,3-Dimethyl-1-[(2*S*)-2-(5-(3-pyridyl)pentanoyl)-1-pyrrolidinel-1,2-pentanedione
3 (2*S*)-2-({1-Oxo-4-phenyl}-butyl-1-(3,3-dimethyl-1,2-dioxobutyl)pyrrolidine
9 2-Phenyl-1-ethyl (2*S*)-1-(3,3-dimethyl-1,2-dioxopentyl)-2-pyrrolidinecarbothioate
10 2-Phenyl-1-ethyl 1-(3,3-dimethyl-1,2-dioxopentyl)-2-piperidinecarbothioate
11 (3-Thioindolyl)methyl (2*S*)-1-(3,3-dimethyl-1,2-dioxopentyl)-2-pyrrolidinecarbothioate
12 2-Phenyl-1-ethyl (2*S*)-1-(2-cyclohexyl-1,2-dioxoethyl)-2-pyrrolidinecarbothioate
14 2-Phenyl-1-ethyl 1-(2-phenyl-1,2-dioxoethyl)-2-piperidinecarbothioate
28 2-Phenyl-1-ethyl (2*S*)-1-(1-cyclopentyl-1,2-dioxoethyl)-2-pyrrolidinecarbothioate
29 3-Phenyl-1-propyl 1-(3,3-dimethyl-1,2-dioxobutyl)-2-piperidinecarbothioate
30 3-Phenyl-1-propyl (2*S*)-1-(3,3-dimethyl-1,2-dioxopentyl)-2-pyrrolidinecarbothioate
31 3-(3-Pyridyl)-1-propyl (2*S*)-1-(3,3-dimethyl-1,2-dioxopentyl)-2-pyrrolidinecarbothioate
32 3-Phenyl-1-propyl (2*S*)-1-(2-cyclohexyl-1,2-dioxoethyl)-2-pyrrolidinecarbothioate
33 4-Phenyl-1-butyl (2*S*)-1-(2-cyclohexyl-1,2-dioxoethyl)-2-pyrrolidinecarbothioate
34 4-Phenyl-1-butyl (2*S*)-1-(3,3-dimethyl-1,2-dioxopentyl)-2-pyrrolidinecarbothioate
35 3-(3-Pyridyl)-1-propyl (2*S*)-1-(2-cyclohexyl-1,2-dioxoethyl)-2-pyrrolidinecarbothioate
36 3,3-Diphenyl-1-propyl (2*S*)-1-(3,3-dimethyl-1,2-dioxopentyl)-2-pyrrolidinecarbothioate
37 3,3-Diphenyl-1-propyl (2*S*)-1-(2-cyclohexyl-1,2-dioxoethyl)-2-pyrrolidinecarbothioate
38 3-(*para*-Methoxyphenyl)-1-propyl (2*S*)-1-(3,3-dimethyl-1,2-dioxopentyl)-2-pyrrolidinecarbothioate
39 4-Phenyl-1-butyl 1-(1,2-dioxo-3,3-dimethylbutyl) - 2-piperidinecarbothioate
40 1,5-Diphenyl-3-pentyl 1-(3,3-dimethyl-1,2-dioxopentyl)-2-piperidinecarbothioate
41 1,5-Diphenyl-3-mercaptopentyl 1-(3-phenyl-1,2-dioxoethyl)-2-piperidinecarbothioate
42 3-(*para*-Methoxyphenyl)-1-propyl 1-(1,2-dioxo-3,3-dimethylpentyl)piperidine-2-carbothioate
43 3-(*para*-Methoxyphenyl)-1-propyl 1-(2-phenyl-1,2-dioxoethyl)piperidine-2-carbothioate
44 3-(1-Naphthyl)-1-propyl 1-(3,3-dimethyl-1,2-dioxopentyl)piperidine-2-carbothioate
45 3,3-Di(*para*-fluoro)phenyl-1-propyl (2*S*)-1-(3,3-dimethyl-1,2-dioxopentyl)-2-pyrrolidinecarbothioate
46 4,4-Di(para-fluorophenyl)butyl 1-(3,3-dimethyl-2-oxopentanoyl)-2-pyrrolidinecarbothioate
47 3-(1-Naphthyl)propyl (2*S*)-1-(3,3-dimethyl-2-oxopentanoyl)-2-pyrrolidinecarbothioate
48 2,2-Diphenylethyl (2*S*)-1-(3,3-dimethyl-2-oxopentanoyl)tetrahydro-1H-2-pyrrolidinecarbothioate
49 2,2-Diphenylethyl (2*S*)-1-(3,3-dimethyl-2-oxopentanoyl)-2-piperidinecarbothioate
50 3,3-Diphenylpropyl 1-(3,3-dimethyl-2-oxopentanoyl)-2-piperidinecarbothioate
51 3-[4-(Trifluoromethyl)phenyl]propyl (2*S*)-1-(3,3-dimethyl-2-oxopentanoyl)-2-pyrrolidinecarbothioate
52 3-(2-Naphthyl)propyl (2*S*)-1-(3,3-dimethyl-2-oxopentanoyl)-2-pyrrolidinecarbothioate
53 3-(2-Naphthyl)propyl (2*R*,*S*)-1-(3,3-dimethyl-2-oxopentanoyl)-2-piperidinecarbothioate
54 3-(3-Chlorophenyl)propyl (2*S*)-1-(3,3-dimethyl-2-oxopentanoyl)-2-pyrrolidinecarbothioate
55 3-[3-(Trifluoromethyl)phenyl]propyl (2*S*)-1-(3,3-dimethyl-2-oxopentanoyl)-2-pyrrolidinecarbothioate
56 3-(1-Biphenyl)propyl (2*S*)-1-(3,3-dimethyl-2-oxopentanoyl)-2-pyrrolidinecarbothioate
57 3-(2-Fluorophenyl)propyl (2*S*)-1-(3,3-dimethyl-2-oxopentanoyl)-2-pyrrolidinecarbothioate
58 3-(3-Fluorophenyl)propyl (2*S*)-1-(3,3-dimethyl-2-oxopentanoyl)-2-pyrrolidinecarbothioate
59 4-Phenylbutyl 1-(3,3-dimethyl-2-oxopentanoyl)-2-piperidinecarbothioate
60 3-Phenylpropyl 1-(3,3-dimethyl-2-oxopentanoyl)-2-piperidinecarbothioate
61 3-(2-Chlorophenyl)propyl (2*S*)-1-(3,3-dimethyl-2-oxopentanoyl)-2-pyrrolidinecarbothioate
62 3-(2-Chlorophenyl)propyl 1-(3,3-dimethyl-2-oxopentanoyl)-2-piperidinecarbothioate
63 3-(2-Fluorophenyl)propyl 1-(3,3-dimethyl-2-oxopentanoyl)-2-piperidinecarbothioate
64 3-(3-Fluorophenyl)propyl 1-(3,3-dimethyl-2-oxopentanoyl)-2-piperidinecarbothioate
65 3-(3,4-Dimethoxyphenyl)propyl (2*S*)-1-(3,3-dimethyl-2-oxopentanoyl)-2-pyrrolidinecarbothioate
66 (2*S*)-2-({1-Oxo-4-phenyl}-butyl-1-(2-Cyclohexyl-1,2-dioxoethyl)pyrrolidine
67 2-({1-Oxo-4-phenyl}-butyl-1-(3,3-dimethyl-1,2-dioxobutyl)pyrrolidine
68 2-({1-Oxo-6-phenyl}-hexyl-1-(2-Cyclohexyl-1,2-dioxoethyl)piperidine
69 2-({1-Oxo-[2-{2'-phenyl}ethyl]-4-phenyl}-butyl-1-(3,3-dimethyl-1,2-dioxobutyl)piperidine
70 1-{(2*S*)-2-[5,5-di(4-Fluorophenyl)pentanoyl]-2-pyrrolidine}-3,3-dimethyl-1,2-pentanedione
71 3,3-Dimethyl-1-[2- (4-phenylpentanoyl)piperidino]-1,2-pentanedione

### FORMULA III

Furthermore, the non-immunosuppressive neuroimmunophilin FKBP ligand may be a compound of formula III 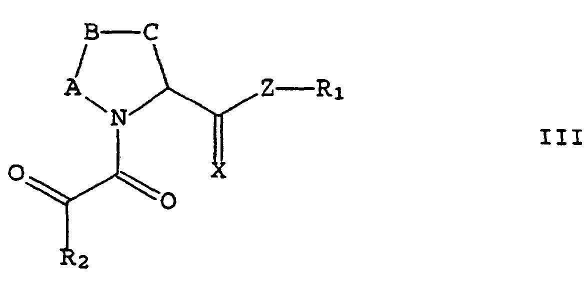 or a pharmaceutically acceptable salt, ester, or solvate thereof, wherein:
A, B, and C are independently CH₂, O, S, SO, SO₂, NH or NR₂;
X is O or S;
Z is S, CH₂, CHR₁ or CR₁R₃;
R₁ and R₃ are independently C₁-C₆ straight or branched chain alkyl or C₂-C₆ straight or branched chain alkenyl, wherein said alkyl or alkenyl is substituted with one or more substituent(s) independently selected from the group consisting of (Ar₁)ₙ, C₁-C₆ straight or branched chain alkyl or C₂-C₆ straight or branched chain alkenyl substituted with (Ar₁)ₙ, C₃-C₈ cycloalkyl, C₁-C₆ straight or branched chain alkyl or C₂-C₆ straight or branched chain alkenyl substituted with C₃-C₈ cycloalkyl, and Ar₂;
n is 1 or 2;
R₂ is either C₁-C₉ straight or branched chain alkyl, C₂-C₉ straight or branched chain alkenyl, C₃-C₈ cycloalkyl, C₅-C₇ cycloalkenyl or Ar₁, wherein said alkyl, alkenyl, cycloalkyl or cycloalkenyl is either unsubstituted or substituted with one or more substituent(s) independently selected from the group consisting of C₁-C₄ straight or branched chain alkyl, C₂-C₄ straight or branched chain alkenyl, and hydroxyl; and
Ar₁ and Ar₂ are independently an alicyclic or aromatic, mono-, bi- or tricyclic, carbo- or heterocyclic ring, wherein said ring is either unsubstituted or substituted with one or more substituent(s) independently selected from the group consisting of halo, hydroxyl, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino; wherein the individual ring size is 5-8 members; and wherein the heterocyclic ring contains 1-6 heteroatom(s) independently selected from the group consisting of O, N, and S.

Preferred compounds of formula III are presented in TABLE II.

**TABLE II**

| No. | A | B | C | X | Z | R₁ | R₂ |
|---|---|---|---|---|---|---|---|
| 72 | CH₂ | S | CH₂ | O | S | 2-phenethyl | 1,1-dimethyl-propyl |
| 73 | CH₂ | S | CH₂ | O | CH₂ | 3-phenylpropyl | 1,1-dimethyl-propyl |
| 74 | CH₂ | CH₂ | NH | O | S | 2-phenethyl | 1,1-dimethyl-propyl |
| 75 | CH₂ | S | CH₂ | S | S | 2-phenethyl | 1,1-dimethyl-propyl |

### FORMULA IV

Alternatively, the non-immunosuppressive neuroimmunophilin FKBP ligand may be a compound of formula IV 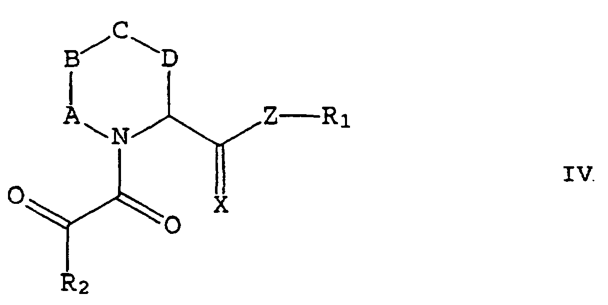 or a pharmaceutically acceptable salt, ester, or solvate thereof, wherein:
A, B, C and D are independently CH₂, O, S, SO, SO₂, NH or NR₂;
X is O or S;
Z is S, CH₂, CHR₁ or CR₁R₃;
R₁ and R₃ are independently C₁-C₆ straight or branched chain alkyl or C₂-C₆ straight or branched chain alkenyl, wherein said alkyl or alkenyl is substituted with one or more substituent(s) independently selected from the group consisting of (Ar₁)ₙ, C₁-C₆ straight or branched chain alkyl or C₂-C₆ straight or branched chain alkenyl substituted with (Ar₁)ₙ, C₃-C₈ cycloalkyl, C₁-C₆ straight or branched chain alkyl or C₂-C₆ straight or branched chain alkenyl substituted with C₃-C₈ cycloalkyl, and Ar₂;
n is 1 or 2;
R₂ is either C₁-C₉ straight or branched chain alkyl, C₂-C₉ straight or branched chain alkenyl, C₃-C₈ cycloalkyl, C₅-C₇ cycloalkenyl or Ar₁, wherein said alkyl, alkenyl, cycloalkyl or cycloalkenyl is either unsubstituted or substituted with one or more substituent(s) independently selected from the group consisting of C₃-C₈ cycloalkyl, C₁-C₄ straight or branched chain alkyl, C₂-C₄ straight or branched chain alkenyl, and hydroxyl; and
Ar₁ and Ar₂ are independently an alicyclic or aromatic, mono-, bi- or tricyclic, carbo- or heterocyclic ring, wherein said ring is either unsubstituted or substituted with one or more substituent(s) independently selected from the group consisting of halo, hydroxyl, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino; wherein the individual ring size is 5-8 members; and wherein the heterocyclic ring contains 1-6 heteroatom(s) independently selected from the group consisting of O, N, and S.

Preferred compounds of formula IV are presented in TABLE III.

**TABLE III**

| No. | A | B | C | D | X | Z | R₁ | R₂ |
|---|---|---|---|---|---|---|---|---|
| 76 | CH₂ | CH₂ | O | CH₂ | O | CH₂ | 3-phenylpropyl | 1,1-dimethylpropyl |
| 77 | CH₂ | CH₂ | O | CH₂ | O | S | 2-phenethyl | 1,1-dimethylpropyl |
| 78 | CH₂ | CH₂ | S | CH₂ | O | CH₂ | 3-phenylpropyl | 1,1-dimethylpropyl |
| 79 | CH₂ | CH₂ | S | CH₂ | O | S | 2-phenethyl | 1,1-dimethylpropyl |

### FORMULA V

The non-immunosuppressive neuroimmunophilin FKBP ligand may further be a compound of formula V 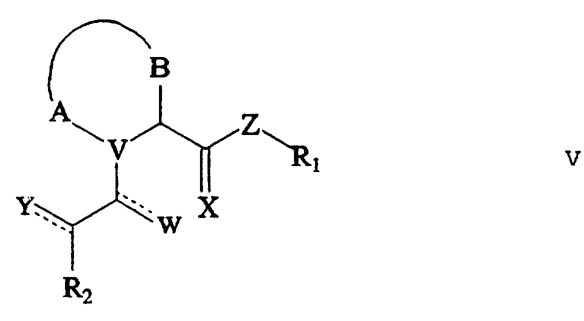 or a pharmaceutically acceptable salt, ester, or solvate thereof, wherein:
V is C, N, or S;
A and B, together with V and the carbon atom to which they are respectively attached, form a 5-7 membered saturated or unsaturated heterocyclic ring containing, in addition to V, one or more heteroatom(s) independently selected from the group consisting of O, S, SO, SO₂, N, NH, and NR₄;
R₄ is either C₁-C₉ straight or branched chain alkyl, C₂-C₉ straight or branched chain alkenyl, C₃-C₉ cycloakyl, C₅-C₇ cycloalkenyl, or Ar₃, wherein R₄ is either unsubstituted or substituted with one or more substituent(s) independently selected from the group consisting of halo, haloalkyl, carbonyl, carboxy, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, thioalkyl, alkylthio, sulfhydryl, amino, alkylamino, aminoalkyl, aminocarboxyl, and Ar₄;
Ar₃ and Ar₄ are independently an alicyclic or aromatic, mono-, bi- or tricyclic, carbo- or heterocyclic ring; wherein the individual ring size is 5-8 members; wherein said heterocyclic ring contains 1-6 heteroatom(s) independently selected from the group consisting of O; N, and S; and
R₁, R₂, W, X, Y, and Z are as defined in Formula I above.

### II. HETEROCYCLIC ESTERS AND AMIDES

### FORMULA VI

Additionally, the non-immunosuppressive neuroimmunophilin FKBP ligand may be a compound of formula VI 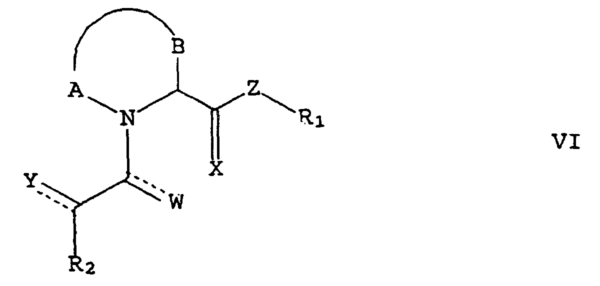 or a pharmaceutically acceptable salt, ester, or solvate thereof, wherein:
A and B, together with the nitrogen and carbon atoms to which they are respectively attached, form a 5-7 membered saturated or unsaturated heterocyclic ring containing, in addition to the nitrogen atom, one or more heteroatom(s) independently selected from the group consisting of O, S, SO, SO₂, N, NH, and NR₁;
X is O or S;
Z is O, NH or NR₁;
W and Y are independently O, S, CH₂ or H₂;
R₁ is C₁-C₆ straight or branched chain alkyl or C₂-C₆ straight or branched chain alkenyl, which is substituted with one or more substituent(s) independently selected from the group consisting of (Ar₁)ₙ, C₁-C₆ straight or branched chain alkyl or C₂-C₆ straight or branched chain alkenyl substituted with (Ar₁)ₙ, C₃-C₈ cycloalkyl, C₁-C₆ straight or branched chain alkyl or C₂-C₆ straight or branched chain alkenyl substituted with C₃-C₈ cycloalkyl, and Ar₂;
n is 1 or 2;
R₂ is either C₁-C₉ straight or branched chain alkyl, C₂-C₉ straight or branched chain or alkenyl, C₃-C₈ cycloalkyl, C₅-C₇ cycloalkenyl, or Ar₁, wherein said alkyl, alkenyl, cycloalkyl or cycloalkenyl is either unsubstituted or substituted with one or more substituent(s) independently selected from the group consisting of C₁-C₄ straight or branched chain alkyl, C₂-C₄ straight or branched chain alkenyl, and hydroxyl; and
Ar₁ and Ar₂ are independently an alicyclic or aromatic, mono-, bi- or tricyclic, carbo- or heterocyclic ring, wherein the ring is either unsubstituted or substituted with one or more substituent(s) independently selected from the group consisting of halo, hydroxyl, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino; wherein the individual ring size is 5-8 members; and wherein the heterocyclic ring contains 1-6 heteroatom(s) independently selected from the group consisting of O, N, and S.

Suitable carbo- and heterocyclic rings include without limitation naphthyl, indolyl, furyl, thiazolyl, thienyl, pyridyl, quinolinyl, isoquinolinyl, fluorenyl and phenyl.

### FORMULA VII

The non-immunosuppressive neuroimmunophilin FKBP ligand may also be a compound of formula VII 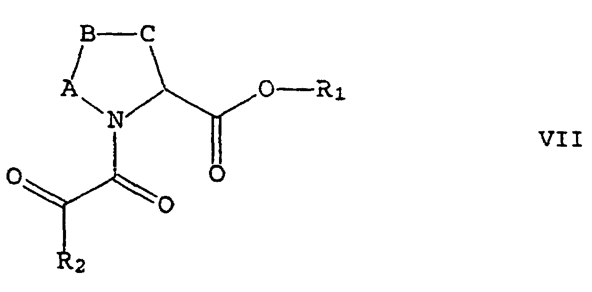 or a pharmaceutically acceptable salt, ester, or solvate thereof, wherein:
A, B and C are independently CH₂, O, S, SO, SO₂, NH or NR₁;
R₁ is C₁-C₅ straight or branched chain alkyl or C₂-C₅ straight or branched chain alkenyl, which is substituted with one or more substituent(s) independently selected from the group consisting of (Ar₁)ₙ and C₁-C₆ straight or branched chain alkyl or C₂-C₆ straight or branched chain alkenyl substituted with (Ar₁)ₙ;
n is 1 or 2;
R₂ is either C₁-C₉ straight or branched chain alkyl, C₂-C₉ straight or branched chain alkenyl, C₃-C₈ cycloalkyl, C₅-C₇ cycloalkenyl, or Ar₁; and
Ar₁ is an alicyclic or aromatic, mono-, bi- or tricyclic, carbo- or heterocyclic ring, wherein the ring is either unsubstituted or substituted with one or more substituent(s) independently selected from the group consisting of halo, hydroxyl, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino; wherein the individual ring size is 5-8 members; and wherein the heterocyclic ring contains 1-6 heteroatom(s) independently selected from the group consisting of O, N, and S.

In a preferred embodiment of the compounds of formula VII, the heterocyclic ester or amide is the Compound GPI 1572, of the formula

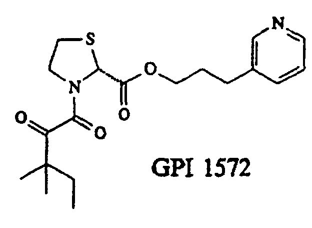

In a particularly preferred embodiment of formula VII compounds:
A is CH₂;
B is CH₂ or S;
C is CH₂ or NH;
R₁ is selected from the group consisting of 3-phenylpropyl and 3-(3-pyridyl)propyl; and
R₂ is selected from the group consisting of 1,1-dimethylpropyl, cyclohexyl, and tert-butyl.

Specific examples of this embodiment are presented in TABLE IV.

**TABLE IV**

| No. | A | B | C | R₁ | R₂ |
|---|---|---|---|---|---|
| 80 | CH₂ | S | CH₂ | 3-phenylpropyl | 1,1-dimethylpropyl |
| 81 | CH₂ | S | CH₂ | 3-(3-pyridyl)propyl | 1,1-dimethylpropyl |
| 82 | CH₂ | S | CH₂ | 3-phenylpropyl | cyclohexyl |
| 83 | CH₂ | S | CH₂ | 3-phenylpropyl | *tert*-butyl |
| 84 | CH₂ | CH₂ | NH | 3-phenylpropyl | 1,1-dimethylpropyl |
| 85 | CH₂ | CH₂ | NH | 3-phenylpropyl | cyclohexyl |
| 86 | CH₂ | CH₂ | NH | 3-phenylpropyl | *tert*-butyl |

### FORMULA VIII

In a further embodiment of this invention, the non-immunosuppressive neuroimmunophilin FKBP ligand may be a compound of formula VIII 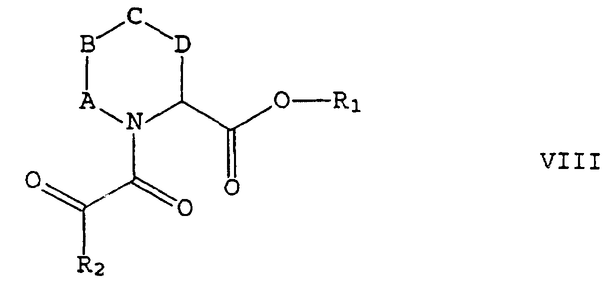 or a pharmaceutically acceptable salt, ester, or solvate thereof, wherein:
A, B, C and D are independently CH₂, O, S, SO, SO₂, NH or NR₁;
R₁ is C₁-C₅ straight or branched chain alkyl or C₂-C₅ straight or branched chain alkenyl, which is substituted with one or more substituent(s) independently selected from the group consisting of (Ar₁)ₙ and C₁-C₆ straight or branched chain alkyl or C₂-C₆ straight or branched chain alkenyl substituted with (Ar₁)ₙ;
n is 1 or 2;
R₂ is either C₁-C₉ straight or branched chain alkyl, C₂-C₉ straight or branched chain alkenyl, C₃-C₈ cycloalkyl, C₅-C₇ cycloalkenyl, or Ar₁; and
Ar₁ is an alicyclic or aromatic, mono-, bi- or tricyclic, carbo- or heterocyclic ring, wherein the ring is either unsubstituted or substituted with one or more substituent (s) independently selected from the group consisting of halo, hydroxyl, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino; wherein the individual ring size is 5-8 members; and wherein the heterocyclic ring contains 1-6 heteroatom(s) independently selected from the group consisting of O, N, and S.

In a particularly preferred embodiment of formula VIII compounds:
A is CH₂;
B is CH₂;
C is S, O or NH;
D is CH₂;
R₁ is selected from the group consisting of 3-phenylpropyl and (3,4,5-trimethoxy)phenylpropyl; and
R₂ is selected from the group consisting of 1,1-dimethylpropyl, cyclohexyl, *tert*-butyl, phenyl, and 3,4,5-trimethoxyphenyl.

Specific examples of this embodiment are presented in TABLE V.

**TABLE V**

| No. | A | B | C | D | R₁ | R₂ |
|---|---|---|---|---|---|---|
| 87 | CH₂ | CH₂ | S | CH₂ | 3-phenylpropyl | 1,1-dimethylpropyl |
| 88 | CH₂ | CH₂ | O | CH₂ | 3-phenylpropyl | 1,1-dimethylpropyl |
| 89 | CH₂ | CH₂ | S | CH₂ | 3-phenylpropyl | cyclohexyl |
| 90 | CH₂ | CH₂ | O | CH₂ | 3-phenylpropyl | cyclohexyl |
| 91 | CH₂ | CH₂ | S | CH₂ | 3-phenylpropyl | phenyl |
| 92 | CH₂ | CH₂ | O | CH₂ | 3-phenylpropyl | phenyl |
| 93 | CH₂ | CH₂ | NH | CH₂ | 3-phenylpropyl | 1,1-dimethylpropyl |
| 94 | CH₂ | CH₂ | NH | CH₂ | 3-phenylpropyl | phenyl |

### FORMULA IX

Additionally, the non-immunosuppressive neuroimmunophilin FKBP ligand may be a compound of formula IX 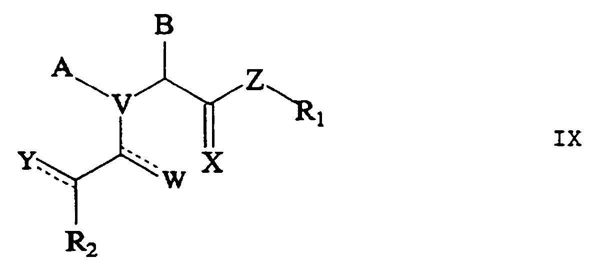 or a pharmaceutically acceptable salt, ester, or solvate thereof, wherein:
V is C, N, or S;
A and B, together with V and the carbon atom to which they are respectively attached, form a 5-7 membered saturated or unsaturated heterocyclic ring containing, in addition to V, one or more heteroatom(s) independently selected from the group consisting of O, S, SO, SO₂, N, NH, and NR;
R is either C₁-C₉ straight or branched chain alkyl, C₂-C₉ straight or branched chain alkenyl, C₃-C₉ cycloakyl, C₅-C₇ cycloalkenyl, or Ar₃, wherein R is either unsubstituted or substituted with one or more substituent(s) independently selected from the group consisting of halo, haloalkyl, carbonyl, carboxy, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, thioalkyl, alkylthio, sulfhydryl, amino, alkylamino, aminoalkyl, aminocarboxyl, and Ar₄;
Ar₃ and Ar₄ are independently an alicyclic or aromatic, mono-, bi- or tricyclic, carbo- or heterocyclic ring; wherein the individual ring size is 5-8 members; wherein said heterocyclic ring contains 1-6 heteroatom(s) independently selected from the group consisting of O, N, and S; and
R₁, R₂, W, X, Y, and Z are as defined in Formula VI above.

### III. N-OXIDES OF HETEROCYCLIC ESTERS, AMIDES, THIOESTERS AND KETONES

### FORMULA X

The non-immunosuppressive neuroimmunophilin FKBP ligand may further be a compound of formula X 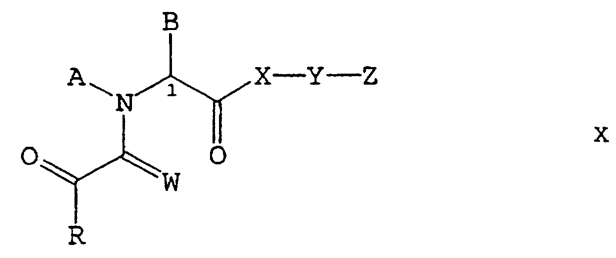 or a pharmaceutically acceptable salt, ester, or solvate thereof, wherein:
A and B, together with the nitrogen and carbon atoms to which they are respectively attached, form a 5-7 membered saturated or unsaturated heterocyclic ring containing one or more heteroatom(s) independently selected from the group consisting of CH, CH₂, O, S, SO, SO₂, N, NH, and NR₁;
W is O, S, CH₂, or H₂;
R is C₁-C₆ straight or branched chain alkyl, C₂-C₆ straight or branched chain alkenyl, C₃-C₈ cycloalkyl, C₅-C₇ cycloalkenyl, or Ar₁, which is optionally substituted with one or more substituent(s) independently selected from the group consisting of C₁-C₄ alkyl, C₂-C₄ alkenyl, hydroxy, C₃-C₈ cycloalkyl, C₅-C₇ cycloalkenyl, and Ar₂;
Ar₁ and Ar₂ are independently selected from the group consisting of 1-napthyl, 2-napthyl, 1-indolyl, 2-indolyl, 2-furyl, 3-furyl, 2-thienyl, 3-thienyl, 2-pyridyl, 3-pyridyl, 4-pyridyl and phenyl, having one or more substituent (s) independently selected from the group consisting of hydrogen, halo, hydroxy, nitro, trifluoromethyl, C₁-C₆ straight or branched chain alkyl, C₂-C₆ straight or branched chain alkenyl, C₂-C₄ alkenyloxy, phenoxy, benzyloxy, and amino;
X is O, NH, NR₁, S, CH, CR₁, or CR₁R₃;
Y is a direct bond, C₁-C₆ straight or branched chain alkyl, or C₂-C₆ straight or branched chain alkenyl; wherein said alkyl or alkenyl is optionally substituted with one or more substituent(s) independently selected from the group consisting of C₁-C₆ straight or branched chain alkyl, C₂-C₆ straight or branched chain alkenyl, C₃-C₈ cycloalkyl, C₅-C₇ cycloalkenyl, hydroxy, carbonyl oxygen, and Ar; wherein said alkyl, alkenyl, cycloalkyl, cycloalkenyl, or Ar is optionally substituted with C₁-C₄ alkyl, C₂-C₄ alkenyl, hydroxy, or carbonyl oxygen; wherein any carbon atom of said alkyl, alkenyl, cycloalkyl, cycloalkenyl, or Ar is optionally replaced with O, NH, NR₂, S, SO, or SO₂;
R₂ is selected from the group consisting of hydrogen, C₁-C₄ straight or branched chain alkyl, C₃-C₄ straight or branched chain alkenyl or alkynyl, and C₁-C₄ bridging alkyl wherein a bridge is formed between the nitrogen and a carbon atom of said alkyl or alkenyl chain containing said heteroatom to form a ring, wherein said ring is optionally fused to an Ar group;
Z is an aromatic amine or a tertiary amine oxidized to a corresponding N-oxide;
said aromatic amine is selected from the group consisting of pyridyl, pyrimidyl, quinolinyl, or isoquinolinyl, which is either unsubstituted or substituted with one or more substituent(s) independently selected from the group consisting of halo, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino;
said tertiary amine is NR₄R₅R₆, wherein R₄, R₅, and R₆ are independently selected from the group consisting of C₁-C₆ straight or branched chain alkyl or C₂-C₆ straight or branched chain alkenyl optionally substituted with one or more substituent(s) independently selected from the group consisting cf C₁-C₆ straight or branched chain alkyl, C₂-C₆ straight or branched chain alkenyl, C₃-C₈ cycloalkyl, C₅-C₇ cycloalkenyl, hydroxy, carbonyl oxygen, and Ar; wherein said alkyl, alkenyl, cycloalkyl, cycloalkenyl, or Ar is optionally substituted with C₁-C₄ alkyl, C₂-C₄ alkenyl, hydroxy, or carbonyl oxygen; wherein any carbon atom of said alkyl, alkenyl, cycloalkyl, cycloalkenyl, or Ar is optionally replaced with O, NH, NR₁, S, SO, or SO₂;
Ar is selected from the group consisting of pyrrolidinyl, pyridyl, pyrimidyl, pyrazyl, pyridazyl, quinolinyl, and isoquinolinyl; and
R₁ and R₃ are independently hydrogen, C₁-C₄ straight or branched chain alkyl, C₃-C₄ straight or branched chain alkenyl or alkynyl, or Y-Z.

### FORMULA XI

Moreover, the non-immunosuppressive neuroimmunophilin FKBP ligand may be a compound of formula XI 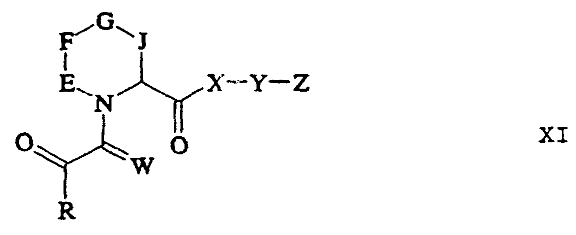 or a pharmaceutically acceptable salt, ester, or solvate thereof, wherein:
E, F, G and J are independently CH₂, O, S, SO, SO₂, NH or NR₁;
W is O, S, CH₂, or H₂;
R is C₁-C₆ straight or branched chain alkyl, C₂-C₆ straight or branched chain alkenyl, C₃-C₈ cycloalkyl, C₅-C₇ cycloalkenyl, or Ar₁, which is optionally substituted with one or more substituent(s) independently selected from the group consisting of C₁-C₄ alkyl, C₂-C₄ alkenyl, hydroxy, C₃-C₈ cycloalkyl, C₅-C₇ cycloalkenyl, and Ar₁;
Ar₁ is selected from the group consisting of 1-napthyl, 2-napthyl, 1-indolyl, 2-indolyl, 2-furyl, 3-furyl, 2-thienyl, 3-thienyl, 2-pyridyl, 3-pyridyl, 4-pyridyl, and phenyl, having one or more substituent(s) independently selected from the group consisting of hydrogen, halo, hydroxy, nitro, trifluoromethyl, C₁-C₆ straight or branched chain alkyl, C₂-C₆ straight or branched chain alkenyl, C₂-C₄ alkenyloxy, phenoxy, benzyloxy, and amino;
X is O, NH, NR₁, S, CH, CR₁, or CR₁R₃;
Y is a direct bond, C₁-C₆ straight or branched chain alkyl, or C₂-C₆ straight or branched chain alkenyl; wherein said alkyl or alkenyl is optionally substituted with one or more substituent(s) independently selected from the group consisting of C₁-C₆ straight or branched chain alkyl, C₂-C₆ straight or branched chain alkenyl, C₃-C₈ cycloalkyl, C₅-C₇ cycloalkenyl, hydroxy, carbonyl oxygen, and Ar; wherein said alkyl, alkenyl, cycloalkyl, cycloalkenyl, or Ar is optionally substituted with C₁-C₄ alkyl, C₂-C₄ alkenyl, hydroxy, or carbonyl oxygen; wherein any carbon atom of said alkyl, alkenyl, cycloalkyl, cycloalkenyl, or Ar is optionally replaced with O, NH, NR₂, S, SO, or SO₂;
R₂ is selected from the group consisting of hydrogen, C₁-C₄ straight or branched chain alkyl, C₃-C₄ straight or branched chain alkenyl or alkynyl, and C₁-C₄ bridging alkyl wherein a bridge is formed between the nitrogen and a carbon atom of said alkyl or alkenyl chain containing said heteroatom to form a ring, wherein said ring is optionally fused to an Ar group;
Z is an aromatic amine or a tertiary amine oxidized to a corresponding N-oxide;
said aromatic amine is pyridyl, pyrimidyl, quinolinyl, and isoquinolinyl, which is either unsubstituted or substituted with one or more substituent(s) independently selected from the group consisting of halo, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino;
said tertiary amine is NR₄R₅R₆, wherein R₄, R₅, and R₆ are independently selected from the group consisting of C₁-C₆ straight or branched chain alkyl and C₂-C₆ straight or branched chain alkenyl; wherein said alkyl or alkenyl is optionally substituted with one or more substituent(s) independently selected from the group consisting of C₁-C₆ straight or branched chain alkyl, C₂-C₆ straight or branched chain alkenyl, C₃-C₈ cycloalkyl; C₅-C₇ cycloalkenyl, hydroxy, carbonyl oxygen, and Ar; wherein said alkyl, alkenyl, cycloalkyl, cycloalkenyl, or Ar is optionally substituted with C₁-C₄ alkyl, C₂-C₄ alkenyl, hydroxy, or carbonyl oxygen; wherein any carbon atom of said alkyl, alkenyl, cycloalkyl, cycloalkenyl, or Ar is optionally replaced with O, NH, NR₁, S, SO, or SO₂;
Ar is selected from the group consisting of pyrrolidinyl, pyridyl, pyrimidyl, pyrazyl, pyridazyl, quinolinyl, and isoquinolinyl; and
R₁ and R₃ are independently hydrogen, C₁-C₄ straight or branched chain alkyl, C₃-C₄ straight or branched chain alkenyl or alkynyl, or Y-Z.

### FORMULA XII

Furthermore, the non-immunosuppressive neuroimmunophilin FKBP ligand may be a compound of formula XII 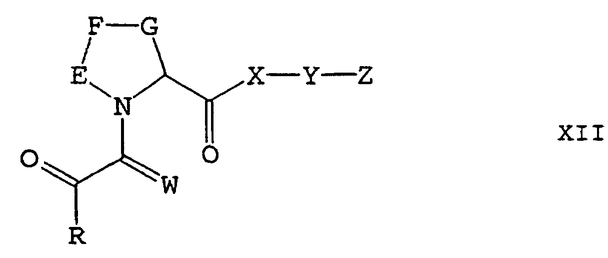 or a pharmaceutically acceptable salt, ester, or solvate thereof, wherein:
E, F, and G are independently CH₂, O, S, SO, SO₂, NH or NR₁;
W is O, S, CH₂, or H₂;
R is C₁-C₆ straight or branched chain alkyl, C₂-C₆ straight or branched chain alkenyl, C₃-C₈ cycloalkyl, C₅-C₇ cycloalkenyl, or Ar₁, which is optionally substituted with one or more substituent(s) independently selected from the group consisting of C₁-C₄ alkyl, C₂-C₄ alkenyl, hydroxy, C₃-C₈ cycloalkyl, C₅-C₇ cycloalkenyl, and Ar₁;
Ar₁ is selected from the group consisting of 1-napthyl, 2-napthyl, 1-indolyl, 2-indolyl, 2-furyl, 3-furyl, 2-thienyl, 3-thienyl, 2-pyridyl, 3-pyridyl, 4-pyridyl and phenyl, having one or more substituent(s) independently selected from the group consisting of hydrogen, halo, hydroxy, nitro, trifluoromethyl, C₁-C₆ straight or branched chain alkyl, C₂-C₆ straight or branched chain alkenyl, C₂-C₄ alkenyloxy, phenoxy, benzyloxy, and amino;
X is O, NH, NR₁, S, CH, CR₁, or CR₁R₃;
Y is a direct bond, C₁-C₆ straight or branched chain alkyl, or C₂-C₆ straight or branched chain alkenyl; wherein said alkyl or alkenyl is optionally substituted with one or more substituen (s) independently selected from the group consisting of C₁-C₆ straight or branched chain alkyl, C₂-C₆ straight or branched chain alkenyl, C₃-C₈ cycloalkyl, C₅-C₇ cycloalkenyl, hydroxy, carbonyl oxygen, and Ar; wherein said alkyl, alkenyl, cycloalkyl, cycloalkenyl, or Ar is optionally substituted with C₁-C₄ alkyl, C₂-C₄ alkenyl, hydroxy, or carbonyl oxygen; wherein any carbon atom of said alkyl, alkenyl, cycloalkyl, cycloalkenyl, or Ar is optionally replaced with 0, NH, NR₂, S, SO, or SO₂;
R₂ is selected from the group consisting of hydrogen, C₁-C₄ straight or branched chain alkyl, C₃-C₄ straight or branched chain alkenyl or alkynyl, and C₁-C₄ bridging alkyl wherein a bridge is formed between the nitrogen and a carbon atom of said alkyl or alkenyl chain containing said heteroatom to form a ring, wherein said ring is optionally fused to an Ar group;
Z is an aromatic amine or a tertiary amine oxidized to a corresponding N-oxide;
said aromatic amine is pyridyl, pyrimidyl, quinolinyl, or isoquinolinyl, which is either unsubstituted or substituted with one or more substituent(s) independently selected from the group consisting of halo, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amine;
said tertiary amine is NR₄R₅R₆, wherein R₄, R₅, and R₆ are independently selected from the group consisting of C₁-C₆ straight or branched chain alkyl and C₂-C₆ straight or branched chain alkenyl; wherein said alkyl or alkenyl is optionally substituted with one or more substituent(s) independently selected from the group consisting of C₁-C₆ straight or branched chain alkyl, C₂-C₆ straight or branched chain alkenyl, C₃-C₈ cycloalkyl, C₅-C₇ cycloalkenyl, hydroxy, carbonyl oxygen, and Ar; wherein said alkyl, alkenyl, cycloalkyl, cycloalkenyl, or Ar is optionally substituted with C₁-C₄ alkyl, C₂-C₄ alkenyl, hydroxy, or carbonyl oxygen; wherein any carbon atom of said alkyl, alkenyl, cycloalkyl, cycloalkenyl, or Ar is optionally replaced with O, NH, NR₁, S, SO, or SO₂;
Ar is selected from the group consisting of pyrrolidinyl, pyridyl, pyrimidyl, pyrazyl, pyridazyl, quinolinyl, and isoquinolinyl; and
R₁ and R₃ are independently hydrogen, C₁-C₄ straight or branched chain alkyl, C₃-C₄ straight or branched chain alkenyl or alkynyl, or Y-Z.

### FORMULA XIII

The non-immunosuppressive neuroimmunophilin FKBP ligand may also be a compound of formula XIII 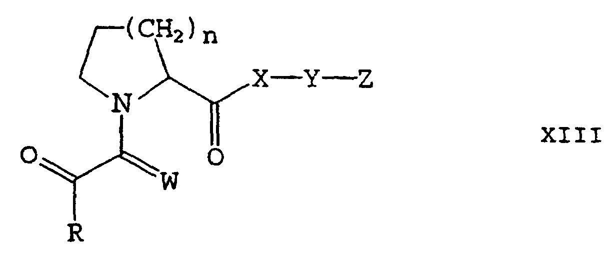 or a pharmaceutically acceptable salt, ester, or solvate thereof, wherein:
n is 1, 2, or 3, forming a 5-7 member heterocyclic ring;
W is O, S, CH₂, or H₂;
R is C₁-C₆ straight or branched chain alkyl, C₂-C₆ straight or branched chain alkenyl, C₃-C₈ cycloalkyl, C₅-C₇ cycloalkenyl, or Ar₁, which is optionally substituted with one or more substituent(s) independently selected from the group consisting of C₁-C₄ alkyl, C₂-C₄ alkenyl, hydroxy, C₃-C₈ cycloalkyl, C₅-C₇ cycloalkenyl, and Ar₁;
Ar₁ is selected from the group consisting of 1-napthyl, 2-napthyl, 1-indolyl, 2-indolyl, 2-furyl, 3-furyl, 2-thienyl, 3-thienyl, 2-pyridyl, 3-pyridyl, 4-pyridyl and phenyl, having one or more substituent(s) independently selected from the group consisting of hydrogen, halo, hydroxy, nitro, trifluoromethyl, C₁-C₆ straight or branched chain alkyl, C₂-C₆ straight or branched chain alkenyl, C₂-C₄ alkenyloxy, phenoxy, benzyloxy, and amino;
X is O, NH, NR₁, S, CH, CR₁, or CR₁R₃;
Y is a direct bond, C₁-C₆ straight or branched chain alkyl, or C₂-C₆ straight or branched chain alkenyl; wherein said alkyl or alkenyl is optionally substituted with one or more substituent(s) independently selected from the group consisting of C₁-C₆ straight or branched chain alkyl, C₂-C₆ straight or branched chain alkenyl, C₃-C₈ cycloalkyl, C₅-C₇ cycloalkenyl, hydroxy, carbonyl oxygen, and Ar; wherein said alkyl, alkenyl, cycloalkyl, cycloalkenyl, or Ar is optionally substituted with C₁-C₄ alkyl, C₂-C₄ alkenyl, hydroxy, or carbonyl oxygen; wherein any carbon atom of said alkyl, alkenyl, cycloalkyl, cycloalkenyl, or Ar is optionally replaced with O, NH, NR₂, S, SO, or SO₂;
R₂ is selected from the group consisting of hydrogen, C₁-C₄ straight or branched chain alkyl, C₃-C₄ straight or branched chain alkenyl or alkynyl, and C₁-C₄ bridging alkyl wherein a bridge is formed between the nitrogen and a carbon atom of said alkyl or alkenyl chain containing said heteroatom to form a ring, wherein said ring is optionally fused to an Ar group;
Z is an aromatic amine or a tertiary amine oxidized to a corresponding N-oxide;
said aromatic amine is pyridyl, pyrimidyl, quinolinyl, or isoquinolinyl, which is either unsubstituted or substituted with one or more substituent(s) independently selected from the group consisting of halo, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino;
said tertiary amine is NR₄R₅R₆, wherein R₄, R₅, and R₆ are independently selected from the group consisting of C₁-C₆ straight or branched chain alkyl and C₂-C₆ straight or branched chain alkenyl; wherein said alkyl or alkenyl is optionally substituted with one or more substituent(s) independently selected from the group consisting of C₁-C₆ straight or branched chain alkyl, C₂-C₆ straight or branched chain alkenyl, C₃-C₈ cycloalkyl, C₅-C₇ cycloalkenyl, hydroxy, carbonyl oxygen, and Ar; wherein said alkyl, alkenyl, cycloalkyl, cycloalkenyl, or Ar is optionally substituted with C₁-C₄ alkyl, C₂-C₄ alkenyl, hydroxy, or carbonyl oxygen; wherein any carbon atom of said alkyl, alkenyl, cycloalkyl, cycloalkenyl, or Ar is optionally replaced with O, NH, NR₁, S, SO, or SO₂;
Ar is selected from the group consisting of pyrrolidinyl, pyridyl, pyrimidyl, pyrazyl, pyridazyl, quinolinyl, and isoquinolinyl; and
R₁ and R₃ hydrogen, C₁-C₄ straight or branched chain alkyl, C₃-C₄ straight or branched chain alkenyl or alkynyl, or Y-Z

Examples of the compounds of formula XIII when W is O are presented in TABLE VI.

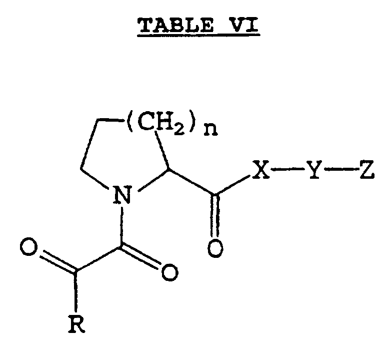

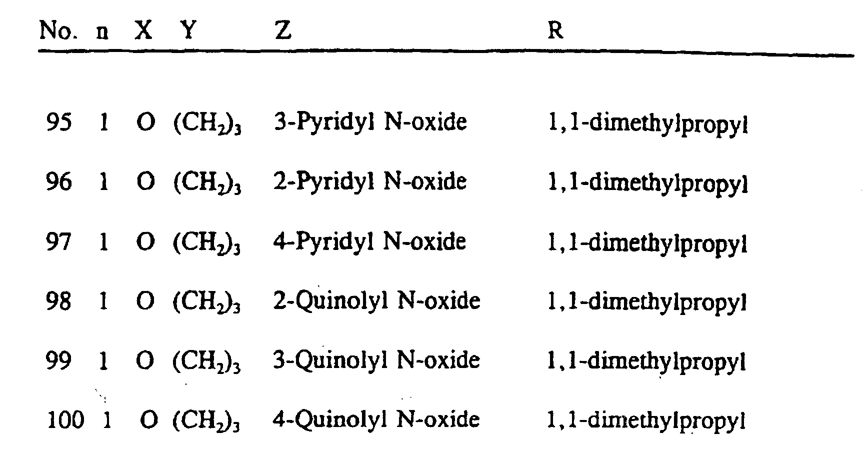

Preferred compounds of formula XIII may be selected from the group consisting of:
3-(2-Pyridyl)-1-propyl(2*S*)-1-(1,1-Dimethyl-1,2-dioxopentyl)-2-pyrrolidinecarboxylate, N-oxide;
3-(3-Pyridyl)-1-propyl (2*S*) -1- (1, 1-Dimethyl-1,2-dioxopentyl)-2-pyrrolidinecarboxylate, N-oxide;
3- (4-Pyridyl)-1-propyl(2*S*)-1- (1, 1-Dimethyl-1,2-dioxopentyl)-2-pyrrolidinecarboxylate, N-oxide;
3- (2-Quinolyl) -1-propyl(2*S*)-1- (1,1-Dimethyl-1,2-dioxopentyl)-2-pyrrolidinecarboxylate, N-oxide;
3-(3-Quinolyl)-1-propyl(2*S*)-1-(1,1-Dimethyl-1,2-dioxopentyl)-2-pyrrolidinecarbaxylate, N-oxide;
3-(4-Quinolyl)-1-propyl(2*S*)-1-(1,1-Dimethyl-1,2-dioxopentyl)-2-pyrrolidinecarboxylate, N-oxide; and
pharmaceutically acceptable salts, esters, and solvates thereof.

### FORMULA XIV

Additionally, the non-immunosuppressive neuroimmunophilin FKBP ligand may be a compound of formula XIV 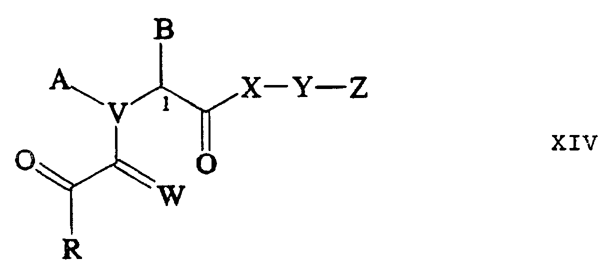 or a pharmaceutically acceptable salt, ester, or solvate thereof, wherein:
V is C, N, or S;
A and B, together with V and the carbon atom to which they are respectively attached, form a 5-7 membered saturated or unsaturated heterocyclic ring containing, in addition to V, one or more heteroatom(s) independently selected from the group consisting of O, S, SO, SO₂, N, NH, and NR₇;
R₇ is either C₁-C₉ straight or branched chain alkyl, C₂-C₉ straight or branched chain alkenyl, C₃-C₉ cycloalkyl, C₅-C₇ cycloalkenyl, or Ar₃, wherein R₇ is either unsubstituted or substituted with one or more substituent(s) independently selected from the group consisting of halo, haloalkyl, carbonyl, carboxy, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, thioalkyl, alkylthio, sulfhydryl, amino, alkylamino, aminoalkyl, aminocarboxyl, and Ar₄;
Ar₃ and Ar₄ are independently an alicyclic or aromatic, mono-, bi- or tricyclic, carbo- or heterocyclic ring; wherein the individual ring size is 5-8 members; wherein said heterocyclic ring contains 1-6 heteroatom(s) independently selected from the group consisting of O, N, and S; and
R, W, X, Y, and Z are as defined in Formula X above.

### IV. N-LINKED UREAS AND CARBAMATES OF HETEROCYCLIC THIOESTERS

The non-immunosuppressive neuroimmunophilin FKBP ligand may further be a compound of formula XV 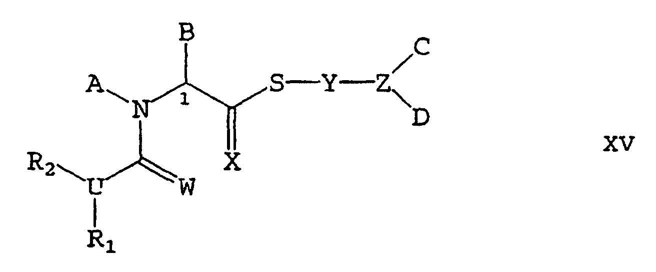 or a pharmaceutically acceptable salt, ester, or solvate thereof, wherein:
A and B, together with the nitrogen and carbon atoms to which they are respectively attached, form a 5-7 membered saturated or unsaturated heterocyclic ring containing, in addition to the nitrogen atom, one or more additional heteroatom(s) independently selected from the group consisting of O, S, SO, SO₂, N, NH, and NR₃;
X is either O or S;
Y is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any carbon atom of said alkyl or alkenyl is optionally replaced with O, NH, NR₃, S, SO, or SO₂;
R₃ is selected from the group consisting of hydrogen, C₁-C₆ straight or branched chain alkyl, C₃-C₆ straight or branched chain alkenyl or alkynyl, and C₁-C₄ bridging alkyl wherein a bridge is formed between the nitrogen and a carbon atom of said alkyl or alkenyl chain containing said heteroatom to form a ring, wherein said ring is optionally fused to an Ar group;
Ar is an alicyclic or aromatic, mono-, bi- or tricyclic, carbo- or heterocyclic ring, wherein the ring is either unsubstituted or substituted with one or more substituent(s) independently selected from the group consisting of alkylamino, amido, amino, aminoalkyl, azo, benzyloxy, C₁-C₉ straight or branched chain alkyl, C₁-C₉ alkoxy, C₂-C₉ alkenyloxy, C₂-C₉ straight or branched chain alkenyl, C₃-C₈ cycloalkyl, C₅-C₇ cycloalkenyl, carbonyl, carboxy, cyano, diazo, ester, formanilido, halo, haloalkyl, hydroxy, imino, isocyano, isonitrilo, nitrilo, nitro, nitroso, phenoxy, sulfhydryl, sulfonylsulfoxy, thio, thioalkyl, thiocarbonyl, thiocyano, thioester, thioformamido, trifluoromethyl, and carboxylic and heterocyclic moieties, including alicyclic and aromatic structures; wherein the individual ring size is 5-8 members; wherein said heterocyclic ring contains 1-6 heteroatom(s) independently selected from the group consisting of O, N, and S; and wherein said aromatic or tertiary alkyl amine is optionally oxidized to a corresponding N-oxide;
Z is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any carbon atom of said alkyl or alkenyl is optionally replaced with O, NH, NR₃, S, SO, or SO₂;
C and D are independently hydrogen, Ar, C₁-C₆ straight or branched chain alkyl, or C₂-C₆ straight or branched chain alkenyl; wherein said alkyl or alkenyl is optionally substituted with one or more substituent(s) independently selected from the group consisting of C₃-C₈ cycloalkyl, C₅-C₇ cycloalkenyl, hydroxy, carbonyl oxygen, and Ar; wherein said alkyl, alkenyl, cycloalkyl or cycloalkenyl is optionally substituted with C₁-C₆ alkyl, C₂-C₆ alkenyl, hydroxy, amino, halo, haloalkyl, thiocarbonyl, ester, thioester, alkoxy, alkenoxy, cyano, nitro, imino, alkylamino, aminoalkyl, sulfhydryl, thioalkyl, or sulfonyl; wherein any carbon atom of said alkyl or alkenyl is optionally substituted in one or more position(s) with oxygen to form a carbonyl; or wherein any carbon atom of said alkyl or alkenyl is optionally replaced with O, NH, NR₃, S, SO, or SO₂;
W is O or S; and
U is either O or N, provided that:
   when U is O, then R₁ is a lone pair of electrons and R₂ is selected from the group consisting of Ar, C₃-C₈ cycloalkyl, C₁-C₆ straight or branched chain alkyl, and C₂-C₆ straight or branched chain alkenyl, wherein said alkyl or alkenyl is optionally substituted with one or more substituent(s) independently selected from the group consisting of Ar and C₃-C₈ cycloalkyl; and
   when U is N, then R₁ and R₂ are independently selected from the group consisting of hydrogen, Ar, C₃-C₈ cycloalkyl, C₁-C₆ straight or branched chain alkyl, and C₂-C₆ straight or branched chain alkenyl, wherein said alkyl or alkenyl is substituted with one or more substituent(s) independently selected from the group consisting of Ar and C₃-C₈ cycloalkyl; or R₁ and R₂ are taken together to form a heterocyclic 5 or 6 membered ring selected from the group consisting of pyrrolidine, imidazolidine, pyrazolidine, piperidine, and piperazine.

Useful carbo- and heterocyclic rings include without limitation phenyl, benzyl, naphthyl, indenyl, azulenyl, fluorenyl, anthracenyl, indolyl, isoindolyl, indolinyl, benzofuranyl, benzothiophenyl, indazolyl, benzimidazolyl, benzthiazolyl, tetrahydrofuranyl, tetrahydropyranyl, pyridyl, pyrrolyl, pyrrolidinyl, pyridinyl, pyrimidinyl, purinyl, quinolinyl, isoquinolinyl, tetrahydroquinolinyl, quinolizinyl, furyl, thiophenyl, imidazolyl, oxazolyl, benzoxazolyl, thiazolyl, isoxazolyl, isotriazolyl, oxadiazolyl, triazolyl, thiadiazolyl, pyridazinyl, pyrimidinyl, pyrazinyl, triazinyl, trithianyl, indolizinyl, pyrazolyl, pyrazolinyl, pyrazolidinyl, thienyl, tetrahydroisoquinolinyl, cinnolinyl, phthalazinyl, quinazolinyl, quinoxalinyl, naphthyridinyl, pteridinyl, carbazolyl, acridinyl, phenazinyl, phenothiazinyl, and phenoxazinyl.

In a preferred embodiment of formula XV, Ar is selected from the group consisting of phenyl, benzyl, naphthyl, indolyl, pyridyl, pyrrolyl, pyrrolidinyl, pyridinyl, pyrimidinyl, purinyl, quinolinyl, isoquinolinyl, furyl, fluorenyl, thiophenyl, imidazolyl, oxazolyl, thiazolyl, pyrazolyl, and thienyl.

### FORMULA XVI

Moreover, the non-immunosuppressive neuroimmunophilin FKBP ligand may be a compound of formula XVI 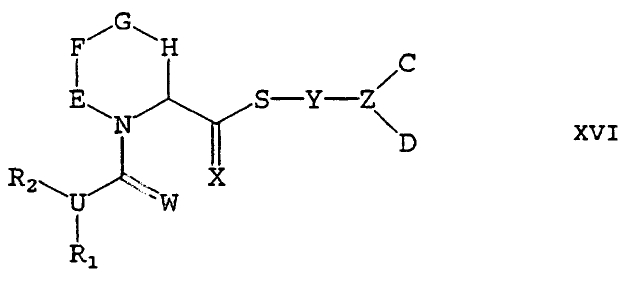 or a pharmaceutically acceptable salt, ester, or solvate thereof, wherein:
E, F, G and J are independently CH₂, O, S, SO, SO₂, NH, or NR₃;
X is either O or S;
Y is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any carbon atom of said alkyl or alkenyl is optionally replaced with O, NH, NR₃, S, SO, or SO₂;
R₃ is selected from the group consisting of hydrogen, C₁-C₄ straight or branched chain alkyl, C₃-C₄ straight or branched chain alkenyl or alkynyl, and C₁-C₄ bridging alkyl wherein a bridge is formed between the nitrogen and a carbon atom of said alkyl or alkenyl chain containing said heteroatom to form a ring, wherein said ring is optionally fused to an Ar group;
Ar is an alicyclic or aromatic, mono-, bi- or tricyclic, carbo- or heterocyclic ring, wherein the ring is either unsubstituted or substituted with one or more substituent(s) independently selected from the group consisting of alkylamino, amido, amino, aminoalkyl, azo, benzyloxy, C₁-C₉ straight or branched chain alkyl, C₁-C₉ alkoxy, C₂-C₉ alkenyloxy, C₂-C₉ straight or branched chain alkenyl, C₃-C₈ cycloalkyl, C₅-C₇ cycloalkenyl, carbonyl, carboxy, cyano, diazo, ester, formanilido, halo, haloalkyl, hydroxy, imino, isocyano, isonitrilo, nitrilo, nitro, nitroso, phenoxy, sulfhydryl, sulfonylsulfoxy, thio, thioalkyl, thiocarbonyl, thiocyano, thioester, thioformamido, trifluoromethyl, and carboxylic and heterocyclic moieties, including alicyclic and aromatic structures; wherein the individual ring size is 5-8 members; wherein said heterocyclic ring contains 1-6 heteroatom(s) independently selected from the group consisting of O, N, and S; and wherein said aromatic or tertiary alkyl amine is optionally oxidized to a corresponding N-oxide;
Z is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any carbon atom of said alkyl or alkenyl is optionally replaced with O, NH, NR₃, S, SO, or SO₂;
C and D are independently hydrogen, Ar, C₁-C₆ straight or branched chain alkyl, or C₂-C₆ straight or branched chain alkenyl; wherein said alkyl or alkenyl is optionally substituted with one or more substituent(s) independently selected from the group consisting of C₃-C₈ cycloalkyl, C₅-C₇ cycloalkenyl, hydroxy, carbonyl oxygen, and Ar; wherein said alkyl, alkenyl, cycloalkyl or cycloalkenyl is optionally substituted with C₁-C₆ alkyl, C₂-C₆ alkenyl, hydroxy, amino, halo, haloalkyl, thiocarbonyl, ester, thioester, alkoxy, alkenoxy, cyano, nitro, imino, alkylamino, aminoalkyl, sulfhydryl, thioalkyl, or sulfonyl; wherein any carbon atom of said alkyl or alkenyl is optionally substituted in one or more position(s) with oxygen to form a carbonyl; or wherein any carbon atom of said alkyl or alkenyl is optionally replaced with O, NH, NR₃, S, SO, or SO₂;
W is O or S; and
U is either O or N, provided that:
   when U is O, then R₁ is a lone pair of electrons and R₂ is selected from the group consisting of Ar, C₃-C₈ cycloalkyl, C₁-C₆ straight or branched chain alkyl, and C₂-C₆ straight or branched chain alkenyl, wherein said alkyl or alkenyl is optionally substituted with one or more substituent(s) independently selected from the group consisting of Ar and C₃-C₈ cycloalkyl; and
   when U is N, then R₁ and R₂ are independently selected from the group consisting of hydrogen, Ar, C₃-C₈ cycloalkyl, C₁-C₆ straight or branched chain alkyl, and C₂-C₆ straight or branched chain alkenyl, wherein said alkyl or alkenyl is optionally substituted with one or more substituent(s) independently selected from the group consisting of Ar and C₃-C₈ cycloalkyl; or R₁ and R₂ are taken together to form a heterocyclic 5 or 6 membered ring selected from the group consisting of pyrrolidine, imidazolidine, pyrazolidine, piperidine, and piperazine.

Useful carbo- and heterocyclic rings include without limitation phenyl, benzyl, naphthyl, indenyl, azulenyl, fluorenyl, anthracenyl, indolyl, isoindolyl, indolinyl, benzofuranyl, benzothiophenyl, indazolyl, benzimidazolyl, benzthiazolyl, tetrahydrofuranyl, tetrahydropyranyl, pyridyl, pyrrolyl, pyrrolidinyl, pyridinyl, pyrimidinyl, purinyl, quinolinyl, isoquinolinyl, tetrahydroquinolinyl, quinolizinyl, furyl, thiophenyl, imidazolyl, oxazolyl, benzoxazolyl, thiazolyl, isoxazolyl, isotriazolyl, oxadiazolyl, triazolyl, thiadiazolyl, pyridazinyl, pyrimidinyl, pyrazinyl, triazinyl, trithianyl, indolizinyl, pyrazolyl, pyrazolinyl, pyrazolidinyl, thienyl, tetrahydroisoquinolinyl, cinnolinyl, phthalazinyl, quinazolinyl, quinoxalinyl, naphthyridinyl, pteridinyl, carbazolyl, acridinyl, phenazinyl, phenothiazinyl, and phenoxazinyl.

In a preferred embodiment of formula XVI, Ar is selected from the group consisting of phenyl, benzyl, naphthyl, pyrrolyl, pyrrolidinyl, pyridinyl, pyrimidinyl, purinyl, quinolinyl, isoquinolinyl, furyl, thiophenyl, imidazolyl, oxazolyl, thiazolyl, pyrazolyl, and thienyl.

### FORMULA XVII

The non-immunosuppressive neuroimmunophilin FKBP ligand may also be a compound of formula XVII 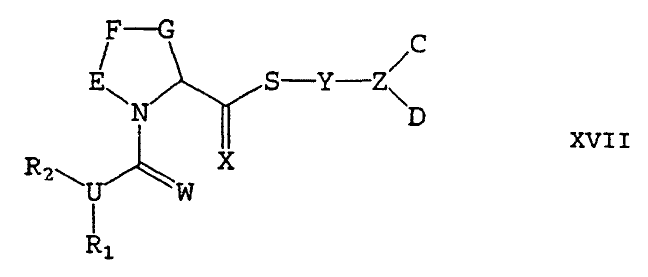 or a pharmaceutically acceptable salt, ester, or solvate thereof, wherein:
E, F, and G are independently CH₂, O, S, SO, SO₂, NH, and NR₃;
X is either O or S;
Y is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any carbon atom of said alkyl or alkenyl is optionally replaced with O, NH, NR₃, S, SO, or SO₂;
R₃ is selected from the group consisting of hydrogen, C₁-C₄ straight or branched chain alkyl, C₃-C₄ straight or branched chain alkenyl or alkynyl, and C₁-C₄ bridging alkyl wherein a bridge is formed between the nitrogen and a carbon atom of said alkyl or alkenyl chain containing said heteroatom to form a ring, wherein said ring is optionally fused to an Ar group;
Ar is an alicyclic or aromatic, mono-, bi- or tricyclic, carbo- or heterocyclic ring, wherein the ring is either unsubstituted or substituted with one or more substituent (s) independently selected from the group consisting of alkylamino, amido, amino, aminoalkyl, azo, benzyloxy, C₁-C₉ straight or branched chain alkyl, C₁-C₉ alkoxy, C₂-C₉ alkenyloxy, C₂-C₉ straight or branched chain alkenyl, C₃-C₈ cycloalkyl, C₅-C₇ cycloalkenyl, carbonyl, carboxy, cyano, diazo, ester, formanilido, halo, haloalkyl, hydroxy, imino, isocyano, isonitrilo, nitrilo, nitro, nitroso, phenoxy, sulfhydryl, sulfonylsulfoxy, thio, thioalkyl, thiocarbonyl, thiocyano, thioester, thioformamido, trifluoromethyl, and carboxylic and heterocyclic moieties, including alicyclic and aromatic structures; wherein the individual ring size is 5-8 members; wherein said heterocyclic ring contains 1-6 heteroatom(s) independently selected from the group consisting of O, N, and S; and wherein said aromatic or tertiary alkyl amine is optionally oxidized to a corresponding N-oxide;
Z is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any carbon atom of said alkyl or alkenyl is optionally replaced with O, NH, NR₃, S, SO, or SO₂;
C and D are independently hydrogen, Ar, C₁-C₆ straight or branched chain alkyl, or C₂-C₆ straight or branched chain alkenyl; wherein said alkyl or alkenyl is optionally substituted with one or more substituent(s) independently selected from the group consisting of C₃-C₈ cycloalkyl, C₅-C₇ cycloalkenyl, hydroxy, carbonyl oxygen, and Ar; wherein said alkyl, alkenyl, cycloalkyl or cycloalkenyl is optionally substituted with C₁-C₆ alkyl, C₂-C₆ alkenyl, hydroxy, amino, halo, haloalkyl, thiocarbonyl, ester, thioester, alkoxy, alkenoxy, cyano, nitro, imino, alkylamino, aminoalkyl, sulfhydryl, thioalkyl, or sulfonyl; wherein any carbon atom of said alkyl or alkenyl is optionally substituted in one or more position(s) with oxygen to form a carbonyl; or wherein any carbon atom of said alkyl or alkenyl is optionally replaced with O, NH, NR₃, S, SO, or SO₂;
W is O or S; and
U is either O or N, provided that:
   when U is O, then R₁ is a lone pair of electrons and R₂ is selected from the group consisting of Ar, C₃-C₈ cycloalkyl, C₁-C₆ straight or branched chain alkyl, and C₂-C₆ straight or branched chain alkenyl, wherein said alkyl or alkenyl is optionally substituted with one or more substituent(s) independently selected from the group consisting of Ar and C₃-C₈ cycloalkyl; and
   when U is N, then R₁ and R₂ are independently selected from the group consisting of hydrogen, Ar, C₃-C₈ cycloalkyl, C₁-C₆ straight or branched chain alkyl, and C₂-C₆ straight or branched chain alkenyl, wherein said alkyl or alkenyl is optionally substituted with one or more substituent(s) independently selected from the group consisting of Ar and C₃-C₈ cycloalkyl; or R₁ and R₂ are taken together to form a heterocyclic 5 or 6 membered ring selected from the group consisting of pyrrolidine imidazolidine, pyrazolidine, piperidine, and piperazine.

Useful carbo- and heterocyclic rings include without limitation phenyl, benzyl, naphthyl, indenyl, azulenyl, fluorenyl, anthracenyl, indolyl, isoindolyl, indolinyl, benzofuranyl, benzothiophenyl, indazolyl, benzimidazolyl, benzthiazolyl, tetrahydrofuranyl, tetrahydropyranyl, pyridyl, pyrrolyl, pyrrolidinyl, pyridinyl, pyrimidinyl, purinyl, quinolinyl, isoquinolinyl, tetrahydroquinolinyl, quinolizinyl, furyl, thiophenyl, imidazolyl, oxazolyl, benzoxazolyl, thiazolyl, isoxazolyl, isotriazolyl, oxadiazolyl, triazolyl, thiadiazolyl, pyridazinyl, pyrimidinyl, pyrazinyl, triazinyl, trithianyl, indolizinyl, pyrazolyl, pyrazolinyl, pyrazolidinyl, thienyl, tetrahydroisoquinolinyl, cinnolinyl, phthalazinyl, quinazolinyl, quinoxalinyl, naphthyridinyl, pteridinyl, carbazolyl, acridinyl, phenazinyl, phenothiazinyl, and phenoxazinyl.

In a preferred embodiment of formula XVII, Ar is selected from the group consisting of phenyl, benzyl, naphthyl, pyrrolyl, pyrrolidinyl, pyridinyl, pyrimidinyl, purinyl, quinolinyl, isoquinolinyl, furyl, thiophenyl, imidazolyl, oxazolyl, thiazolyl, pyrazolyl, and thienyl.

### FORMULA XVIII

The non-immunosuppressive neuroimmunophilin FKBP ligand may further be a compound of formula XVIII 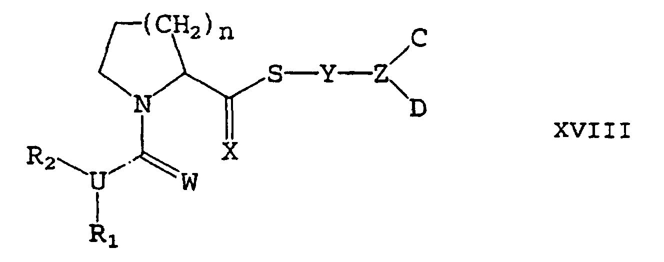 or a pharmaceutically acceptable salt, ester, or solvate thereof, wherein:
n is 1, 2 or 3;
X is either O or S;
Y is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any carbon atom of said alkyl or alkenyl is optionally replaced with O, NH, NR₃, S, SO, or SO₂;
R₃ is selected from the group consisting of hydrogen, C₁-C₄ straight or branched chain alkyl, C₃-C₄ straight or branched chain alkenyl or alkynyl, and C₁-C₄ bridging alkyl wherein a bridge is formed between the nitrogen and a carbon atom of said alkyl or alkenyl chain containing said heteroatom to, form a ring, wherein said ring is optionally fused to an Ar group;
Ar is an alicyclic or aromatic, mono-, bi- or tricyclic, carbo- or heterocyclic ring, wherein the ring is either unsubstituted or substituted with one or more substituent(s) independently selected from the group consisting of alkylamino, amido, amino, aminoalkyl, azo, benzyloxy, C₁-C₉ straight or branched chain alkyl, C₁-C₉ alkoxy, C₂-C₉ alkenyloxy, C₂-C₉ straight or branched chain alkenyl, C₃-C₈ cycloalkyl, C₅-C₇ cycloalkenyl, carbonyl, carboxy, cyano, diazo, ester, formanilido, halo, haloalkyl, hydroxy, imino, isocyano, isonitrilo, nitrilo, nitro, nitroso, phenoxy, sulfhydryl, sulfonylsulfoxy, thio, thioalkyl, thiocarbonyl, thiocyano, thioester, thioformamido, trifluoromethyl, and carboxylic and heterocyclic moieties, including alicyclic and aromatic structures; wherein the individual ring size is 5-8 members; wherein said heterocyclic ring contains 1-6 heteroatom(s) independently selected from the group consisting of O, N, and S; and wherein said aromatic or tertiary alkyl amine is optionally oxidized to a corresponding N-oxide;
Z is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any carbon atom of said alkyl or alkenyl is optionally replaced with O, NH, NR₃, S, SO, or SO₂;
C and D are independently hydrogen, Ar, C₁-C₆ straight or branched chain alkyl, or C₂-C₆ straight or branched chain alkenyl; wherein said alkyl or alkenyl is optionally substituted with one or more substituent(s) independently selected from the group consisting of C₃-C₈ cycloalkyl, C₅-C₇ cycloalkenyl, hydroxy, carbonyl oxygen, and Ar; wherein said alkyl, alkenyl, cycloalkyl or cycloalkenyl is optionally substituted with C₁-C₆ alkyl, C₂-C₆ alkenyl, hydroxy, amino, halo, haloalkyl, thiocarbonyl, ester, thioester, alkoxy, alkenoxy, cyano, nitro, imino, alkylamino, aminoalkyl, sulfhydryl, thioalkyl, or sulfonyl; wherein any carbon atom of said alkyl or alkenyl is optionally substituted in one or more position(s) with oxygen to form a carbonyl; or wherein any carbon atom of said alkyl or alkenyl is optionally replaced with O, NH, NR₃, S, SO, or SO₂;
W is 0 or S; and
U is either O or N, provided that:
   when U is O, then R₁ is a lone pair of electrons and R₂ is selected from the group consisting of Ar, C₃-C₈ cycloalkyl, C₁-C₆ straight or branched chain alkyl, and C₂-C₆ straight or branched chain or alkenyl, wherein said alkyl or alkenyl is optionally substituted with one or more substituent(s) independently selected from the group consisting of Ar and C₃-C₈ cycloalkyl; and
   when U is N, then R₁ and R₂ are independently selected from the group consisting of hydrogen, Ar, C₃-C₈ cycloalkyl, C₁-C₆ straight or branched chain alkyl, and C₂-C₆ straight or branched chain alkenyl, wherein said alkyl or alkenyl is optionally substituted with one or more substituent(s) independently selected from the group consisting of Ar and C₃-C₈ cycloalkyl; or R₁ and R₂ are taken together to form a heterocyclic 5 or 6 membered ring selected from the group consisting of pyrrolidine, imidazolidine, pyrazolidine, piperidine, and piperazine.

Useful carbo- and heterocyclic rings include without limitation phenyl, benzyl, naphthyl, indenyl, azulenyl, fluorenyl, anthracenyl, indolyl, isoindolyl, indolinyl, benzofuranyl, benzothiophenyl, indazolyl, benzimidazolyl, benzthiazolyl, tetrahydrofuranyl, tetrahydropyranyl, pyridyl, pyrrolyl, pyrrolidinyl, pyridinyl, pyrimidinyl, purinyl, quinolinyl, isoquinolinyl, tetrahydroquinolinyl, quinolizinyl, furyl, thiophenyl, imidazolyl, oxazolyl, benzoxazolyl, thiazolyl, isoxazolyl, isotriazalyl, oxadiazolyl, triazolyl, thiadiazolyl, pyridazinyl, pyrimidinyl, pyrazinyl, triazinyl, trithianyl, indolizinyl, pyrazolyl, pyrazolinyl, pyrazolidinyl, thienyl, tetrahydroisoquinolinyl, cinnolinyl, phthalazinyl, quinazolinyl, quinoxalinyl, naphthyridinyl, pteridinyl, carbazolyl, acridinyl, phenazinyl, phenothiazinyl, and phenoxazinyl.

In a preferred embodiment of formula XVIII, Ar is selected from the group consisting of phenyl, benzyl, naphthyl, pyrrolyl, pyrrolidinyl, pyridinyl, pyrimidinyl, purinyl, quinolinyl, isoquinolinyl, furyl, thiophenyl, imidazolyl, oxazolyl, thiazolyl, pyrazolyl, and thienyl.

Exemplary compounds of formula XVIII are presented in TABLE VII.

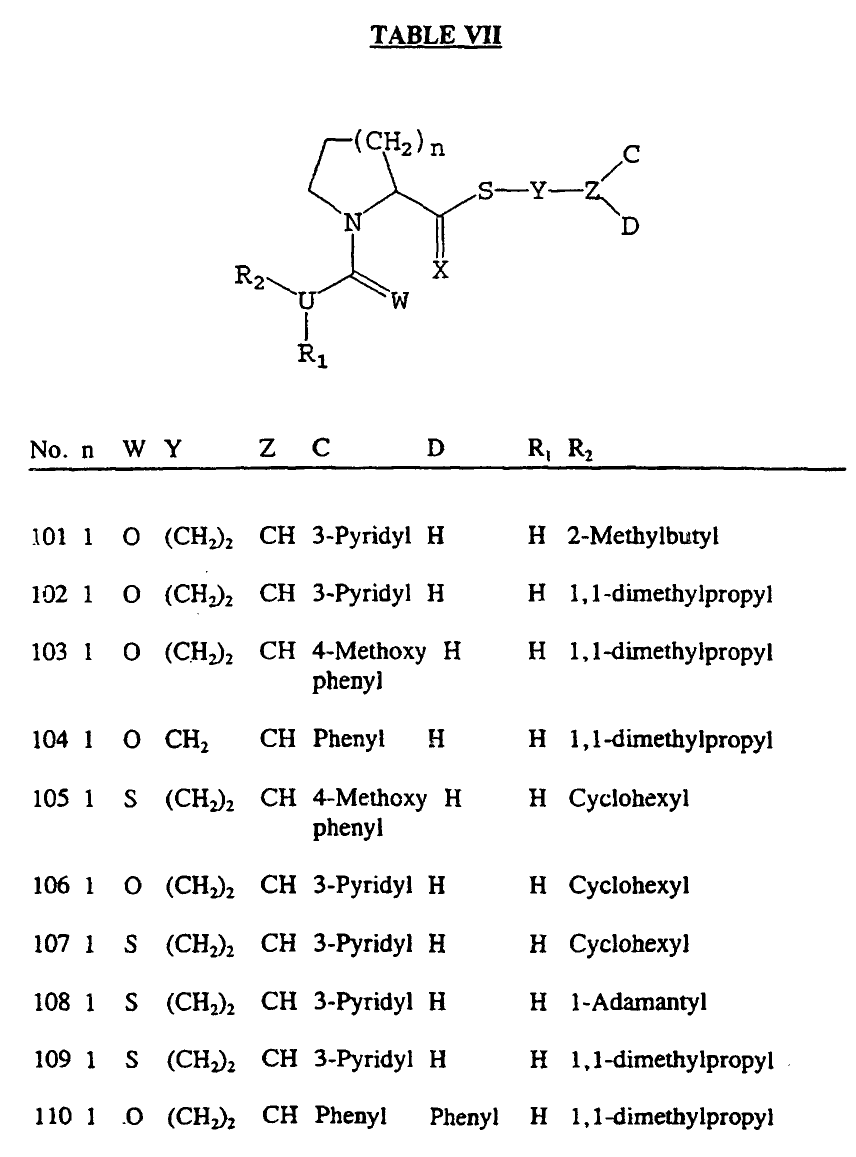

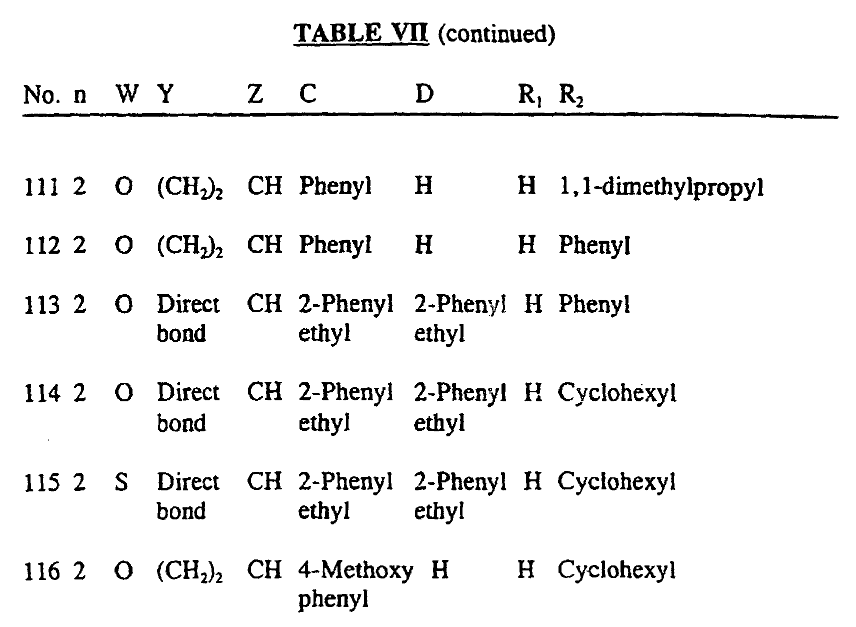

The most preferred compounds of formula XVIII are selected from the group consisting of:
3-(3-Pyridyl)-1-propyl-2S-1-[(2-methylbutyl) carbamoyl]pyrrolidine-2-carboxylate;
3-(3-Pyridyl)-1-propyl-2S-1-[(1',1'-Dimethylpropyl) carbamoyl]pyrrolidine-2-carboxylate;
3-(3-Pyridyl)-1-propyl-2S-1-[(cyclohexyl) thiocarbamoyl]pyrrolidine-2-carboxylate; and
pharmaceutically acceptable salts, esters, and solvates thereof.

### FORMULA XIX

Additionally, the non-immunosuppressive neuroimmunophilin FKBP ligand may be a compound of formula XIX 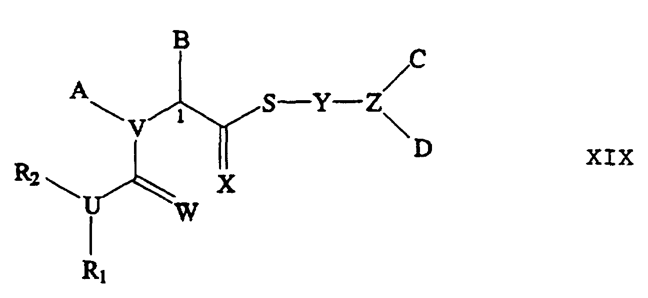 or a pharmaceutically acceptable salt', ester, or solvate thereof, wherein:
V is C, N, or S;
Y is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any carbon atom of said alkyl or alkenyl is optionally replaced with O, NH, NR₃, S, SO, or SO₂;
R₃ is selected from the group consisting of hydrogen, C₁-C₆ straight or branched chain alkyl, C₃-C₆ straight or branched chain alkenyl or alkynyl, and C₁-C₄ bridging alkyl wherein a bridge is formed between the nitrogen and a carbon atom of said alkyl or alkenyl chain containing said heteroatom to form a ring, wherein said ring is optionally fused to an Ar group;
Ar is an alicyclic or aromatic, mono-, bi- or tricyclic, carbo- or heterocyclic ring, wherein the ring is either unsubstituted or substituted with one or more substituent(s); wherein the individual ring size is 5-8 members; wherein said heterocyclic ring contains 1-6 heteroatom(s) independently selected from the group consisting of O, N, and S; and wherein said aromatic or tertiary alkyl amine is optionally oxidized to a corresponding N-oxide;
Z is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any carbon atom of said alkyl or alkenyl is optionally replaced with O, NH, NR₃, S, SO, or SO₂;
C and D are independently hydrogen, Ar, C₁-C₆ straight or branched chain alkyl, or C₂-C₆ straight or branched chain alkenyl; wherein said alkyl or alkenyl is optionally substituted with one or more substituent(s) independently selected from the group consisting of C₃-C₈ cycloalkyl, C₅-C₇ cycloalkenyl, hydroxy, carbonyl oxygen, and Ar; wherein said alkyl, alkenyl, cycloalkyl or cycloalkenyl is optionally substituted with C₁-C₆ alkyl, C₂-C₆ alkenyl, hydroxy, amino, halo, haloalkyl, thiocarbonyl, ester, thioester, alkoxy, alkenoxy, cyano, nitro, imino, alkylamino, aminoalkyl, sulfhydryl, thioalkyl, or sulfonyl; wherein any carbon atom of said alkyl or alkenyl is optionally substituted in one or more position(s) with oxygen to form a carbonyl; or wherein any carbon atom of said alkyl or alkenyl is optionally replaced with O, NH, NR₃, S, SO, or SO₂; and
A, B, R₁, R₂, U, W, and X are as otherwise defined in formula XV.

### V. N-LINKED SULFONAMIDES OF HETEROCYCLIC THIOESTERS

### FORMULA XX

The non-immunosuppressive neuroimmunophilin FKBP ligand may further be a compound of formula XX 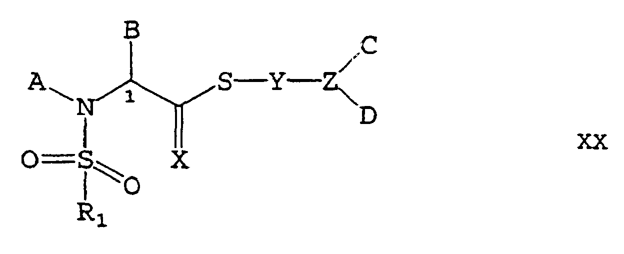 or a pharmaceutically acceptable salt, ester, or solvate thereof, wherein:
A and B, together with the nitrogen and carbon atoms to which they are respectively attached, form a 5-7 membered saturated or unsaturated heterocyclic ring containing, in addition to the nitrogen atom, one or more heteroatom(s) independently selected from the group consisting of O, S, SO, SO₂, N, NH, and NR₂;
X is either O or S;
Y is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any carbon atom of said alkyl or alkenyl is optionally replaced with O, NH, NR₃, S, SO, or SO₂;
R₂ is selected from the group consisting of hydrogen, C₁-C₄ straight or branched chain alkyl, C₃-C₄ straight or branched chain alkenyl or alkynyl, and C₃-C₄ bridging alkyl wherein a bridge is formed between the nitrogen and a carbon atom of said alkyl or alkenyl chain containing said heteroatom to form a ring, wherein said ring is optionally fused to an Ar group;
Ar is an alicyclic or aromatic, mono-, bi- or tricyclic, carbo- or heterocyclic ring, wherein the ring is either unsubstituted or substituted with one or more substituent(s); wherein the individual ring size is 5-8 members; wherein the heterocyclic ring contains 1-6 heteroatom(s) independently selected from the group consisting of O, N, and S; wherein an aromatic or tertiary alkyl amine is optionally oxidized to a corresponding N-oxide;
Z is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any atom of said alkyl or alkenyl is optionally replaced with O, NH, NR₂, S, SO, or SO₂;
C and D are independently hydrogen, Ar, C₁-C₆ straight or branched chain alkyl, or C₂-C₆ straight or branched chain alkenyl; wherein said alkyl or alkenyl is optionally substituted with one or more substituent(s) independently selected from the group consisting of C₃-C₈ cycloalkyl, C₅-C₇ cycloalkenyl, hydroxy, carbonyl oxygen, and Ar; wherein said alkyl, alkenyl, cycloalkyl or cycloalkenyl is optionally substituted with C₁-C₆ alkyl, C₂-C₆ alkenyl, hydroxy, amino, halo, haloalkyl, thiocarbonyl, ester, thioester, alkoxy, alkenoxy, cyano, nitro, imino, alkylamino, aminoalkyl, sulfhydryl, thioalkyl, or sulfonyl; wherein any carbon atom of said alkyl or alkenyl is optionally substituted in one or more position(s) with oxygen to form a carbonyl; or wherein any carbon atom of said alkyl or alkenyl is optionally replaced with O, NH, NR₃, S, SO, or SO₂; and
R₁ is selected from the group consisting of Ar, C₃-C₈ cycloalkyl, C₁-C₆ straight or branched chain alkyl, and C₂-C₆ straight or branched chain alkenyl, wherein said alkyl or alkenyl is optionally substituted with one or more substituent(s) independently selected from the group consisting of Ar, C₃-C₈ cycloalkyl, amino, halo, haloalkyl, hydroxy, trifluoromethyl, C₁-C₆ straight or branched chain alkyl, C₂-C₆ straight or branched chain alkenyl, carbonyl, thiocarbonyl, ester, thioester, alkoxy, alkenoxy, cyano, nitro, imino, alkylamino, aminoalkyl, sulfhydryl, thioalkyl, and sulfonyl, wherein any carbon atom of said alkyl or alkenyl is optionally replaced with O, NH, NR₃, S, SO, or SO₂.

Useful carbo- and heterocyclic rings include without limitation phenyl, benzyl, naphthyl, indenyl, azulenyl, fluorenyl, anthracenyl, indolyl, isoindolyl, indolinyl, benzofuranyl, benzothiophenyl, indazolyl, benzimidazolyl, benzthiazolyl, tetrahydrofuranyl, tetrahydropyranyl, pyridyl, pyrrolyl, pyrrolidinyl, pyridinyl, pyrimidinyl, purinyl, quinolinyl, isoquinolinyl, tetrahydroquinolinyl, quinolizinyl, furyl, thiophenyl, imidazolyl, oxazolyl, benzoxazolyl, thiazolyl, isoxazolyl, isotriazolyl, oxadiazolyl, triazolyl, thiadiazolyl, pyridazinyl, pyrimidinyl, pyrazinyl, triazinyl, trithianyl, indolizinyl, pyrazolyl, pyrazolinyl, pyrazolidinyl, thienyl, tetrahydroisoquinolinyl, cinnolinyl, phthalazinyl, quinazolinyl, quinoxalinyl, naphthyridinyl, pteridinyl, carbazolyl, acridinyl, phenazinyl, phenothiazinyl, and phenoxazinyl.

In a preferred embodiment of formula XX, Ar is selected from the group consisting of phenyl, benzyl, naphthyl, indolyl, pyridyl, pyrrolyl, pyrrolidinyl, pyridinyl, pyrimidinyl, purinyl, quinolinyl, isoquinolinyl, furyl, fluorenyl, thiophenyl, imidazolyl, oxazolyl, thiazolyl, pyrazolyl, and thienyl.

In another preferred embodiment of formula XX, A and B, together with the nitrogen and carbon atoms to which they are respectfully attached, form a 6 membered saturated or unsaturated heterocyclic ring; and R₂ is C₄-C₇ branched chain alkyl, C₄-C₇ cycloalkyl, phenyl, or 3,4,5-trimethoxyphenyl.

In the most preferred embodiment of formula XX, the compound is selected from the group consisting of:
3-(*para*-Methoxyphenyl)-1-propylmercaptyl(2*S*)-N-(benzenesulfonyl)pyrrolidine-2-carboxylate;
3-(*para*-Methoxyphenyl)-1-propylmercaptyl(2*S*)-N-(α-toluenesulfonyl)pyrrolidine-2-carboxylate;
3-(*para*-Methoxyphenyl)-1-propylmercaptyl(2*S*)-N-(α-toluenesulfonyl)pyrrolidine-2-carboxylate;
1,5-Diphenyl-3-pentylmercaptyl N-(*para*-toluenesulfonyl)pipecolate; and
pharmaceutically acceptable salts, esters, and solvates thereof.

### FORMULA XXI

Moreover, the non-immunosuppressive neuroimmunophilin FKBP ligand may be a compound of formula XXI 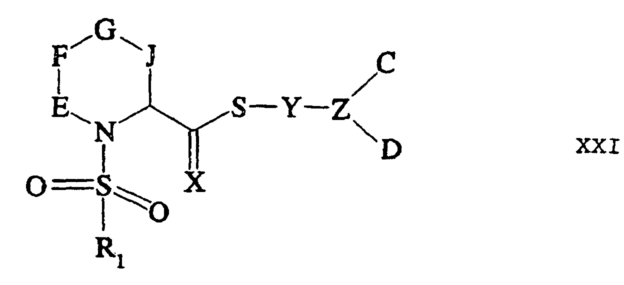 or a pharmaceutically acceptable salt, ester, or solvate thereof, wherein:
E, F, G and J are independently CH₂, O, S, SO, SO₂, NH or NR₂;
X is either O or S;
Y is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any carbon atom of said alkyl or alkenyl is optionally replaced with O, NH, NR₃, S, SO, or SO₂;
R₂ is selected from the group consisting of hydrogen, C₁-C₄ straight or branched chain alkyl, C₃-C₄ straight or branched chain alkenyl or alkynyl, and C₁-C₄ bridging alkyl wherein a bridge is formed between the nitrogen and a carbon atom of said alkyl or alkenyl chain containing said heteroatom to form a ring, wherein said ring is optionally fused to an Ar group;
Z is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any atom of said alkyl or alkenyl is optionally replaced with O, NH, NR₂, S, SO, or SO₂;
Ar is an alicyclic or aromatic, mono-, bi- or tricyclic, carbo- or heterocyclic ring, wherein the ring is either unsubstituted or substituted with one or more substituent(s); wherein the individual ring size is 5-8 members; wherein the heterocyclic ring contains 1-6 heteroatom(s) independently selected from the group consisting of O, N, and S; wherein an aromatic or tertiary alkyl amine is optionally oxidized to a corresponding N-oxide;
C and D are independently hydrogen, Ar, C₁-C₆ straight or branched chain alkyl, or C₂-C₆ straight or branched chain alkenyl; wherein said alkyl or alkenyl is optionally substituted with one or more substituent(s) independently selected from the group consisting of C₃-C₈ cycloalkyl, C₅-C₇ cycloalkenyl, hydroxy, carbonyl oxygen, and Ar; wherein said alkyl, alkenyl, cycloalkyl or cycloalkenyl is optionally substituted with C₁-C₆ alkyl, C₂-C₆ alkenyl, hydroxy, amino, halo, haloalkyl, thiocarbonyl, ester, thioester, alkoxy, alkenoxy, cyano, nitro, imino, alkylamino, aminoalkyl, sulfhydryl, thioalkyl, or sulfonyl; wherein any carbon atom of said alkyl or alkenyl is optionally substituted in one or more position(s) with oxygen to form a carbonyl; or wherein any carbon atom of said alkyl or alkenyl is optionally replaced with O, NH, NR₃, S, SO, or SO₂, and
R₁ is selected from the group consisting of Ar, C₃-C₈ cycloalkyl, C₁-C₆ straight or branched chain alkyl, and C₂-C₆ straight or branched chain alkenyl, wherein said alkyl or alkenyl is optionally substituted with one or more substituent(s) independently selected from the group consisting of Ar, C₃-C₈ cycloalkyl, amino, halo, haloalkyl, hydroxy, trifluoromethyl, C₁-C₆ straight or branched chain alkyl, C₂-C₆ straight or branched chain alkenyl, carbonyl, thiocarbonyl, ester, thioester, alkoxy, alkenoxy, cyano, nitro, imino, alkylamino, aminoalkyl, sulfhydryl, thioalkyl, and sulfonyl, wherein any carbon atom of said alkyl or alkenyl is optionally replaced with O, NH, NR₃, S, SO, or SO₂.

Useful carbo- and heterocyclic rings include without limitation phenyl, benzyl, naphthyl, indenyl, azulenyl, fluorenyl, anthracenyl, indolyl, isoindolyl, indolinyl, benzofuranyl, benzothiophenyl, indazolyl, benzimidazolyl, benzthiazolyl, tetrahydrofuranyl, tetrahydropyranyl, pyridyl, pyrrolyl, pyrrolidinyl, pyridinyl, pyrimidinyl, purinyl, quinolinyl, isoquinolinyl, tetrahydroquinolinyl, quinolizinyl, furyl, thiophenyl, imidazolyl, oxazolyl, benzoxazolyl, thiazolyl, isoxazolyl, isotriazolyl, oxadiazolyl, triazolyl, thiadiazolyl, pyridazinyl, pyrimidinyl, pyrazinyl, triazinyl, trithianyl, indolizinyl, pyrazolyl, pyrazolinyl, pyrazolidinyl, thienyl, tetrahydroisoquinolinyl, cinnolinyl, phthalazinyl, quinazolinyl, quinoxalinyl, naphthyridinyl, pteridinyl, carbazolyl, acridinyl, phenazinyl, phenothiazinyl, and phenoxazinyl.

In a preferred embodiment of formula XXI, Ar is selected from the group consisting of phenyl, benzyl, naphthyl, indolyl, pyridyl, pyrrolyl, pyrrolidinyl, pyridinyl, pyrimidinyl, purinyl, quinolinyl, isoquinolinyl, furyl, fluorenyl, thiophenyl, imidazolyl, oxazolyl, thiazolyl, pyrazolyl, and thienyl.

### FORMULA XXII

The non-immunosuppressive neuroimmunophilin FKBP ligand may also be a compound of formula XXII 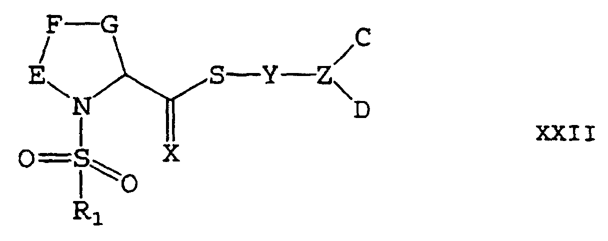 or a pharmaceutically acceptable salt, ester, or solvate thereof, wherein:
E, F, and G are independently CH₂, O, S, SO, SO₂, NH or NR₂;
X is either O or S;
Y is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any carbon atom of said alkyl or alkenyl is optionally replaced with O, NH, NR₂, S, SO, or SO₂;
R₂ is selected from the group consisting of hydrogen, C₁-C₄ straight or branched chain alkyl, C₃-C₄ straight or branched chain alkenyl or alkynyl, and C₁-C₄ bridging alkyl wherein a bridge is formed between the nitrogen and a carbon atom of said alkyl or alkenyl chain containing said heteroatom to form a ring, wherein said ring is optionally fused to an Ar group;
Ar is an alicyclic or aromatic, mono-, bi- or tricyclic, carbo- or heterocyclic ring, wherein the ring is either unsubstituted or substituted with one or more substituent(s); wherein the individual ring size is 5-8 members; wherein the heterocyclic ring contains 1-6 heteroatom(s) independently selected from the group consisting of O, N, and S; wherein an aromatic or tertiary alkyl amine is optionally oxidized to a corresponding N-oxide;
Z is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any atom of said alkyl or alkenyl is optionally replaced with O, NH, NR₂, S, SO, or SO₂;
R₂ is selected from the group consisting of hydrogen, C₁-C₄ straight or branched chain alkyl, C₃-C₄ straight or branched chain alkenyl or alkynyl, and C₁-C₄ bridging alkyl wherein a bridge is formed between the nitrogen and a carbon atom of said alkyl or alkenyl chain containing said heteroatom to form a ring, wherein said ring is optionally fused to an Ar group;
C and D are independently hydrogen, Ar, C₁-C₆ straight or branched chain alkyl, or C₂-C₆ straight or branched chain alkenyl, wherein said alkyl or alkenyl is optionally substituted with one or more substituent(s) independently selected from the group consisting of C₃-C₈ cycloalkyl, C₅-C₇ cycloalkenyl, hydroxy, carbonyl oxygen, and Ar; wherein said alkyl, alkenyl, cycloalkyl or cycloalkenyl is optionally substituted with C₁-C₄ alkyl, C₂-C₄ alkenyl, or hydroxy; wherein any carbon atom of said alkyl or alkenyl is optionally substituted in one or more position(s) with oxygen to form a carbonyl, or wherein any carbon atom of said alkyl or alkenyl is optionally replaced with O, NH, NR₂, S, SO, or SO₂; and
R₁ is selected from the group consisting of Ar, C₃-C₈ cycloalkyl, C₁-C₆ straight or branched chain alkyl, and C₂-C₆ straight or branched chain alkenyl, wherein said alkyl or alkenyl is optionally substituted with one or more substituent(s) independently selected from the group consisting of Ar, C₃-C₈ cycloalkyl, amino, halo, haloalkyl, hydroxy, trifluoromethyl, C₁-C₆ straight or branched chain alkyl, C₂-C₆ straight or branched chain alkenyl, carbonyl, thiocarbonyl, ester, thioester, alkoxy, alkenoxy, cyano, nitro, imino, alkylamino, aminoalkyl, sulfhydryl, thioalkyl, and sulfonyl, wherein any carbon atom of said alkyl or alkenyl is optionally replaced with O, NH, NR₃, S, SO, or SO₂.

Useful carbo- and heterocyclic rings include without limitation phenyl, benzyl, naphthyl, indenyl, azulenyl, fluorenyl, anthracenyl, indolyl, isoindolyl, indolinyl, benzofuranyl, benzothiophenyl, indazolyl, benzimidazolyl, benzthiazolyl, tetrahydrofuranyl, tetrahydropyranyl, pyridyl, pyrrolyl, pyrrolidinyl, pyridinyl, pyrimidinyl, purinyl, quinolinyl, isoquinolinyl, tetrahydroquinolinyl, quinolizinyl, furyl, thiophenyl, imidazolyl, oxazolyl, benzoxazolyl, thiazolyl, isoxazolyl, isotriazolyl, oxadiazolyl, triazolyl, thiadiazolyl, pyridazinyl, pyrimidinyl, pyrazinyl, triazinyl, trithianyl, indolizinyl, pyrazolyl, pyrazolinyl, pyrazolidinyl, thienyl, tetrahydroisoquinolinyl, cinnolinyl, phthalazinyl, quinazolinyl, quinoxalinyl, naphthyridinyl, pteridinyl, carbazolyl, acridinyl, phenazinyl, phenothiazinyl, and phenoxazinyl.

In a preferred embodiment of formula XXII, Ar is selected from the group consisting of phenyl, benzyl, naphthyl, indolyl, pyridyl, pyrrolyl, pyrrolidinyl, pyridinyl, pyrimidinyl, purinyl, quinolinyl, isoquinolinyl, furyl, fluorenyl, thiophenyl, imidazolyl, oxazolyl, thiazolyl, pyrazolyl, and thienyl.

### FORMULA XXIII

Additionally, the non-immunosuppressive neuroimmunophilin FKBP ligand may be a compound of formula XXIII 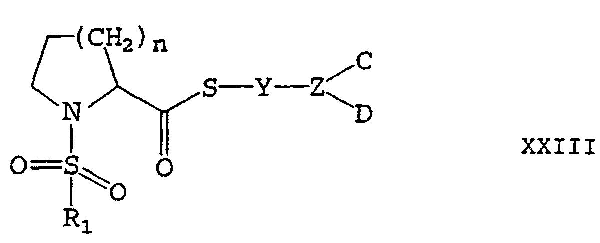 or a pharmaceutically acceptable salt, ester, or solvate thereof, wherein:
n is 1, 2 or 3;
X is either O or S;
Y is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any carbon atom of said alkyl or alkenyl is optionally replaced with O, NH, NR₂, S, SO, or SO₂;
R₂ is selected from the group consisting of hydrogen, C₁-C₄ straight or branched chain alkyl, C₃-C₄ straight or branched chain alkenyl or alkynyl, and C₁-C₄ bridging alkyl wherein a bridge is formed between the nitrogen and a carbon atom of said alkyl or alkenyl chain containing said heteroatom to form a ring, wherein said ring is optionally fused to an Ar group;
Z is a direct bond, C₁-C₆ straight or branched chain alkyl, or C₂-C₆ straight or branched chain alkenyl, wherein any carbon atom of said alkyl or alkenyl is optionally substituted in one or more position(s) with amino, halo, haloalkyl, thiocarbonyl, ester, thioester, alkoxy, alkenoxy, cyano, nitro, imino, alkylamino, aminoalkyl, sulfhydryl, thioalkyl, sulfonyl, or oxygen to form a carbonyl, or wherein any atom of said alkyl or alkenyl is optionally replaced with O, NH, NR₂, S, SO, or SO₂;
R₂ is selected from the group consisting of hydrogen, C₁-C₄ straight or branched chain alkyl, C₃-C₄ straight or branched chain alkenyl or alkynyl, and C₁-C₄ bridging alkyl wherein a bridge is formed between the nitrogen and a carbon atom of said alkyl or alkenyl chain containing said heteroatom to form a ring, wherein said ring is optionally fused to an Ar group;
Ar is an alicyclic or aromatic, mono-, bi- or tricyclic, carbo- or heterocyclic ring, wherein the ring is either unsubstituted or substituted with one or more substituent(s); wherein the individual ring size is 5-8 members; wherein the heterocyclic ring contains 1-6 heteroatom(s) independently selected from the group consisting of O, N, and S; wherein an aromatic or tertiary alkyl amine is optionally oxidized to a corresponding N-oxide;
C and D are independently hydrogen, Ar, C₁-C₆ straight or branched chain alkyl, or C₂-C₆ straight or branched chain alkenyl, wherein said alkyl or alkenyl is optionally substituted with one or more substituent(s) independently selected from the group consisting of C₃-C₈ cycloalkyl, C₅-C₇ cycloalkenyl, hydroxy, carbonyl oxygen, and Ar; wherein said alkyl, alkenyl, cycloalkyl or cycloalkenyl is optionally substituted with C₁-C₄ alkyl, C₂-C₄ alkenyl, or hydroxy; wherein any carbon atom of said alkyl or alkenyl is optionally substituted in one or more position(s) with oxygen to form a carbonyl, or wherein any carbon atom of said alkyl or alkenyl is optionally replaced with O, NH, NR₂, S, SO, or SO₂; and
R₁ is selected from the group consisting of Ar, C₃-C₈ cycloalkyl, C₁-C₆ straight or branched chain alkyl, and C₂-C₆ straight or branched chain alkenyl, wherein said alkyl or alkenyl is optionally substituted with one or more substituent(s) independently selected from the group consisting of Ar, C₃-C₈ cycloalkyl, amino, halo, haloalkyl, hydroxy, trifluoromethyl, C₁-C₆ straight or branched chain alkyl, C₂-C₆ straight or branched chain alkenyl, carbonyl, thiocarbonyl, ester, thioester, alkoxy, alkenoxy, cyano, nitro, imino, alkylamino, aminoalkyl, sulfhydryl, thioalkyl, and sulfonyl, wherein any carbon atom of said alkyl or alkenyl is optionally replaced with O, NH, NR₃, S, SO, or SO₂.

Useful carbo- and heterocyclic rings include without limitation phenyl, benzyl, naphthyl, indenyl, azulenyl, fluorenyl, anthracenyl, indolyl, isoindolyl, indolinyl, benzofuranyl, benzothiophenyl, indazolyl, benzimidazolyl, benzthiazolyl, tetrahydrofuranyl, tetrahydropyranyl, pyridyl, pyrrolyl, pyrrolidinyl, pyridinyl, pyrimidinyl, purinyl, quinolinyl, isoquinolinyl, tetrahydroquinolinyl, quinolizinyl, furyl, thiophenyl, imidazolyl, oxazolyl, benzoxazolyl, thiazolyl, isoxazolyl, isotriazolyl, oxadiazolyl, triazolyl, thiadiazolyl, pyridazinyl, pyrimidinyl, pyrazinyl, triazinyl, trithianyl, indolizinyl, pyrazolyl, pyrazolinyl, pyrazolidinyl, thienyl, tetrahydroisoquinolinyl, cinnolinyl, phthalazinyl, quinazolinyl, quinoxalinyl, naphthyridinyl, pteridinyl, carbazolyl, acridinyl, phenazinyl, phenothiazinyl, and phenoxazinyl.

In a preferred embodiment of formula XXIII, Ar is selected from the group consisting of phenyl, benzyl, naphthyl, indolyl, pyridyl, pyrrolyl, pyrrolidinyl, pyridinyl, pyrimidinyl, purinyl, quinolinyl, isoquinolinyl, furyl, fluorenyl, thiophenyl, imidazolyl, oxazolyl, thiazolyl, pyrazolyl, and thienyl.

Exemplary compounds of formula XXIII are presented in TABLE VIII.

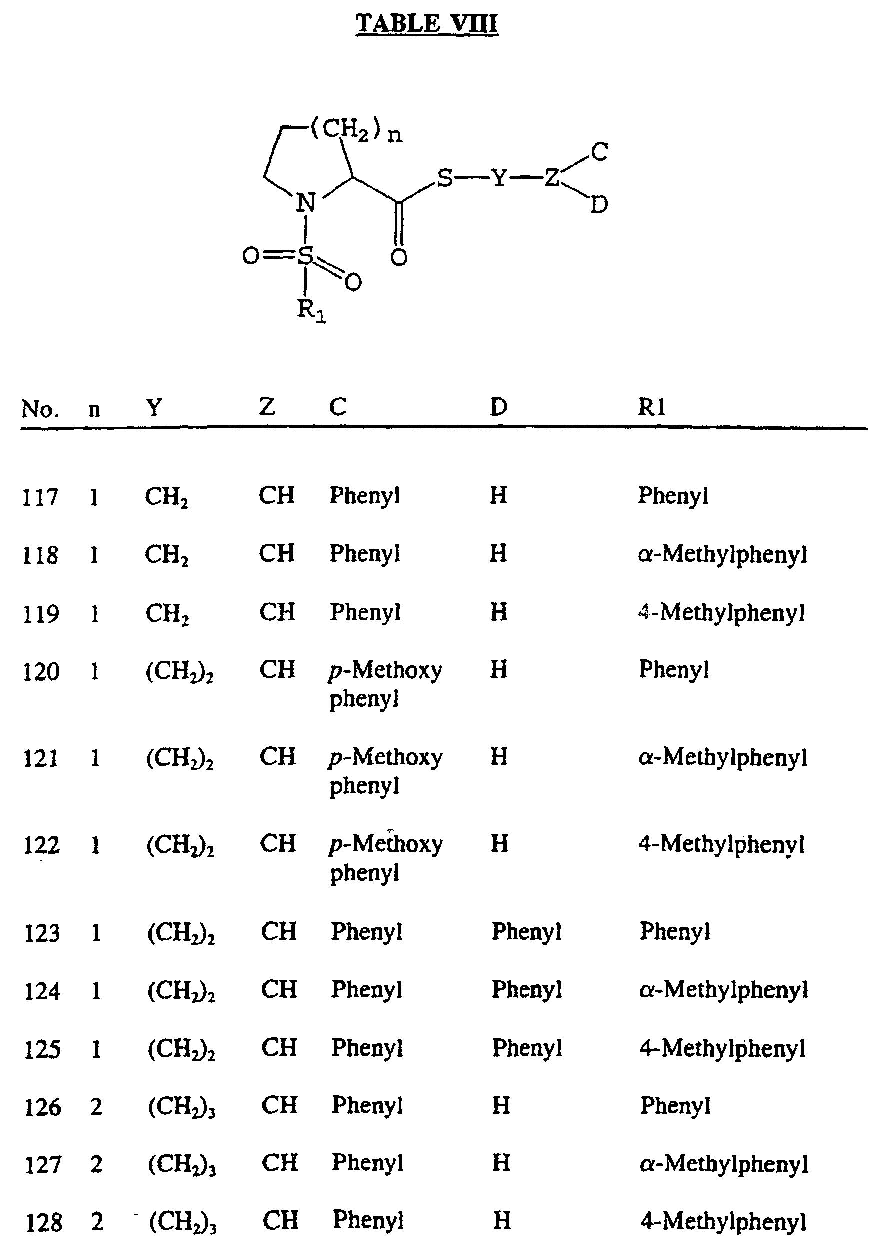

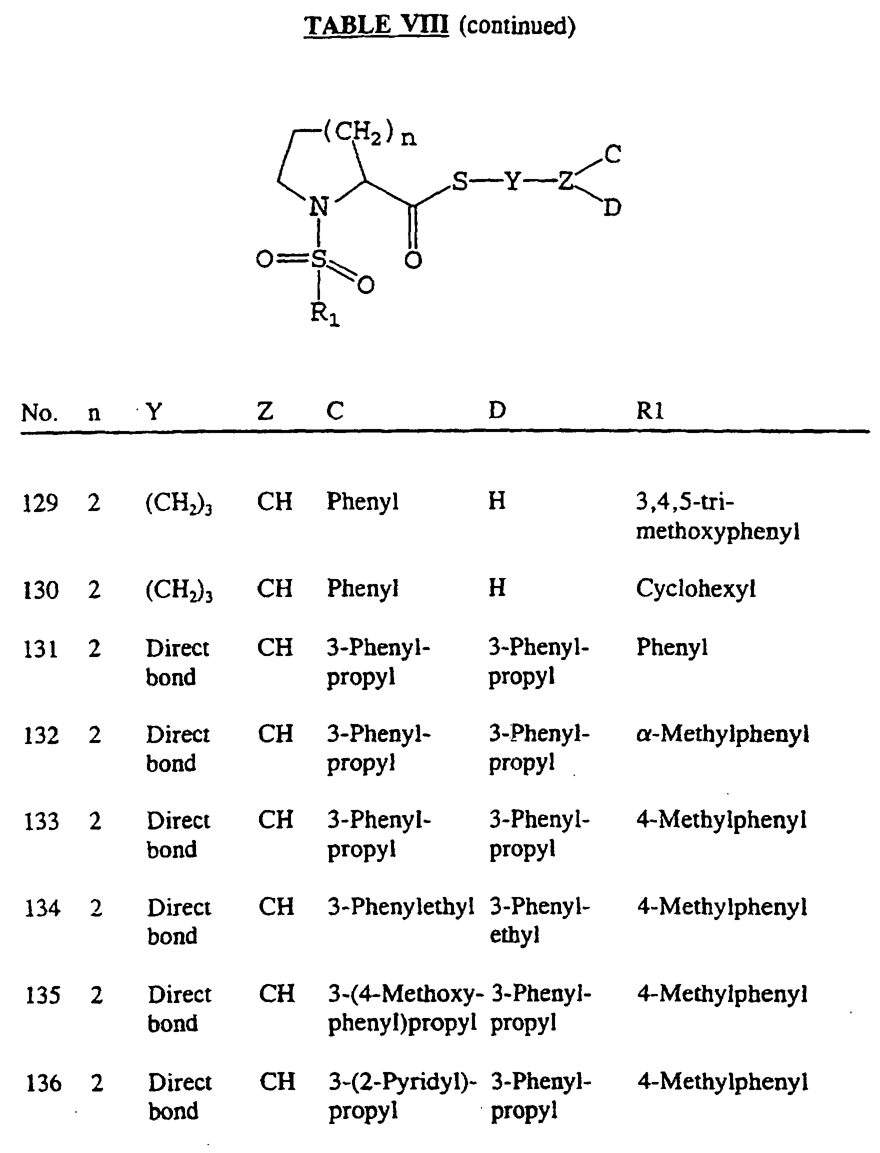

The most preferred compounds of formula XXIII are selected from the group consisting of:
3-(*para*-Methoxyphenyl)-1-propylmercaptyl(2*S*)-N-(benzenesulfonyl)pyrrolidine-2-carboxylate;
3-(*para*-Methoxyphenyl)-1-propylmercaptyl(2*S*)-N-(α-toluenesulfonyl)pyrrolidine-2-carboxylate;
3-(*para*-Methoxyphenyl)-1-propylmercaptyl(2*S*)-N-(α-toluenesulfonyl)pyrrolidine-2-carboxylate;
1,5-Diphenyl-3-pentylmercaptyl N-(*para*-toluenesulfonyl)pipecolate; and
pharmaceutically acceptable salts, esters, and solvates thereof.

### FORMULA XXIV

Moreover, the non-immunosuppressive neuroimmunophilin FKBP ligand may be a compound of formula XXIV 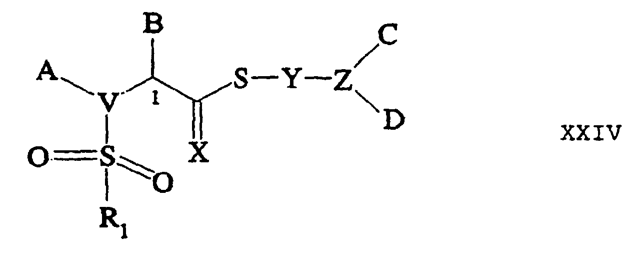 or a pharmaceutically acceptable salt, ester, or solvate thereof, wherein:
V is C, N, or S;
A, B, C, D, R₁, X, Y, and Z are as defined in formula XX above.

### VI. PYRROLIDINE DERIVATIVES

### FORMULA XXV

The non-immunosuppressive neuroimmunophilin FKBP ligand may also be a compound of formula XXV 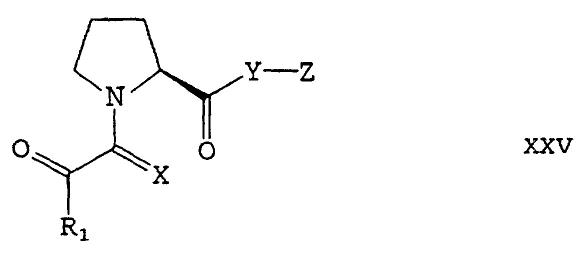 or a pharmaceutically acceptable salt, ester, or solvate thereof, wherein:
R₁ is C₁-C₉ straight or branched chain alkyl, C₂-C₉ straight or branched chain alkenyl, C₃-C₈ cycloalkyl, C₅-C₇ cycloalkenyl or Ar₁, wherein said R₁ is unsubstituted or substituted with one or more substituents independently selected from the group consisting of C₁-C₆ alkyl, C₂-C₆ alkenyl, C₃-C₈ cycloalkyl, C₅-C₇ cycloalkenyl, hydroxy, and Ar₂;
Ar₁ and Ar₂ are independently selected from the group consisting of 1-napthyl, 2-napthyl, 2-indolyl, 3-indolyl, 2-furyl, 3-furyl, 2-thienyl, 3-thienyl, 2-pyridyl, 3-pyridyl, 4-pyridyl and phenyl, wherein said Ar₁ is unsubstituted or substituted with one or more substituent(s) independently selected from the group consisting of hydrogen, halo, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino;
X is O, S, CH₂ or H₂;
Y is O or NR₂, wherein R₂ is hydrogen or C₁-C₆ alkyl; and
Z is C₁-C₆ straight or branched chain alkyl, or C₂-C₆ straight or branched chain alkenyl, wherein said Z is substituted with one or more substituent(s) independently selected from the group consisting of Ar₁, C₃-C₈ cycloalkyl, and C₁-C₆ straight or branched chain alkyl or C₂-C₆ straight or branched chain alkenyl substituted with C₃-C₈ cycloalkyl; or Z is fragment 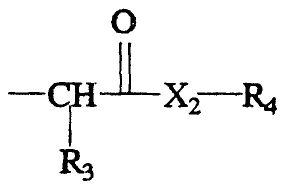
wherein:
R₃ is C₁-C₉ straight or branched chain alkyl which is unsubstituted or substituted with C₃-C₈ cycloalkyl or Ar₁;
X₂ is O or NR₅, wherein R₅ is selected from the group consisting of hydrogen, C₁-C₆ straight or branched chain alkyl, and C₂-C₆ straight or branched chain alkenyl; and
R₄ is selected from the group consisting of phenyl, benzyl, C₁-C₅ straight or branched chain alkyl, C₂-C₅ straight or branched chain alkenyl, C₁-C₅ straight or branched chain alkyl substituted with phenyl, and C₂-C₅ straight or branched chain alkenyl substituted with phenyl.

In a preferred embodiment of formula XXV, Z and R₁ are lipophilic.

In a more preferred embodiment of formula XXV, the compound is selected from the group consisting of:
3-phenyl-1-propyl (2*S*)-1-(3,3-dimethyl-1,2-dioxopentyl)-2-pyrrolidinecarboxylate;
3-phenyl-1-prop-2-(E)-enyl (2*S*)-1-(3,3-dimethyl-1,2-dioxopentyl)-2-pyrrolidinecarboxylate;
3-(3,4,5-trimethoxyphenyl)-1-propyl (2*S*)-1-(3,3-dimethyl-1,2-dioxopentyl)-2-pyrrolidinecarboxylate;
3-(3,4,5-trimethoxyphenyl)-1-prop-2-(E) -enyl (2*S*)-1-(3,3-dimethyl-1,2-dioxopentyl)-2-pyrrolidinecarboxylate;
3-(4,5-dichlorophenyl)-1-propyl (2*S*)-1-(3,3-dimethyl-1,2-dioxopentyl)-2-pyrrolidinecarboxylate;
3-(4,5-dichlorophenyl)-1-prop-2-(E)-enyl (2*S*)-1-(3,3-dimethyl-1,2-dioxopentyl)-2-pyrrolidinecarboxylate;
3-(4,5-methylenedioxyphenyl)-1-propyl (2*S*)-1-(3,3-dimethyl-1,2-dioxopentyl)-2-pyrrolidinecarboxylate;
3-(4,5-methylenedioxyphenyl)-1-prop-2-(E)-enyl (2*S*)-1-(3,3-dimethyl-1,2-dioxopentyl)-2-pyrrolidinecarboxylate;
3-cyclohexyl-1-propyl (2*S*)-1-(3,3-dimethyl-1,2-dioxopentyl)-2-pyrrolidinecarboxylate;
3-cyclohexyl-1-prop-2-(E)-enyl (2*S*)-1- (3,3-dimethyl-1,2-dioxopentyl)-2-pyrrolidinecarboxylate;
(1*R*)-1,3-diphenyl-1-propyl (2*S*)-1-(3,3-dimethyl-1,2-dioxopentyl)-2-pyrrolidinecarboxylate;
(1*R*)-1,3-diphenyl-1-prop-2-(E)-enyl (2*S*)-1-(3,3-dimethyl-1,2-dioxopentyl)-2-pyrrolidinecarboxylate;
(1*R*)-1-cyclohexyl-3-phenyl-1-propyl (2*S*)-1-(3,3-dimethyl-1,2-dioxopentyl)-2-pyrrolidinecarboxylate;
(1*R*)-1-cyclohexyl-3-phenyl-1-prop-2-(E)-enyl (2*S*)-1-(3,3-dimethyl-1,2-dioxopentyl)-2-pyrrolidinecarboxylate;
(1*R*)-1-(4,5-dichlorophenyl)-3-phenyl-1-propyl (2*S*)-1-(3,3-dimethyl-1,2-dioxopentyl)-2-pyrrolidinecarboxylate;
3-phenyl-1-propyl (2*S*)-1-(1,2-dioxo-2-cyclohexyl)ethyl-2-pyrrolidinecarboxylate;
3-phenyl-1-propyl (2*S*)-1-(1,2-dioxo-4-cyclohexyl)butyl-2-pyrrolidinecarboxylate;
3-phenyl-1-propyl (2*S*)-1-(1,2-dioxo-2-[2-furanyl])ethyl-2-pyrrolidinecarboxylate;
3-phenyl-1-propyl (2*S*)-1-(1,2-dioxo-2-[2-thienyl])ethyl-2-pyrrolidinecarboxylate;
3-phenyl-1-propyl (2*S*)-1-(1,2-dioxo-2-[2-thiazolyl])ethyl-2-pyrrolidinecarboxylate;
3-phenyl-1-propyl (2*S*)-1-(1,2-dioxo-2-phenyl)ethyl-2-pyrrolidinecarboxylate;
1,7-diphenyl-4-heptyl (2*S*)-1-(3,3-dimethyl-1,2-dioxopentyl)-2-pyrrolidinecarboxylate;
3-phenyl-3-propyl (2*S*)-1-(3,3-dimethyl-1,2-dioxo-4-hydroxybutyl)-2-pyrrolidinecarboxylate;
3-phenyl-1-propyl (2*S*)-1-(3,3-dimethyl-1,2-dioxopentyl)-2-pyrrolidinecarboxamide;
1-[1-(3,3-dimethyl-1,2-dioxopentyl)-L-proline]-L-phenylalanine ethyl ester;
1-[1-(3,3-dimethyl-1,2-dioxopentyl)-L-proline]-L-leucine ethyl ester;
1-[1-(3,3-dimethyl-1,2-dioxopentyl)-L-proline]-L-phenylglycine ethyl ester;
1-[1-(3,3-dimethyl-1,2-dioxopentyl)-L-proline]-L-phenylalanine phenyl ester;
1-[1-(3,3-dimethyl-1,2-dioxopentyl)-L-proline]-L-phenylalanine benzyl ester;
1-[1-(3,3-dimethyl-1,2-dioxopentyl)-L-proline]-L-isoleucine ethyl ester; and
pharmaceutically acceptable salts, esters, and solvates thereof.

### FORMULA XXVI

Additionally, the non-immunosuppressive neuroimmunophilin FKBP ligand may be a compound of formula XXVI 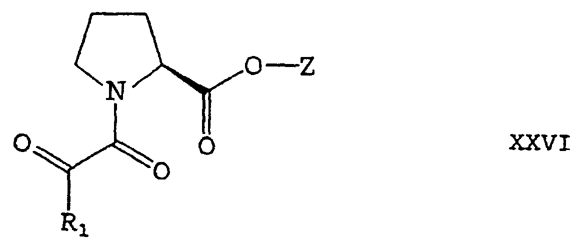 or a pharmaceutically acceptable salt, ester, or solvate thereof, wherein:
R₁ is C₁-C₉ straight or branched chain alkyl, C₂-C₉ straight or branched chain alkenyl, C₃-C₈ cycloalkyl, C₅-C₇ cycloalkenyl or Ar₁, wherein said R₁ is unsubstituted or substituted with one or more substituents independently selected from the group consisting of C₁-C₆ alkyl, C₂-C₆ alkenyl, C₃-C₈ cycloalkyl, C₅-C₇ cycloalkenyl, hydroxy, and Ar₂;
Ar₁ and Ar₂ are independently selected from the group consisting of 1-napthyl, 2-napthyl, 2-indolyl, 3-indolyl, 2-furyl, 3-furyl, 2-thienyl, 3-thienyl, 2-pyridyl, 3-pyridyl, 4-pyridyl and phenyl, wherein said Ar₁ is unsubstituted or substituted with one or more substituent(s) independently selected from the group consisting of hydrogen, halo, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, and amino;
Z is C₁-C₆ straight or branched chain alkyl, or C₂-C₆ straight or branched chain alkenyl, wherein said Z is substituted with one or more substituent(s) independently selected from the group consisting of Ar₁, C₃-C₈ cycloalkyl, and C₁-C₆ straight or branched chain alkyl or C₂-C₆ straight or branched chain alkenyl substituted with C₃-C₈ cycloalkyl; or Z is fragment 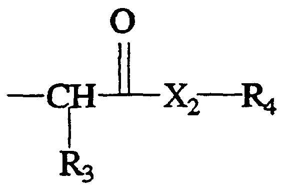
wherein:
R₃ is C₁-C₉ straight or branched chain alkyl which is unsubstituted or substituted with C₃-C₈ cycloalkyl or Ar₁;
X₂ is O or NR₅, wherein R₅ is selected from the group consisting of hydrogen, C₁-C₆ straight or branched chain alkyl, and C₂-C₆ straight or branched chain alkenyl; and
R, is selected from the group consisting of phenyl, benzyl, C₁-C₅ straight or branched chain alkyl, C₂-C₅ straight or branched chain alkenyl, C₁-C₅ straight or branched chain alkyl substituted with phenyl, and C₂-C₅ straight or branched chain alkenyl substituted with phenyl.

In a preferred embodiment of formula XXVI, R₁ is selected from the group consisting of C₁-C₉ straight or branched chain alkyl, 2-cyclohexyl, 4-cyclohexyl, 2-furanyl, 2-thienyl, 2-thiazolyl, and 4-hydroxybutyl.

In another preferred embodiment of formula XXVI, Z and R₁ are lipophilic.

### FORMULA XXVII

Furthermore, the non-immunosuppressive neurcimmunophilin FKBP ligand may be a' compound of formula XXVII 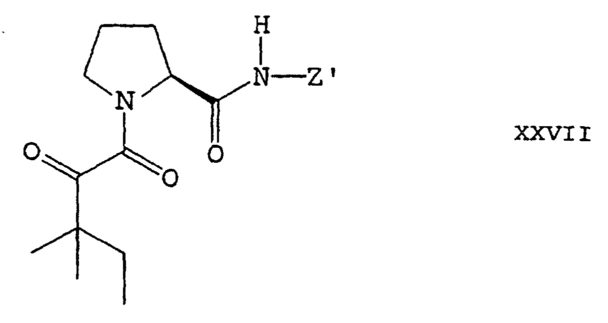 or a pharmaceutically acceptable salt, ester, or solvate thereof, wherein:
Z' is fragment 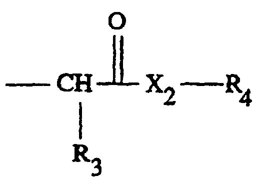
wherein:
R₃ is C₁-C₉ straight or branched chain alkyl or unsubstituted Ar₁, wherein said alkyl is unsubstituted or substituted with C₃-C₈ cycloalkyl or Ar₁;
X₂ is O or NR₅, wherein R₅ is selected from the group consisting of hydrogen, C₁-C₆ straight or branched chain alkyl, and C₂-C₆ straight or branched chain alkenyl;
R₄ is selected from the group consisting of phenyl, benzyl, C₁-C₅ straight or branched chain alkyl, C₂-C₅ straight or branched chain alkenyl, C₁-C₅ straight or branched chain alkyl substituted with phenyl, and C₂-C₅ straight or branched chain alkenyl substituted with phenyl; and
Ar₁ is as defined in formula XXVI.

In a preferred embodiment of formula XXVII, Z' is lipophilic.

### FORMULA XXVIII

The non-immunosuppressive neuroimmunophilin FKBP ligand may also be a compound of formula XXVIII 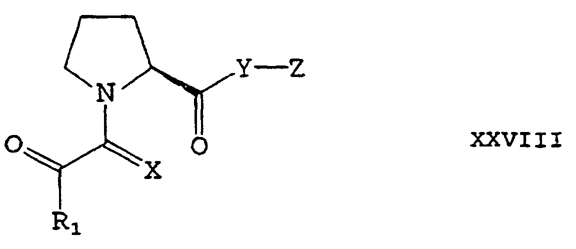 wherein:
R₁ is C₁-C₆ straight or branched chain alkyl, C₂-C₆ straight or branched chain alkenyl, C₃-C₆ cycloalkyl or Ar₁, wherein said alkyl or alkenyl is unsubstituted or substituted with C₃-C₆ cycloalkyl or Ar₂;
Ar₁ and Ar₂ are independently selected from the group consisting of 2-furyl, 2-thienyl, and phenyl;
X is selected from the group consisting of oxygen and sulfur;
Y is oxygen;
Z is C₁-C₆ straight or branched chain alkyl, or C₂-C₆ straight or branched chain alkenyl, wherein said Z is substituted with one or more substituent(s) independently selected from the group consisting of 2-furyl, 2-thienyl, C₃-C₆ cycloalkyl, pyridyl, and phenyl, each having one or more substituent(s) independently selected from the group consisting of hydrogen and C₁-C₄ alkoxy.

In a preferred embodiment of formula XXVIII, Z and R₁ are lipophilic.

In another preferred embodiment of formula XXVIII, the compound is selected from the' group consisting of:
3-(2,5-dimethoxyphenyl)-1-propyl (2*S*)-1-(3,3-dimethyl-1,2-dioxopentyl)-2-pyrrolidinecarboxylate;
3-(2,5-dimethoxyphenyl)-1-prop-2-(E)-enyl (2*S*)-1-(3,3-dimethyl-1,2-dioxopentyl)-2-pyrrolidinecarboxylate;
2-(3,4,5-trimethoxyphenyl)-1-ethyl (2*S*)-1-(3,3-dimethyl-1,2-dioxopentyl)-2-pyrrolidinecarboxylate;
3-(3-pyridyl)-1-propyl (2*S*)-1-(3,3-dimethyl-1,2-dioxopentyl)-2-pyrrolidinecarboxylate;
3-(2-pyridyl)-1-propyl (2*S*)-1-(3,3-dimethyl-1,2-dioxopentyl)-2-pyrrolidinecarboxylate;
3-(4-pyridyl)-1-propyl (2*S*)-1-(3,3-dimethyl-1,2-dioxopentyl)-2-pyrrolidinecarboxylate;
3-phenyl-1-propyl (2*S*)-1-(2-*tert*-butyl-1,2-dioxoethyl)-2-pyrrolidinecarboxylate;
3-phenyl-1-propyl (2*S*)-1-(2-cyclohexylethyl-1,2-dioxoethyl)-2-pyrrolidinecarboxylate;
3-(3-pyridyl)-1-propyl (2*S*)-1-(2-cyclohexylethyl-1,2-dioxoethyl)-2-pyrrolidinecarboxylate;
3-(3-pyridyl)-1-propyl (2*S*)-1-(2-*tert*-butyl-1,2-dioxoethyl)-2-pyrrolidinecarboxylate;
3,3-diphenyl-1-propyl (2*S*)-1-(3,3-dimethyl-1,2-dioxopentyl)-2-pyrrolidinecarboxylate;
3-(3-pyridyl)-1-propyl (2*S*)-1-(2-cyclohexyl-1,2-dioxoethyl)-2-pyrrolidinecarboxylate;
3-(3-pyridyl)-1-propyl (2*S*)-N-([2-thienyl] glyoxyl)pyrrolidinecarboxylate;
3,3-diphenyl-1-propyl (2*S*)-1-(3,3-dimethyl-1,2-dioxobutyl)-2-pyrrolidinecarboxylate;
3,3-diphenyl-1-propyl (2*S*)-1-cyclohexylglyoxyl-2-pyrrolidinecarboxylate;
3,3-diphenyl-1-propyl (2*S*)-1-(2-thienyl)glyoxyl-2-pyrrolidinecarboxylate; and
pharmaceutically acceptable salts, esters, and solvates thereof.

In a more preferred embodiment of formula XXVIII, the compound is selected from the group consisting of:
3-(3-pyridyl)-1-propyl (2*S*)-1-(3,3-dimethyl-1,2-dioxopentyl)-2-pyrrolidinecarboxylate;
3-(2-pyridyl)-1-propyl (2*S*)-1-(3,3-dimethyl-1,2-dioxopentyl)-2-pyrrolidinecarboxylate;
3-(3-pyridyl)-1-propyl (2*S*)-1-(2-cyclohexyl-1,2-dioxoethyl)-2-pyrrolidinecarboxylate; and
pharmaceutically acceptable salts, esters, and solvates thereof.

In the most preferred embodiment of formula XXVIII, the compound is 3-(3-pyridyl)-1-propyl (2*S*)-1-(3,3-dimethyl-1,2-dioxopentyl)-2-pyrrolidinecarboxylate, and pharmaceutically acceptable salts, esters, and solvates thereof.

### FORMULA XXIX

Additionally, the non-immunosuppressive neuroimmunophilin FKBP ligand may be a compound of formula XXIX 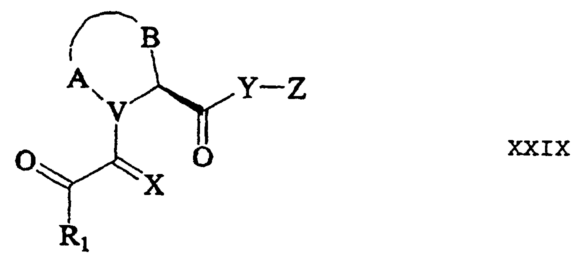 or a pharmaceutically acceptable salt, ester, or solvate thereof, wherein:
V is C, N, or S;
A and B, together with V and the carbon atom to which they are respectively attached, form a 5-7 membered saturated or unsaturated heterocyclic ring containing, in addition to V, one or more heteroatom(s) independently selected from the group consisting of O, S, SO, SO₂, N, NH, and NR;
R is either C₁-C₉ straight or branched chain alkyl, C₂-C₉ straight or branched chain alkenyl, C₃-C₉ cycloakyl, C₅-C₇ cycloalkenyl, or Ar₁, wherein R is either unsubstituted of substituted with one or more substituent(s) independently selected from the group consisting of halo, haloalkyl, carbonyl, carboxy, hydroxy, nitro, trifluoromethyl, C₁-C₆ straight or branched chain alkyl, C₂-C₆ straight or branched chain alkenyl, C₁-C₄ alkoxy, C₂-C₄ alkenyloxy, phenoxy, benzyloxy, thioalkyl, alkylthio, sulfhydryl, amino, alkylamino, aminoalkyl, aminocarboxyl, and Ar₂;
R₁ is C₁-C₉ straight or branched chain alkyl, C₂-C₉ straight or branched chain alkenyl, C₃-C₈ cycloalkyl, C₅-C₇ cycloalkenyl or Ar₁, wherein said R₁ is unsubstituted or substituted with one or more substituents independently selected from the group consisting of C₁-C₆ alkyl, C₂-C₆ alkenyl, C₃-C₈ cycloalkyl, C₅-C₇ cycloalkenyl, hydroxy, and Ar₂;
Ar₁ and Ar₂ are independently an alicyclic or aromatic, mono-, bi- or tricyclic, carbo- or heterocyclic ring, wherein the ring is either unsubstituted or substituted with one or more substituent(s); wherein the individual ring size is 5-8 members; wherein said heterocyclic ring contains 1-6 heteroatom(s) independently selected from the group consisting of O, N, and S;
X is O, S, CH₂ or H₂;
Y is O or NR₂, wherein R₂ is hydrogen or C₁-C₆ alkyl; and
Z is C₁-C₆ straight or branched chain alkyl, or C₂-C₆ straight or branched chain alkenyl, wherein said Z is substituted with one or more substituent(s) independently selected from the group consisting of Ar₁, C₃-C₈ cycloalkyl, and C₁-C₆ straight or branched chain alkyl or C₂-C₆ straight or branched chain alkenyl substituted with C₃-C₈ cycloalkyl; or Z is fragment 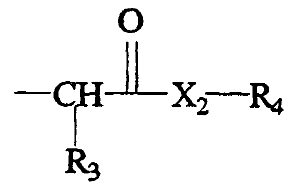
wherein:
R₃ is C₁-C₉ straight or branched chain alkyl which is unsubstituted or substituted with C₃-C₈ cycloalkyl or Ar₁;
X₂ is O or NR₅, wherein R₅ is selected from the group consisting of hydrogen, C₁-C₆ straight or branched chain alkyl, and C₂-C₆ straight or branched chain alkenyl; and
R₄ is selected from the group consisting of phenyl, benzyl, C₁-C₅ straight or branched chain alkyl, C₂-C₅ straight or branched chain alkenyl, C₁-C₅ straight or branched chain alkyl substituted with phenyl, and C₂-C₅ straight or branched chain alkenyl substituted with phenyl.

All the compounds of Formulas I-XXIX possess asymmetric centers and thus can be produced as mixtures of stereoisomers or as individual R- and S-stereoisomers. The individual stereoisomers may be obtained by using an optically active starting material, by resolving a racemic or non-racemic mixture of an intermediate at some appropriate stage of the synthesis, or by resolving the compounds of Formulas I-XXIX. It is understood that the compounds of Formulas I-XXIX encompass individual stereoisomers as well as mixtures (racemic and non-racemic) of stereoisomers. Preferably, S-stereoisomers are used in the pharmaceutical compositions and methods of the present invention.

### Synthesis of Non-Immunosuppressive Neuroimmunophilin FKBP ligands

The compounds of formulas XV to XIX may be readily prepared by standard techniques of organic chemistry, utilizing the general synthetic pathway depicted below. As described by Scheme I, cyclic amino acids 1 protected by suitable blocking groups P on the amino acid nitrogen may be reacted with thiols RSH to generate thioesters 2. After removal of the protecting group, the free amine 3 may be reacted with a variety of isocyanates or isothiocyanates to provide the final ureas or thioureas, respectively.

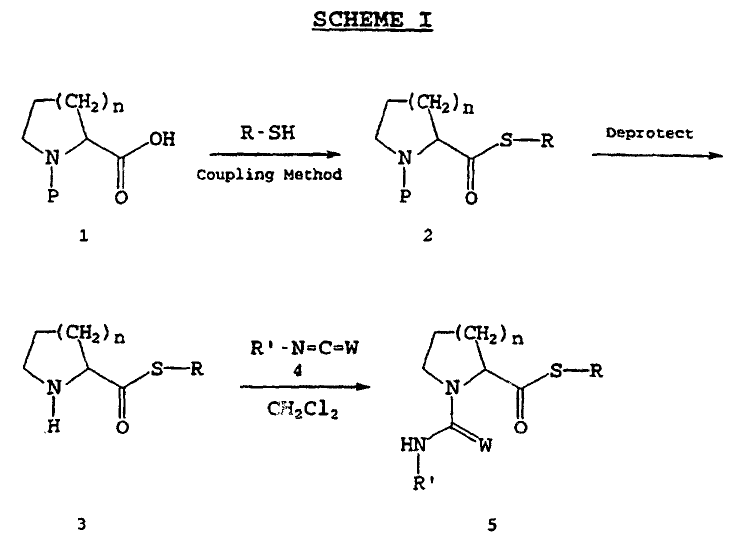

Isocyanates (R'NCO) or isothiocyanates (R'NCS) 4 may be conveniently prepared from the corresponding readily available amines by reaction with phosgene or thiophosgene, as depicted in Scheme II.

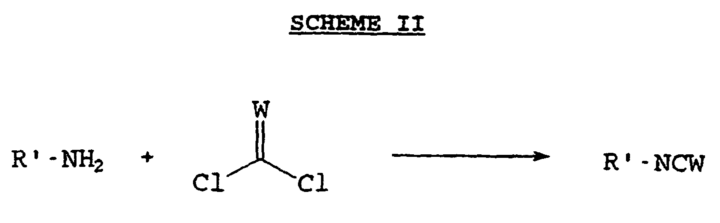

Thiols R-SH may be conveniently prepared from the corresponding readily available alcohols or halides via a two step replacement of halide by sulfur, as described in Scheme III. Halides may be reacted with. thiourea, and the corresponding alkyl thiouronium salts hydrolyzed to provide thiols RSH. If alcohols are used as the starting materials, they may be first converted to the corresponding halides by standard methods.

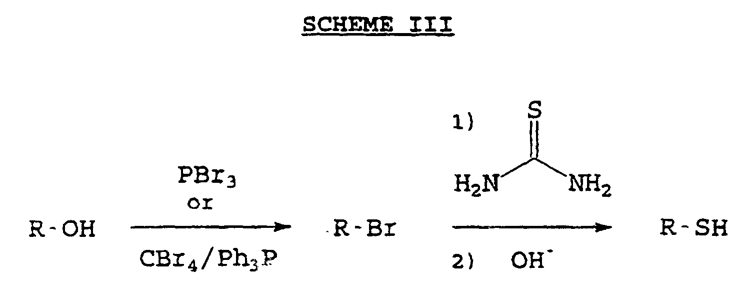

The compounds of formulas XX to XXIV may be readily prepared by standard techniques of organic chemistry, utilizing the general synthetic pathway depicted below. As described by Scheme IV, cyclic amino acids 1 protected by suitable blocking groups P on the amino acid nitrogen may be reacted with thiols RSH to generate thioesters 2. After removal of the protecting group, the free amine 3 may be reacted with various sulfonyl chlorides 4 to provide final products 5 in good to excellent yield.

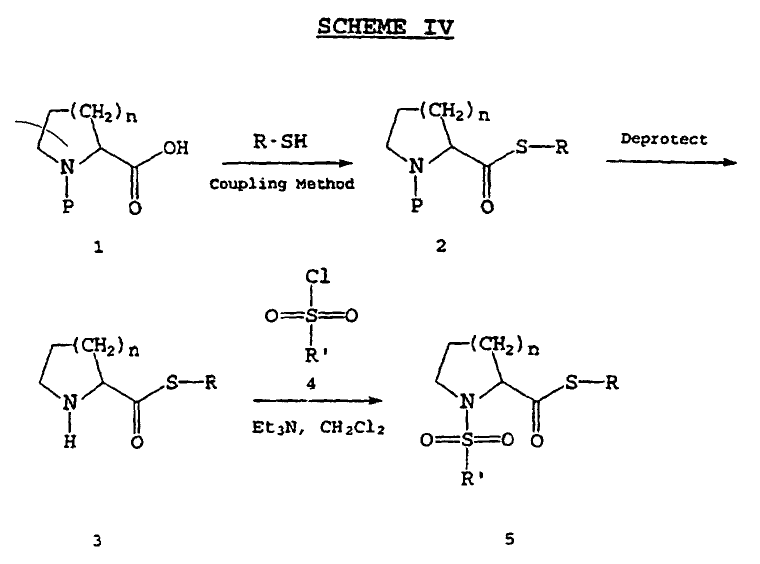

Thiols R-SH may be conveniently prepared from the corresponding readily available alcohols or halides via a two step replacement of halogen by sulfur, as described in Scheme V. Halides may be reacted with thiourea, and the corresponding alkyl thiouronium salts hydrolyzed to provide thiols RSH. If alcohols are used as the starting materials, they may be first converted to the corresponding halides by standard methods.

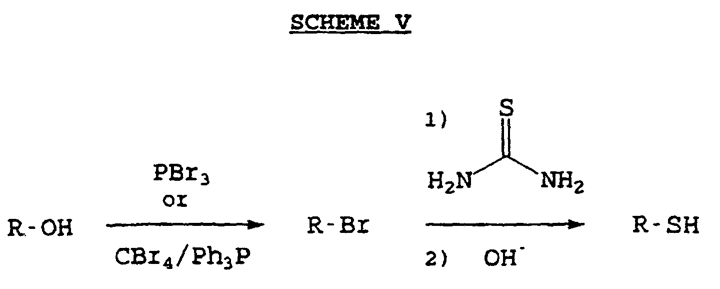

The compounds of formulas XXV to XXIX may be prepared by a variety of synthetic sequences that utilize established chemical transformations. The general pathway to the present compounds is described in Scheme VI. N-glyoxylproline derivatives may be prepared by reacting L-proline methyl ester with methyl oxalyl chloride as shown in Scheme VI. The resulting oxamates may be reacted with a variety of carbon nucleophiles to obtain intermediates compounds. These intermediates are then reacted with a variety of alcohols, amides, or protected amino acid residues to obtain the propyl esters and amides of the invention.

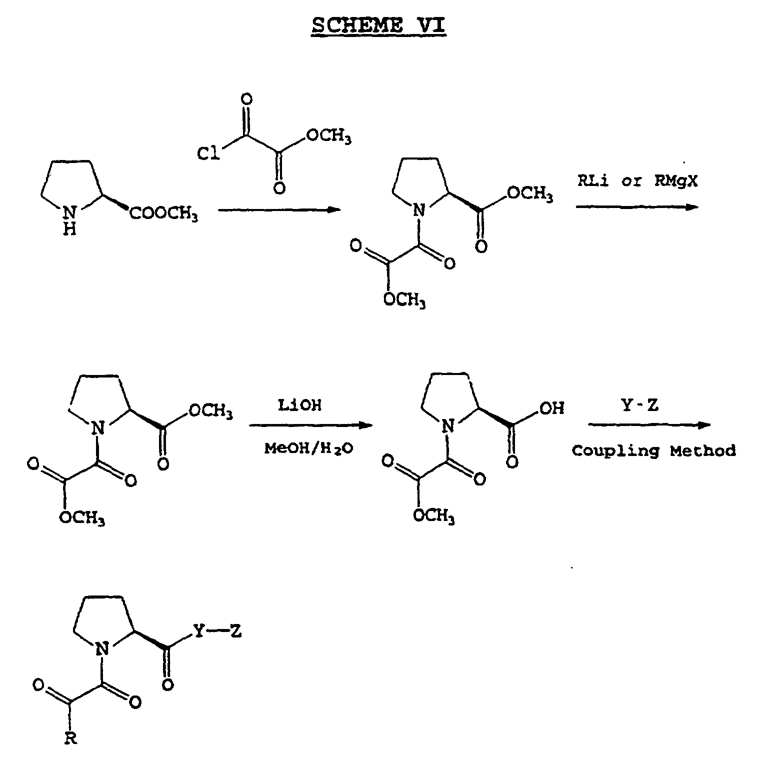

The substituted alcohols may be prepared by a number of methods known to those skilled in the art of organic synthesis. As described in Scheme VII, alkyl or aryl aldehydes may be homologated to phenyl propanols by reaction with methyl(triphenylphosphoranylidene)acetate to provide a variety of trans-cinnamates; these latter may be reduced to the saturated alcohols by reaction with excess lithium aluminum hydride, or sequentially by reduction of the double bond by catalytic hydrogenation and reduction of the saturated ester by appropriate reducing agents. Alternatively, the trans-cinnamates may be reduced to (E)-allylic alcohols by the use of diisobutylaluminum hydride.

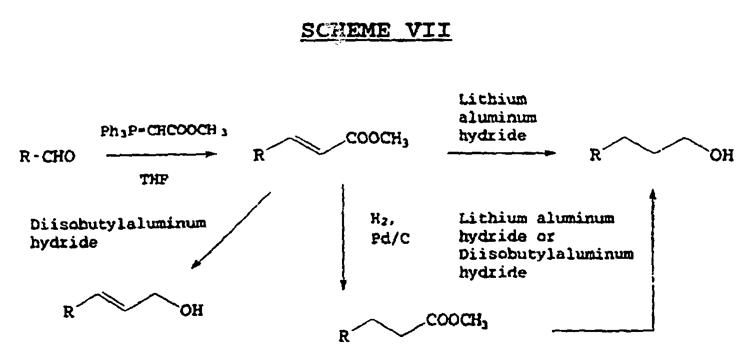

Longer chain alcohols may be prepared by homologation of benzylic and higher aldehydes. Alternatively, these aldehydes may be prepared by conversion of the corresponding phenylacetic and higher acids, and phenethyl and higher alcohols.

### Affinity for FKBP12

The compounds used in the inventive methods and pharmaceutical compositions have an affinity for the FK506 binding protein, particularly FKBP12. The inhibition of the prolyl peptidyl *cis-trans* isomerase activity of FKBP may be measured as an indicator of this affinity.

### Kᵢ Test Procedure

Inhibition of the peptidyl-prolyl isomerase (rotamase) activity of the compounds used in the inventive methods and pharmaceutical compositions can be evaluated by known methods described in the literature (Harding et al., *Nature,* 1989, 341:758-760; Holt et al. *J. Am. Chem. Soc.,* 115:9923-9938). These values are obtained as apparent Kᵢ's and are presented for representative compounds in TABLES IX to XVI.

The *cis-trans* isomerization of an alanine-proline bond in a model substrate, N-succinyl-Ala-Ala-Pro-Phe-p-nitroanilide, is monitored spectrophotometrically in a chymotrypsin-coupled assay, which releases *para*-nitroanilide from the trans form of the substrate. The inhibition of this reaction caused by the addition of different concentrations of inhibitor is determined, and the data is analyzed as a change in first-order rate constant as a function of inhibitor concentration to yield the apparent Kᵢ values.

In a plastic cuvette are added 950 mL of ice cold assay buffer (25 mM HEPES, pH 7.8, 100 mM NaCl), 10 mL of FKBP (2.5 mM in 10 mM Tris-Cl pH 7.5, 100 mM NaCl, 1 mM dithiothreitol), 25 mL of chymotrypsin (50 mg/ml in 1 mM HCl) and 10 mL of test compound at various concentrations in dimethyl sulfoxide. The reaction is initiated by the addition of 5 mL of substrate (succinyl-Ala-Phe-Pro-Phe-*para*-nitroanilide, 5 mg/mL in 2.35 mM LiCl in trifluoroethanol).

The absorbance at 390 nm versus time is monitored for 90 seconds using a spectrophotometer and the rate constants are determined from the absorbance versus time data files.

**TABLE IX**

| *In Vitro* Test Results - Formulas I to V | |
|---|---|
| Compound | Kᵢ (nM) |
| 1 | 31 |
| 2 | 210 |
| 3 | 85 |
| 9 | 104 |
| 10 | 12 |
| 11 | 299 |
| 12 | 442 |
| 14 | 313 |
| 28 | 108 |
| 29 | 59 |
| 30 | 11 |
| 31 | 8.7 |
| 32 | 362 |
| 33 | 1698 |
| 34 | 34 |
| 35 | 62 |
| 36 | 7 |
| 37 | 68 |
| 38 | 8.9 |
| 39 | 347 |
| 40 | 1226 |
| 41 | 366 |
| 42 | 28 |
| 43 | 259 |
| 44 | 188 |
| 45 | 31 |
| 46 | 757 |
| 47 | 21 |
| 48 | 127 |
| 49 | 1334 |
| 50 | 55 |
| 51 | 33 |
| 52 | 6 |
| 53 | 261 |
| 54 | 37 |
| 55 | 30 |
| 56 | 880 |
| 57 | 57 |
| 58 | 79 |
| 59 | 962 |
| 60 | 90 |
| 61 | 139 |
| 62 | 196 |
| 63 | 82 |
| 64 | 163 |
| 65 | 68 |
| 66 | 306 |
| 67 | 177 |
| 68 | 284 |
| 69 | 49 |
| 70 | 457 |
| 71 | 788 |

**TABLE X**

| *In Vitro* Test Results - Formulas VI to IX | |
|---|---|
| Compound | Kᵢ (nM) |
| 80 | 215 |
| 81 | 638 |

**TABLE XI**

| *In Vitro* Test Results - Formulas X to XIV | |
|---|---|
| Compound | Kᵢ (nM) |
| Parent (unoxidized) | 7.5 |
| compound of Example 6 | |
| 95 (Example 6) | 225 |

**TABLE XII**

| *In Vitro* Test Results - Formulas XV to XIX | |
|---|---|
| Compound | Kᵢ (nM) |
| 101 | +++ |
| 102 | ++ |
| 103 | ++ |
| 104 | ++ |
| 105 | ++ |
| 106 | + |
| 107 | ++ |
| 108 | +++ |
| 109 | +++ |
| 110 | +++ |
| 111 | ++ |
| 112 | +++ |
| 113 | +++ |
| 114 | +++ |
| 115 | +++ |
| 116 | ++ |
| Relative potencies of compounds are ranked according to the following scale: ++++ denotes Kᵢ or ED50 < 1 nM; +++ denotes Kᵢ or ED50 of 1-50 nM; ++ denotes Kᵢ or ED 50 of 51-200 nM; + denotes Kᵢ or ED of 201-500 nM. | |

**TABLE XIII**

| *In Vitro* Test Results - Formulas XX to XXIV | |
|---|---|
| Compound | Kᵢ (nM) |
| 117 | +++ |
| 118 | ++ |
| 119 | ++ |
| 120 | ++ |
| 121 | ++ |
| 122 | + |
| 123 | ++ |
| 124 | +++ |
| 125 | +++ |
| 126 | +++ |
| 127 | ++ |
| 128 | +++ |
| 129 | +++ |
| 130 | +++ |
| 131 | +++ |
| 132 | ++ |
| Relative potencies of compounds are ranked according to the following scale: ++++ denotes Kᵢ or ED50 < 1 nM; +++ denotes Kᵢ or ED50 of 1-50 nM; ++ denotes Kᵢ or ED 50 of 51-200 nM; + denotes Kᵢ or ED of 201-500 nM. | |

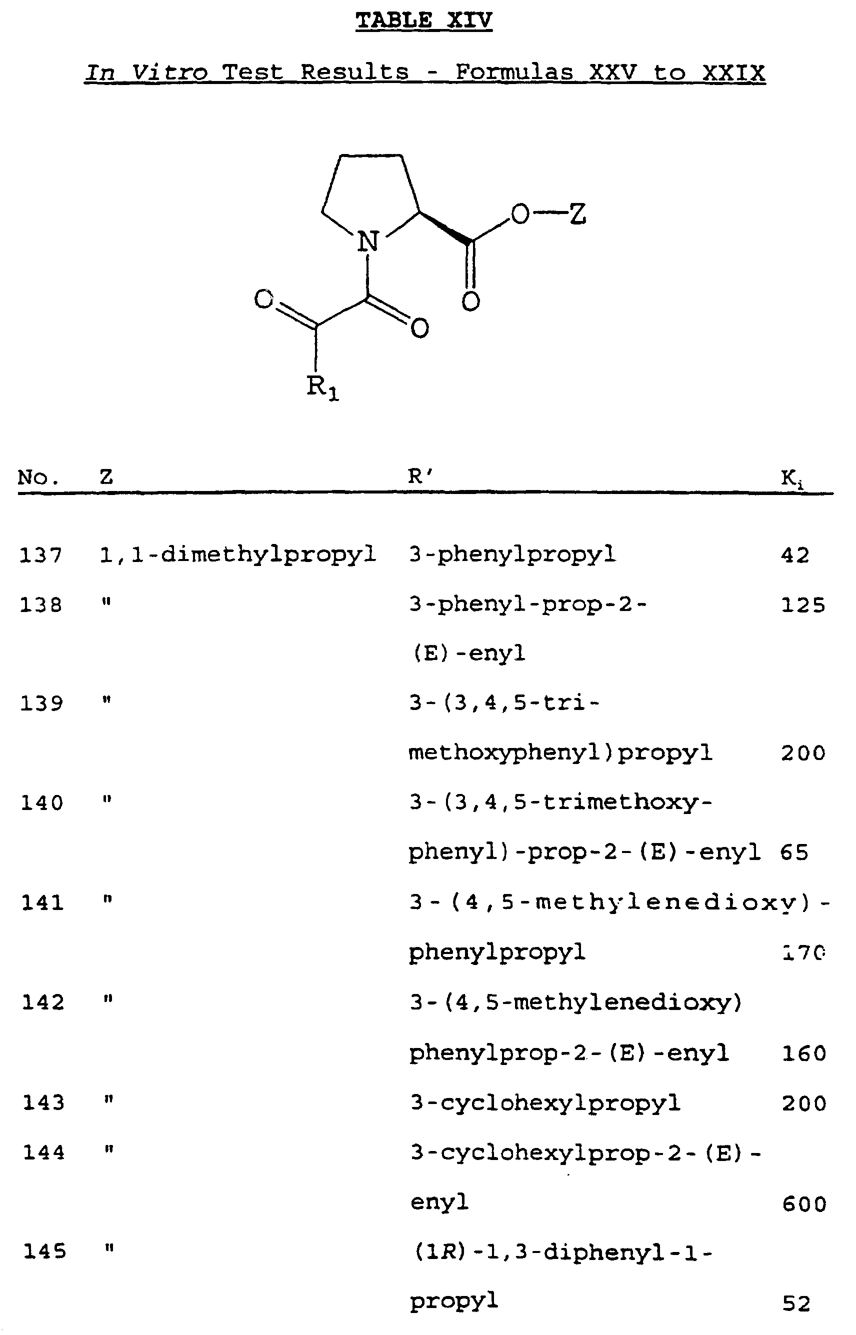

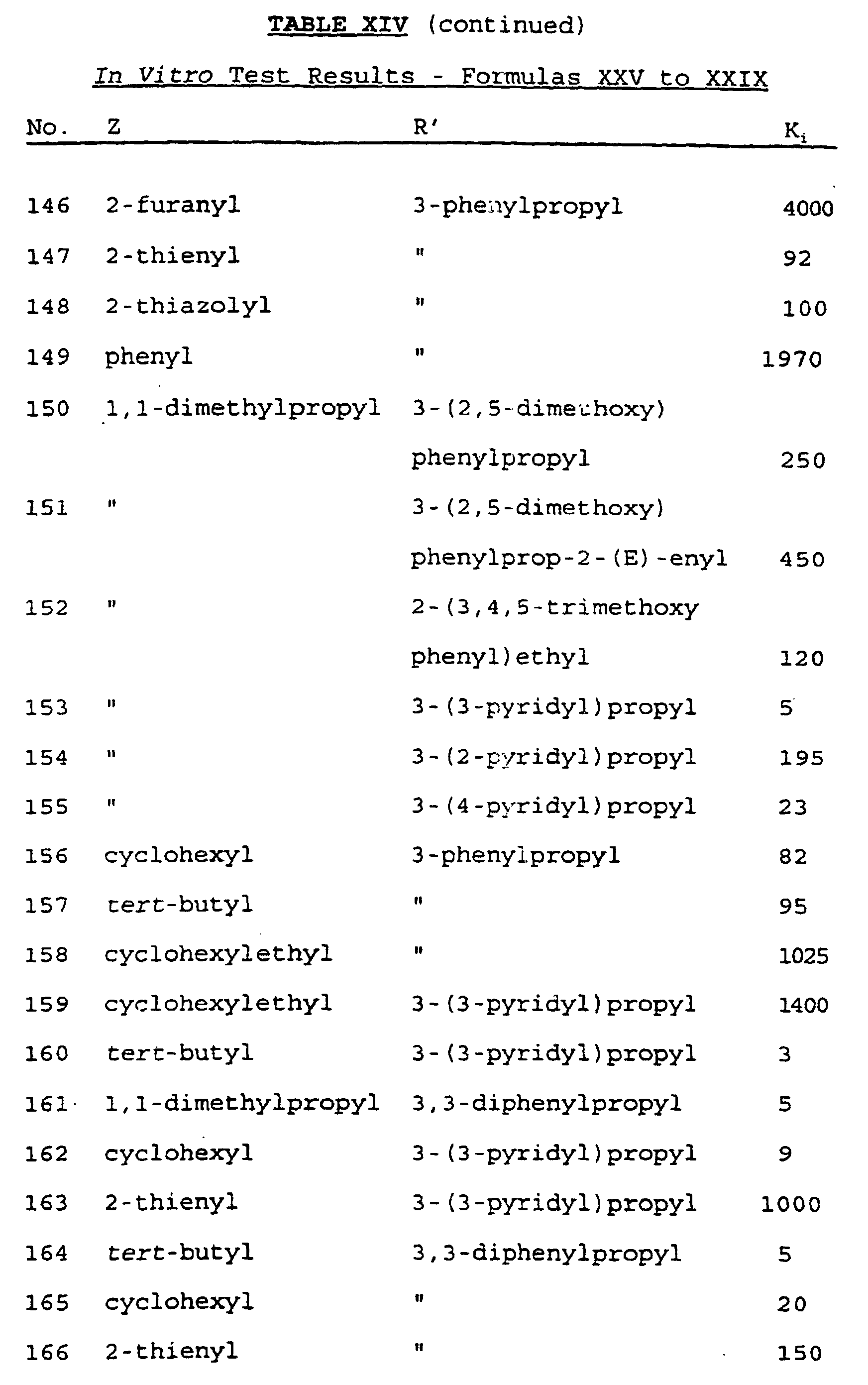

### Route of Administration

To effectively treat alopecia or promote hair growth, the compounds used in the inventive methods and pharmaceutical compositions must readily affect the targeted areas. For these purposes, the compounds are preferably administered topically to the skin.

For topical application to the skin, the compounds can be formulated into suitable ointments containing the compounds suspended or dissolved in, for example, mixtures with one or more of the following: mineral oil, liquid petrolatum, white petrolatum, propylene glycol, polyoxyethylene polyoxypropylene compound, emulsifying wax and water. Alternatively, the compounds can be formulated into suitable lotions or creams containing the active compound suspended or dissolved in, for example, a mixture of one or more of the following: mineral oil, sorbitan monostearate, polysorbate 60, cetyl ester wax, cetearyl alcohol, 2-octyldodecanol, benzyl alcohol and water.

Other routes of administration known in the pharmaceutical art are also contemplated by this invention.

### Dosage

Dosage levels on the order of about 0.1 mg to about 10,000 mg of the active ingredient compound are useful in the treatment of the above conditions, with preferred levels of about 0.1 mg to about 1,000 mg. The specific dose level for any particular patient will vary depending upon a variety of factors, including the activity of the specific compound employed; the age, body weight, general health, sex and diet of the patient; the time of administration; the rate of excretion; drug combination; the severity of the particular disease being treated; and the form of administration. Typically, in vitro dosage-effect results provide useful guidance on the proper doses for patient administration. Studies in animal models are also helpful. The considerations for determining the proper dose levels are well known in the art.

The compounds can be administered with other hair revitalizing agents. Specific dose levels for the other hair revitalizing agents will depend upon the factors previously stated and the effectiveness of the drug combination.

### EXAMPLES

The following examples are illustrative of the present invention and are not intended to be limitations thereon. Unless otherwise indicated, all percentages are based upon 100% by weight of the final composition.

### Example 1

### Synthesis of (2S)-2-({1-oxo-5-phenyl}-pentyl-1-(3,3-dimethyl-1,2-dioxopentyl)pyrrolidine (1)

### (2S)-2-(1-oxo-4-phenyl)butyl-N-benzylpyrrolidine

1-chloro-4-phenylbutane (1.78 g; 10.5 mmol) in 20 mL of THF was added to 0.24 g (10 mmol) of magnesium turnings in 50 mL of refluxing THF. After the addition was complete, the mixture was refluxed for an additional 5 hours, and then added slowly to a refluxing solution of N-benzyl-L-proline ethyl ester (2.30 g (10 mmol) in 100 mL of THF. After 2 hours of further reflux, the mixture was cooled and treated with 5 mL of 2 N HCl. The reaction mixture was diluted with ether (100 mL) and washed with saturated NaHCO₃, water and brine. The organic phase was dried, concentrated and chromatographed, eluting with 5:1 CH₂Cl₂:EtOAc to obtain 2.05 g (64%) of the ketone as an oil. ¹H NMR (CDCl₃; 300 MHz): 1.49-2.18 (m, 8H); 2.32-2.46 (m, 1H); 2.56-2.65 (m, 2H) ; 2.97-3.06 (m, 1H); 3.17-3.34 (m, 1H); 3.44-3.62 (m, 1H); 4.02-4.23 (m, 2H); 7.01-7.44 (m, 10H).

### (2S)-2-(1-oxo-4-phenyl)butylpyrrolidine

The ketone compound (500 mg) and palladium hydroxide (20% on carbon, 50 mg) was hydrogenated at 40 psi in a Paar shaker overnight. The catalyst was removed by filtration and the solvent was removed in vacuo. The free amine was obtained as a yellow oil (230 mg; 100%). ¹H NMR (CDCl₃; 300 MHz): 1.75-2.34 (m, 10H); 2.55 (m, 2H); 2.95 (dm, 1H); 3.45-3.95 (m, 1H); 4.05 (m, 1H); 7.37 (m, 5H).

### (2S)-2-(1-oxo-4-phenyl)butyl-1-(1,2-dioxo-2-methoxyethyl)pyrrolidine

To a solution of (2*S*)-2-(1-oxo-4-phenyl)butylpyrrolidine (230 mg; 1.0 mmol) in CH₂Cl₂(20 mL) at 0°C was added dropwise methyloxalyl chloride (135 mg; 1.1 mmol). After stirring at 0°C for 3 hours, the reaction was quenched with saturated NH₄Cl and the organic phase was washed with water and brine and dried and concentrated. The crude residue was purified on a silica gel column, eluting with 20:1 CH₂Cl₂:EtOAc to obtain 300 mg of the oxamate as a clear oil (98%). ¹H NMR (CDCl₃; 300 MHz) : 1.68 (m, 4H); 1.91-2.38 (m, 4H); 2.64 (t, 2H); 3.66-3.80 (m, 2H); 3.77, 3.85 (s, 3H total); 4.16 (m, 2H); 4.90 (m, 1H); 7.16 (m, 3H); 7.27 (m, 2H).

### (2S)-2-({1-oxo-5-phenyl}-pentyl-1-(3,3-dimethyl-1,2-dioxopentyl)pyrrolidine (1)

To a solution of the oxamate above (250 mg; 0.79 mmol) in anhydrous ether (15 mL), cooled to - 78°C, was added 1,1-dimethylpropyl-magnesium chloride (0.8 mL of a 1.0 M solution in ether; 0.8 mmol). After stirring the resulting mixture at -78°C for 2 hours, the reaction was quenched by the addition of 2 mL of saturated NH₄Cl, followed by 100 mL of EtOAc. The organic phase was washed with brine, dried, concentrated, and purified on a silica gel column, eluting with 50:1 CH₂Cl₂:EtOAc. Compound 1 was obtained as a clear oil, 120 mg. ¹H NMR (CDCl₃, 300 MHz): δ 0.87 (t, 3H, 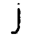 = 7.5); 1.22 (s, 3H); 1.25 (s, 3H); 1.67 (m, 4H); 1.70-2.33 (m, 6H); 2.61 (t, 2H, 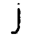 = 7.1); 3.52 (m, 2H); 4.17 (t, 2H, 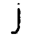 = 6.2); 4.52 (m, 1H); 7.16-7.49 (m, 5H). Analysis calculated for C₂₂H₃₁NO₃ - H₂O: C, 70.37; H, 8.86; N, 3.73. Found: 70.48; H, 8.35; N, 3.69.

### Example 2

### Synthesis of 2-phenyl-1-ethyl 1-(3,3-dimethyl-1,2-dioxopentyl)-2-piperidinecarbothioate (10)

### Methyl(2S)-1-(1,2-dioxo-2-methoxyethyl)-2-pyrrolidinecarboxylate

A solution of L-proline methyl ester hydrochloride (3.08 g; 18.60 mmol) in dry methylene chloride was cooled to 0°C and treated with triethylamine (3.92 g; 38.74 mmol; 2.1 eq). After stirring the formed slurry under a nitrogen atmosphere for 15 min, a solution of methyl oxalyl chloride (3.20 g; 26.12 mmol) in methylene chloride (45 mL) was added dropwise. The resulting mixture was stirred at 0°C for 1,5 hour. After filtering to remove solids, the organic phase was washed with water, dried over MgSO₄ and concentrated. The crude residue was purified on a silica gel column, eluting with 50% ethyl acetate in hexane, to obtain 3.52 g (88%) of the product as a reddish oil. Mixture of cis-trans amide rotamers; data for trans rotamer given. ¹H NMR (CDCl₃): δ 1.93 (dm, 2H); 2.17 (m, 2H); 3.62 (m, 2H); 3.71 (s, 3H); 3.79, 3.84 (s, 3H total); 4.86 (dd, 1H, 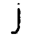 = 8.4, 3.3).

### Methyl(2S)-1-(1,2-dioxo-3,3-dimethylpentyl)-2-pyrrolidinecarboxylate

A solution of methyl (2*S*)-1-(1,2-dioxo-2-methoxyethyl)-2-pyrrolidinecarboxylate (2.35 g; 10.90 mmol) in 30 mL of tetrahydrofuran (THF) was cooled to -78°C and treated with 14.2 mL of a 1.0 M solution of 1,1-dimethylpropylmagnesium chloride in THF. After stirring the resulting homogeneous mixture at -78°C for three hours, the mixture was poured into saturated ammonium chloride (100 mL) and extracted into ethyl acetate. The organic phase was washed with water, dried, and concentrated, and the crude material obtained upon removal of the solvent was purified on a silica gel column, eluting with 25% ethyl acetate in hexane, to obtain 2.10 g (75%) of the oxamate as a colorless oil. ¹H NMR (CDCl₃): δ 0.88 (t, 3H); 1.22, 1.26 (s, 3H each); 1.75(dm, 2H); 1.87-2.10 (m, 3H); 2.23 (m, 1H); 3.54 (m, 2H); 3.76 (s, 3H); 4.52 (dm, 1H, 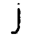 = 8.4, 3.4).

### (2S)-1-(1,2-dioxo-3,3-dimethylpentyl)-2-pyrrolidinecarboxylic acid

A mixture of methyl (2*S*)-1-(1,2-dioxo-3,3-dimethylpentyl)-2-pyrrolidinecarboxylate (2.10 g; 8.23 mmol), 1 N LiOH (15 mL), and methanol (50 mL) was stirred at 0°C for 30 minutes and at room temperature overnight. The mixture was acidified to pH 1 with 1 N HCl, diluted with water, and extracted into 100 mL of methylene chloride. The organic extract was washed with brine and concentrated to deliver 1.73 g (87%) of snow-white solid which did not require further purification. ¹H NMR (CDCl₃): δ 0.87 (t, 3H); 1.22, 1.25 (s, 3H each); 1.77 (dm, 2H); 2.02 (m, 2H); 2.17 (m, 1H); 2.25 (m, 1H); 3.53 (dd, 2H, 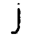 = 10.4, 7.3); 4.55 (dd, 1H, 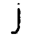 = 8.6, 4.1).

### 2-phenyl-1-ethyl 1-(3,3-dimethyl-1,2-dioxopentyl)-2-piperidinecarbothioate (10)

To a solution of (2S)-1-(1,2-dioxo-3,3-dimethylpentyl)-2-pyrrolidinecarboxylic acid (241 mg; 1.0 mmol) in CH₂Cl₂ (10 mL) was added dicyclohexylcarbo-diimide (226 mg; 1.1 mmol). After stirring the resulting mixture for 5 minutes, the solution was cooled to 0°C and treated with a solution of phenyl mercaptan (138 mg; 1.0 mmol) and 4-dimethylaminopyridine (6 mg) in 5 ml of CH₂Cl₂. The mixture was allowed to warm to room temperature with stirring overnight. The solids were removed by filtration and the filtrate was concentrated in vacuo; the crude residue was purified by flash chromatography (10:1 hexane:EtOAc) to obtain 302 mg (84%) of compound 10 as an oil. ¹H NMR (CDCl₃, 300 MHz) : δ 0.85 (t, 3H, 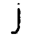 = 7.5); 1.29 (s, 3H); 1.31 (s, 3H); 1.70-2.32 (m, 6H); 2.92 (t, 2H, 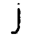 = 7.4); 3.22(t, 2H, 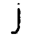 = 7.4); 3.58 (m, 2H); 4.72 (m, 1H); 7.23-7.34 (m, 5H). Analysis calculated for C₂₀H₂₇NO₃S - 0.4 H₂O: C, 65.15; H, 7.60; N, 3.80. Found: C, 65.41; H, 7.49; N, 3.72.

### Example 3

### Synthesis of 2-phenyl-1-ethyl (2S)-1-(3,3-dimethyl-1,2-dioxopentyl)-2-pyrrolidinecarbothioate (9)

### Methyl 1-(1,2-dioxo-2-methoxyethyl)-2-piperidinecarboxylate

A solution of methyl pipecolate hydrochloride (8.50 g; 47.31 mmol) in dry methylene chloride (100 mL) was cooled to 0°C and treated with triethylamine (10.5 g; 103 mmol; 2.1 eq). After stirring the formed slurry under a nitrogen atmosphere for 15 minutes, a solution of methyl oxalyl chloride (8.50 g; 69.4 mmol) in methylene chloride (75 mL) was added dropwise. The resulting mixture was stirred at 0°C for 1,5 hours. After filtering to remove solids, the organic phase was washed with water, dried over MgSO₄ and concentrated. The crude residue was purified on a silica gel column, eluting with 50% ethyl acetate in hexane, to obtain 9.34 g (86%) of the product as a reddish oil. Mixture of *cis-trans* amide rotamers; data for trans rotamer given. ¹H NMR (CDCl₃): δ 1.22-1.45 (m, 2H); 1.67-1.78 (m, 3H); 2.29 (m, 1H); 3.33 (m, 1H); 3.55 (m, 1H); 3.76 (s, 3H); 3.85, 3.87 (s, 3H total); 4.52 (dd, 1H).

### Methyl 1-(1,2-dioxo-3,3-dimethylpentyl)-2-piperidinecarboxylate

A solution of methyl 1-(1,2-dioxo-2-methoxyethyl)-2-piperidinecarboxylate (3.80 g; 16.57 mmol) in 75 mL of tetrahydrofuran (THF) was cooled to -78°C and treated with 20.7 mL of a 1.0 M solution of 1,1-dimethyl-propylmagnesium chloride in THF. After stirring the resulting homogeneous mixture at -78°C for three hours, the mixture was poured into saturated ammonium chloride (100 mL) and extracted into ethyl acetate. The organic phase was washed with water, dried, and concentrated, and the crude material obtained upon removal of the solvent was purified on a silica gel column, eluting with 25% ethyl acetate in hexane, to obtain 3.32 g (74%) of the oxamate as a colorless oil. ¹H NMR (CDCl₃): δ 0.88 (t, 3H); 1.21, 1.25 (s, 3H each); 1.35-1.80 (m, 7H); 2.35 (m, 1H); 3.24 (m, 1H); 3.41 (m, 1H); 3.76 (s, 3H); 5.32 (d, 1H).

### 1-(1,2-dioxo-3,3-dimethylpentyl)-2-piperidinecarboxylic acid

A mixture of methyl 1-(1,2-dioxo-3,3-dimethylpentyl)-2-piperidinecarboxylate (3.30 g; 12.25 mmol), 1 N LiOH (15 mL), and methanol (60 mL) was stirred at 0°C for 30 minutes and at room temperature overnight. The mixture was acidified to pH 1 with 1 N HCl, diluted with water, and extracted into 100 mL of methylene chloride. The organic extract was washed with brine and concentrated to deliver 2.80 g (87%) of snow-white solid which did not require further purification. ¹H NMR (CDCl₃): δ 0.89 (t, 3H); 1.21, 1.24 (s, 3H each); 1.42-1.85 (m, 7H); 2.35 (m, 1H); 3.22 (d, 1H); 3.42(m, 1H); 5.31 (d, 1H).

### 2-phenyl-1-ethyl (2S)-1-(3,3-dimethyl-1,2-dioxopentyl)-2-pyrrolidinecarbothioate (9)

To a solution of 1-(1,2-dioxo-3,3-dimethylpentyl)-2-piperidine-carboxylic acid (255 mg; 1.0 mmol) in CH₂Cl₂ (10 mL) was added dicyclohexylcarbodiimide (226 mg; 1.1 mmol). After stirring the resulting mixture for 5 minutes, the solution was cooled to 0°C and treated with a solution of phenyl mercaptan (138 mg; 1.0 mmol) and 4-dimethylaminopyridine (6 mg) in 5 ml of CH₂Cl₂. The mixture was allowed to warm to room temperature with stirring overnight. The solids were removed by filtration and the filtrate was concentrated in vacuo; the crude residue was purified by flash chromatography (10:1 hexane:EtOAc) to obtain 300 mg (80%) of compound 9 as an oil. ¹H NMR (CDCl₃, 300 MHz): d 0.94 (t, 3H, J = 7.5); 1.27 (s, 3H); 1.30 (s, 3H); 1.34-1.88 (m, 7H); 2.45 (m, 1H); 2.90 (t, 2H, J = 7.7); 3.26 (t, 2H, J = 7.7); 3.27 (m, 1H); 3.38 (m, 1H); 5.34 (m, 1H); 7.24-7.36 (m, 5H). Analysis calculated for C₂₁H₂₉NO₃S: C, 67.17; H, 7.78; N, 3.73. Found: C, 67.02; H, 7.83; N, 3.78.

### Example 4

### Synthesis of 3-phenyl-1-propyl(2S)-1-(3,3-dimethyl-1,2-dioxopentyl)-2-(4-thiazolidine)carboxylate (80)

### 1- (1,2-dioxo-2-methoxyethyl)2-(4-thiazolidine)-carboxylate

A solution of L-thioproline (1.51 g; 11.34 mmol)in 40 mL of dry methylene chloride was cooled to 0°C and treated with 3.3 mL (2.41 g; 23,81 mmol) of triethylamine. After stirring this mixture for 30 minutes, a solution of methyl oxalyl chloride (1.81 g; 14.74 mmol) was added dropwise. The resulting mixture was stirred at 0°C for 1.5 hours, filtered through Celite to remove solids, dried and concentrated. The crude material was purified on a silic gel column, eluting with 10% MeOH in methylene chloride, to obtain 2.0 g of the oxamate as an orange-yellow solid.

### 3-phenyl-1-propyl(2S)-1-(1,2-dioxo-2-methoxyethyl)2-(4-thiazolidine)carboxylate

1-(1,2-dioxo-2-methoxyethyl)2-(4-thiazolidine)-carboxylate (500 mg; 2.25 mmol), 3-phenyl-1-propanol (465 mg; 3.42 mmol), dicyclohexylcarbodiimide (750 mg; 3.65 mmol), 4-dimethylaminopyridine (95 mg; 0.75 mmol) and camphorsulfonic acid (175 mg; 0.75 mmol) in 30 mL of methylene chloride were stirred together overnight. The mixture was filtered through Celite to remove solids and chromatographed (25% ethyl acetate/hexane) to obtain 690 mg of material. ¹H NMR (CDCl₃, 300 MHz) : δ1.92-2.01 (m, 2H); 2.61-2.69 (m, 2H); 3.34 (m, 1H); 4.11-4.25 (m, 2H); 4.73 (m, 1H); 5.34 (m, 1H); 7.12 (m, 3H); 7.23 (m, 2H).

### 3-phenyl-1-propyl(2S)-1-(3,3-dimethyl-1,2-dioxopentyl)-2-(4-thiazolidine)carboxylate (80)

A solution of 3-phenyl-1-propyl (2*S*)-1-(1, 2-dioxo-2-methoxyethyl)2-(4-thiazolidine)carboxylate (670 mg; 1.98 mmol) in tetrahydrofuran (10 mL) was cooled to -78°C and treated with 2.3 mL of a 1.0 M solution of 1,1-dimethylpropylmagnesium chloride in ether. After stirring the mixture for 3 hours, it was poured into saturated ammonium chloride, extracted into ethyl acetate, and the organic phase was washed with water, dried and concentrated. The crude material was purified on a silica gel column, eluting with 25% ethyl acetate in hexane, to obtain 380 mg of the compound of Example 4 as a yellow oil. ¹H NMR (CDCl₃, 300 MHz): d 0.86 (t, 3H); 1.21 (s, 3H); 1.26 (s, 3H); 1.62-1.91 (m, 3H); 2.01 (m, 2H); 2.71 (m, 2H); 3.26-3.33 (m, 2H); 4.19 (m, 2H); 4.58 (m, 1H); 7.19 (m, 3H); 7.30 (m, 2H). Analysis calculated for C₂₀H₂₇NO₄S: C, 63.63: H, 7 23; N, 3.71. Found: C, 64.29; H, 7.39; N, 3.46.

### Example 5

### Synthesis of 3-(3-pyridyl)-1-propyl(2S)-1-(3,3-dimethyl-1,2-dioxopentyl)-2-(4-thiazolidine) carboxylate (81)

The compound of Example 5 was prepared according to the procedure of Example 4, using 3-(3-pyridyl)-1-propanol in the final step, to yield 3-(3-pyridyl)-1-propyl(2*S*)-1-(3,3-dimethyl-1,2-dioxopentyl)-2-(4-thiazolidine)carboxylate. ¹H NMR (CDCl₃, 300 MHz): δ 0.89 (t, 3H, 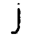 = 7.3); 1.25 (s, 3H); 1.28 (s, 3H); 1.77 (q, 2H, 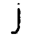 = 7.3); 2.03 (tt, 2H, 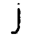 = 6.4, 7.5); 2.72 (t, 2H, 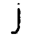 = 7.5); 3.20 (dd, 1H, 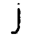 = 4.0, 11.8); 3.23 (dd, 1H, 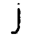 = 7.0, 11.8); 4.23 (t, 2H, 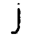 = 6.4); 4.55 (d, 2H, 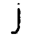 = 8.9); 5.08 (dd, 1H, 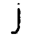 = 4.0, 7.0); 7.24 (m, 1H); 8.48 (m, 2H). Analysis calculated for C₁₉H₂₆N₂O₄S - 0.5 H₂O: C, 58.89; H, 7.02; N, 7.23. Found: C, 58.83; H, 7.05; N, 7.19.

### Example 6

### Synthesis of 3-(3-pyridyl)-1-propyl (2S)-1-(3,3-Dimethyl-1,2-dioxopentyl)-2-pyrrolidinecarboxylate, N-oxide (95)

### Methyl (2S)-1-(1,2-dioxo-2-methoxyethyl)-2-pyrrolidinecarboxylate

A solution of L-proline methyl ester hydrochloride (3.08 g; 18.60 mmol) in dry methylene chloride was cooled to 0°C and treated with triethylamine (3.92 g; 38.74 mmol; 2.1 eq). After stirring the formed slurry under a nitrogen atmosphere for 15 minutes, a solution of methyl oxalyl chloride (3.20 g; 26.12 mmol) in methylene chloride (45 mL) was added dropwise. The resulting mixture was stirred at 0°C for 1.5 hour. After filtering to remove solids, the organic phase was washed with water, dried over MgSO₄ and concentrated. The crude residue was purified on a silica gel column, eluting with 50% ethyl acetate in hexane, to obtain 3.52 g (88%) of the product as a reddish oil. Mixture of cis-trans amide rotamers; data for trans rotamer given. ¹H NMR (CDCl₃): δ 1.93 (dm, 2H); 2.17 (m, 2H); 3.62 (m, 2H); 3.71 (s, 3H); 3.79, 3.84 (s, 3H total); 4.86 (dd, 1H, 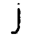 = 8.4, 3.3).

### Methyl(2S)-1-(1,2-dioxo-3,3-dimethylbentyl)-2-pyrrolidinecarboxylate

A solution of methyl (2*S*)-1-(1,2-dioxo-2-methoxyethyl)-2-pyrrolidinecarboxylate (2.35 g; 10.90 mmol) in 30 mL of tetrahydrofuran (THF) was cooled to - 78°C and treated with 14.2 mL of a 1.0 M solution of 1,1-dimethylpropylmagnesium chloride in THF. After stirring the resulting homogeneous mixture at -78°C for three hours, the mixture was poured into saturated ammonium chloride (100 mL) and extracted into ethyl acetate. The organic phase was washed with water, dried, and concentrated, and the crude material obtained upon removal of the solvent was purified on a silica gel column, eluting with 25% ethyl acetate in hexane, to obtain 2.10 g (75%) of the oxamate as a colorless oil. ¹H NMR (CDCl₃): δ 0.88 (t, 3H); 1.22, 1.26 (s, 3H each); 1.75 (dm, 2H); 1.87-2.10 (m, 3H); 2.23 (m, 1H); 3.54 (m, 2H); 3.76 (s, 3H); 4.52 (dm, 1H, 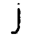 = 8.4, 3.4).

### (2S)-1-(1,2-dioxo-3,3-dimethylpentyl)-2-pyrrolidinecarboxylic acid

A mixture of methyl (2*S*)-1-(1,2-dioxo-3,3-dimethylpentyl-2-pyrrolidine-carboxylate (2.10 g; 8.23 mmol), 1 N LiOH (15 mL), and methanol (50 mL) was stirred at 0°C for 30 minutes and at room temperature overnight. The mixture was acidified to pH 1 with 1 N HCl, diluted with water, and extracted into 100 mL of methylene chloride. The organic extract was washed with brine and concentrated to deliver 1.73 g (87%) of snow-white solid which did not require further purification. ¹H NMR (CDCl₃): d 0.87 (t, 3H); 1.22, 1.25 (s, 3H each); 1.77 (dm, 2H); 2.02 (m, 2H); 2.17 (m, 1H); 2.25 (m, 1H); 3.53 (dd, 2H, 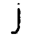 = 10.4, 7.3); 4.55 (dd, 1H, 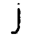 = 8.6, 4.1).

### 3-(3-Pyridyl)-1-propyl(2S)-1-(3,3-dimethyl-1,2-dioxopentyl)-2-pyrrolidinecarboxylate

A mixture of (2*S*)-1-(1,2-dioxo-3,3-dimethylpentyl)-2-pyrrolidinecarboxylic acid (4.58 g; 19 mmol), 3-pyridinepropanol (3.91 g; 28.5 mmol), dicyclohexylcarbodiimide (6.27 g; 30.4 mmol), camphorsulfonic acid (1.47 g; 6.33 mmol) and 4-dimethyl aminopyridine (773 mg; 6.33 mmol) in methylene chloride (100 mL) was stirred overnight under a nitrogen atmosphere. The reaction mixture was filtered through Celite to remove solids and concentrated in vacuo. The crude material was triturated with several portions of ether, and the ether portions were filtered through Celite to remove solids and concentrated in vacuo. The concentrated filtrate was purified on a flash column (gradient elution, 25% ethyl acetate in hexane to pure ethyl acetate) to obtain 5.47 g (80%) of GPI 1046 as a colorless oil (partial hydrate). ¹H NMR (CDCl₃, 300 MHz): δ 0.85 (t, 3H); 1.23, 1.26 (s, 3H each); 1.63-1.89 (m, 2H); 1.90-2.30 (m, 4H); 2.30-2.50 (m, 1H); 2.72 (t, 2H) ; 3.53 (m, 2H); 4.19 (m, 2H); 4.53 (m, 1H); 7.22 (m, 1H); 7.53 (dd, 1H); 8.45. Analysis calculated for C₂₀H₂₈NO₄ - 0.25 H₂O: C, 65.82; H, 7.87; N, 7.68. Found: C, 66.01; H, 7.85; N, 7.64.

### 3-(3-Pyridyl)-1-propyl (2S)-1-(3,3-dimethyl-1,2-dioxopentyl)-2-pyrrolidinecarboxylate, N-oxide (95)

A solution of 3-(3-pyridyl)-1-propyl (2*S*)-1-(3,3-dimethyl-1,2-dioxopentyl)-2-pyrrolidinecarboxylate (190 mg; 0.52 mmol) and m-chloroperbenzoic acid (160 mg of 57%-86% material, 0.53 mmol) was stirred in methylene chloride (20 mL) at room temperature for 3 hours. The reaction mixture was diluted with methylene chloride and washed twice with 1 N NaOH. The organic extract was dried and concentrated, and the crude material was chromatographed, eluting with 10% methanol in ethyl acetate, to obtain 130 mg of the Compound 95 of Example 6. ¹H NMR (CDCl₃, 300 MHz): δ 0.83 (t, 3H); 1.21 (s, 3H); 1.25 (s, 3H); 1.75-2.23 (m, 8H); 2.69 (t, 2H, 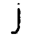 = 7.5); 3.52 (t, 2H, 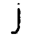 = 6.3); 4.17 (dd, 2H, 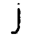 = 6.3); 4.51 (m, 1H); 7.16-7.22 (m, 2H); 8.06-8.11 (m, 2H). Analysis calculated for C₂₀H₂₈N₂O₅ - 0.75 H₂O: C, 61.60; H, 7.63; N, 7.18. Found: C, 61.79; H, 7.58; N, 7.23.

### Example 7

### Synthesis of 3-(3-Pyridyl)-1-propylmercaptyl 2S-1-[(2-methylbutyl)carbamoyl]pyrrolidine-2-carboxylate (101)

### 3-(3-Pyridyl)-1-propylchloride

To a solution of 3-(3-pyridyl)-1-propanol (10 g; 72.4 mmol) in chloroform (100 mL) was added dropwise a solution of thionyl chloride (12.9 g; 108.6 mmol) in chloroform (50 mL). The resulting mixture was refluxed for 1 hour, then poured into ice-cold 50% aqueous potassium hydroxide (150 mL). The layers were separated, and the organic phase was dried, concentrated, and purified on a silica gel column, eluting with 40% ethylacetate in hexane, to obtain 10 g (65%) of the chloride as a clear oil. ¹H NMR (300 MHz, CDCl₃): δ 2.02-2.11 (m, 2H); 2.77 (m, 2H); 3.51 (m, 2H); 7.20 (m, 1H); 7.49 (m, 1H); 8.45 (m, 2H).

### 3-(3-Pyridyl)-1-propylmercaptan

A mixture of 3-(3-pyridyl)-1-propylchloride (3 g; 19.4 mmol) and thiourea (1.48 g; 19.4 mmol) in ethanol (10 mL) was refluxed for 24 hours. Aqueous sodium hydroxide, 15 mL of a 0.75 N solution, was added, and the mixture was refluxed for an additional 2 hours. After cooling to room temperature, the solvent was removed in vacuo. Chromatographic purification of the crude thiol on a silica gel column eluting with 50% ethyl acetate in hexane delivered 1.2 g of 3-(3-Pyridyl)-1-propylmercaptan as a clear liquid. ¹H NMR (300 MHz, CDCl₃): δ 1.34 (m, 1H); 1.90 (m, 2H); 2.52 (m, 2H); 2.71 (m, 2H); 7.81 (m, 1H); 7.47 (m, 1H); 8.42 (m, 2H).

### 3-(3-Pyridyl)-1-propylmercaptyl N-(tert-butyloxycarbonyl)pyrrolidine-2-carboxylate

A mixture of N-(*tert*-butyloxycarbonyl)-(*S*)-proline (3.0 g; 13.9 mmol); 3-(3-Pyridyl)-1-propylmercaptan (3.20 g; 20.9 mmol), dicyclohexylcarbodiimide (4.59 g; 22.24 mmol), camphorsulfonic acid (1.08 g; 4.63 mmol), and 4-dimethylaminopyridine (0.60 g; 4.63 mmol) in dry methylene chloride (100 mL) was stirred overnight. The reaction mixture was diluted with methylene chloride (50 mL) and water (100 mL), and the layers were separated. The organic phase was washed with water (3 x 100 mL), dried over magnesium sulfate, and concentrated, and the crude residue was purified on a silica gel column eluting with ethyl acetate to obtain 4.60 g (95%) of the thioester as a thick oil. ¹H NMR (300 MHz, CDCl₃): δ 1.45 (s, 9H); 1.70-2.05 (m, 5H); 2.32 (m, 2H); 2.71 (t, 2H); 2.85 (m, 2H); 3.50 (m, 2H); 4.18 (m, 1H); 7.24 (m, 1H); 7.51 (m, 1H); 8.48 (m, 2H).

### 3-(3-Pyridyl)-1-propylmercaptyl pyrrolidine-2-carboxylate

A solution of 3-(3-Pyridyl)-1-mercaptyl N-(*tert*-butyloxycarbonyl)pyrrolidine-2-carboxylate (4.60 g; 13.1 mmol) in methylene chloride (60 mL) and trifluoroacetic acid (6 mL) was stirred at room temperature for three hours. Saturated potassium carbonate was added until the pH was basic, and the reaction mixture was extracted with methylene chloride (3x). The combined organic extracts were dried and concentrated to yield 2.36 g (75%) of the free amine as a thick oil. ¹H NMR (300 MHz, CDCl₃): δ 1.87-2.20 (m, 6H); 2.79 (m, 2H); 3.03-3.15 (m, 4H total); 3.84 (m, 1H); 7.32 (m, 1H); 7.60 (m, 1H); 8.57 (m, 2H).

### 3-(3-Pyridyl)-1-propylmercaptyl 2S-1-[(2-methylbutyl)carbamoyl]pyrrolidine-2-carboxylate (101)

A solution of 2-methylbutylamine (113 mg; 1.3 mmol) and triethylamine (132 mg; 1.3 mmol) in methylene chloride (5 mL) was added to a solution of triphosgene (128 mg; 0.43 mmol) in methylene chloride (5 mL). The resulting mixture was refluxed for 1 hour and then cooled to room temperature. 3-(3-Pyridyl)-1-propylmercaptyl pyrrolidine-2-carboxylate (300 mg; 1.3 mmol) in 5 mL of methylene chloride was added and the resulting mixture was stirred for 1 hour and then partitioned between water and a 1:1 mixture of ethyl acetate and hexane. The organic phase was dried, concentrated and purified by column chromatography (50% ethyl acetate/hexane) to obtain 250 mg (55%) of the compound of Example 7 (Compound 101, Table VII) as an oil. ¹H NMR (300 MHz, CDCl₃) : d ¹H NMR (CDCl₃, 300 MHz) : δ 0.89-0.93 (m, 6H); 1.10-1.20 (m, 1H); 1.27 (s, 1H); 1.36-1.60 (m, 2H); 1.72 (s, 2H); 1.97-2.28 (m, 6H); 2.70-2.75 (m, 2H); 2.92-3.54 (m, 6H); 4.45-4.47 (m, 1H); 7.21-7.29 (m, 1H); 7.53-7.56 (dd, 1H); 8.46-8.48 (s, 2H).

### Example 8

### Synthesis of 3-(3-Pyridyl)-1-propyl 2S-1-[(1',1'-Dimethylpropyl)carbamoyl]pyrrolidine-2-carboxylate (102)

Reaction of 3-(3-pyridyl)-1-propylmercaptyl pyrrolidine-2-carboxylate with the isocyanate generated from tert-amylamine and triphosgene, as described for Example 7, provided the compound of Example 8 (Compound 102, Table VII) in 62% yield. ¹H NMR (CDCl₃, 300 MHz): δ 0.83 (t, 3H); 1.27 (s, 6H) ; 1.64-1.71 (m, 2H); 1.91-2.02 (m, 7H); 2.66-2.71 (t, 2H); 2.85 (m, 2H); 3.29-3.42 (m, 2H); 4.11 (br, 1H); 4.37-4.41 (m, 1H).

### Example 9

### Synthesis of 3-(3-pyridyl)-1-propylmercaptyl 2S-1-[(cyclohexyl)thiocarbamoyl]-pyrrolidine-2-carboxylate (107)

A mixture of cyclohexylisothiocyanate (120 mg; 0.9 mmol), 3-(3-pyridyl)-1-propylmercaptyl pyrrolidine-2-carboxylate (200 mg; 0.9 mmol) and triethylamine (90 mg; 0.9 mmol) in 20 mL of methylene chloride was stirred for 1 hour and then partitioned between water and a 1:1 mixture of ethyl acetate and hexane. The organic phase was dried, concentrated and purified by column chromatography (50% ethyl acetate/hexane) to obtain 160 mg (47%) of the compound of Example 9 (Compound 107, Table VII). ¹H NMR (CDCl₃, 300 MHz): δ 1.16-1.40 (m, 6H); 1.50-1.71 (m, 4H); 1.95-2.08 (m, 7H); 2.70-2.75 (t, 2H); 3.03 (m, 2H); 3.40-3.60 (m, 2H); 4.95-4.98 (d, 1H); 5.26-5.29 (d, 1H); 7.17-7.25 (m, 1H).

### Example 10

### Synthesis of 3-(para-Methoxyphenyl)-1-propylmercaptyl(2S)-N-(benzenesulfonyl)pyrrolidine-2-carboxylate (120)

### 3-(p-methoxyphenyl)-1-propylbromide

To a solution of 3-(*p*-methoxyphenyl)-1-propanol (16.6 g; 0.1 mol) in 250 mL of toluene, cooled to 0°C, was added dropwise 26 mL of phosphorus tribromide (0.27 mol). Following completion of the addition, the reaction was stirred at room temperature for 1 hour, then refluxed for an additional hour. The reaction was cooled and poured onto ice, the layers were separated, and the organic phase washed with saturated sodium bicarbonate (3x) and brine (3x). The crude material obtained upon drying and evaporation of the solvent was chromatographed, eluting with 10% EtOAc/hexane, to obtain 14 g (61%) of 3-(*p*-methoxyphenyl)-1-propylbromide.

### 3-(p-Methoxyphenyl)-1-propylmercaptan

A mixture of 3-(*p*-methoxyphenyl)-1-propylbromide (14 g; 61 mmol) and thiourea (5.1 g; 67 mmol) in ethanol (150 mL) was refluxed for 48 hours. Evaporation of the solvent provided a clear glassy compound, which was dissolved in 50 mL of water and treated with 100 mL of 40% aqueous sodium hydroxide. After stirring the resulting mixture for two hours, the product was extracted into ether (3x), and the combined organic extracts were washed with sodium bicarbonate and brine, dried, and concentrated. Chromatographic purification of the crude thiol on a silica gel column eluting with 2% either in hexane delivered 10.2 g of 3-(*p*-methoxyphenyl)- 1-propylmercaptan as a clear liquid. ¹H NMR (300 MHz, CDCl₃): δ 1.34 (t, 1H); 1.88-1.92 (m, 2H); 2.49-2.53 (m, 2H); 2.64-2.69 (m, 2H); 3.77 (s, 3H); 6.80-6.84 (m, 2H); 7.06-7.24 (m, 2H).

### 3-(p-Methoxyphenyl)-1-mercaptyl N-(tert-butyloxycarbonyl)pyrrolidine-2-carboxylate

A mixture of N- (*tert*-butyloxycarbonyl)-(*S*)-proline (2.0 g; 9.29 mmol), 3-(*p*-methoxyphenyl)-1-propylmercaptan (1.86 g; 10.22 mmol), 1-(3-dimethylaminopropyl)-3-ethylcarbodiimide hydrochloride (1.96 g; 10.22 mmol), and 4-dimethylaminopyridine (catalytic) in dry methylene chloride (50 mL) was stirred overnight. The reaction mixture was diluted with methylene chloride (50 mL) and water 100 (mL), and the layers were separated. The organic phase was washed with water (3 x 100 mL), dried over magnesium sulfate, and concentrated to provide 3.05 g of the product (100%) as a thick oil. ¹H NMR (300 MHz, CDCl₃): δ 1.15 (s, 9H) ; 1.84-2.31 (m, 6H); 2.61 (m, 2H); 2.83 (m, 2H); 3.51 (m, 2H) ; 3.75 (s, 3H); 6.79 (d, 2H, 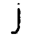 = 8.04); 7.05 (m, 2H).

### 3-(p-Methoxyphenyl)-1-mercaptyl pyrrolidine-2-carboxylate

A solution of 3-(*p*-methoxyphenyl)-mercaptyl N-(*tert*-butyloxycarbonyl)pyrrolidine-2-carboxylate (3.0 g; 8.94 mmol) in methylene chloride (60 mL) and trifluoroacetic acid (6 mL) was stirred at room temperature for three hours. Saturated potassium carbonate was added until the pH was basic, and the reaction mixture was extracted with methylene chloride (3x). The combined organic extracts were dried and concentrated to yield 1.73 g (69%) of the free amine as a thick oil. ¹H NMR (300 MHz, CDCl₃): δ 1.80-2.23 (m, 6H); 2.62 (m, 2H); 2.81 (m, 2H); 3.01 (m, 2H); 3.75 (s, 3H); 3.89(m, 1H); 6.81 (m, 2H); 7.06 (m, 2H).

### 3-(para-Methoxyphenyl)-1-propylmercaptyl (2S)-N-(benzenesulfonyl)pyrrolidine-2-carboxylate (120)

A solution of 3-(*p*-methoxyphenyl)-1-mercaptyl pyrrolidine-2-carboxylate (567 mg; 2.03 mmol) and benzenesulfonyl chloride (358 mg; 2.03 mmol) in methylene chloride (5 mL) was treated with diisopropylethylamine (290 mg; 2.23 mmol) and stirred overnight at room temperature. The reaction mixture was filtered to remove solids and applied directly to a silica gel column, eluting with 25% ethyl acetate in hexane, to obtain 540 mg of Compound 120 (Table VIII) as a clear oil. ¹H NMR (300 MHz, CDCl₃): δ 1.65-1.89 (m, 6H); 2.61 (t, 2H, 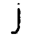 = 7.3); 2.87 (t, 2H, 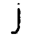 = 7.6); 3.26 (m, 1H); 3.54 (m, 1H); 3.76 (s, 3H); 4.34 (dd, 1H, 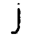 = 2.7, 8.6); 6.79 (d, 2H, 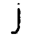 = 8.7); 7.06 (d, 2H, 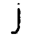 = 8.6); 7.49-7.59 (m, 3H); 7.86 (dd, 2H, 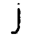 = 1.5, 6.8).

### Example 11

### Synthesis of 3-(para-Methoxyphenyl)-1-propylmercaptyl(2S)-N-(α-toluenesulfonyl)pyrrolidine-2-carboxylate (121)

A solution of 3-(*p*-Methoxyphenyl)-1-mercaptyl pyrrolidine-2-carboxylate (645 mg; 2.30 mmol) and α-toluenesulfonyl chloride (440 mg; 2.30 mmol) in methylene chloride (5 mL) was treated with diisopropylethylamine (330 mg; 2.53 mmol) and stirred overnight at room temperature. Purification as described for Example 10 provided the compound of Example 11 (Compound 121, Table VIII) as a clear oil. ¹H NMR (300 MHz, CDCl₃): δ 1.65-2.25 (m, 8H); 2.65 (t, 2H); 2.89-2.96 (m, 2H); 3.55-3.73 (m, 2H); 3.80 (s, 3H); 4.32 (s, 2H); 4.70-4.81 (m, 1H); 6.83 (d, 2H); 7.09 (d, 2H); 7.14 (m, 3H); 7.26 (m, 2H).

### Example 12

### Synthesis of 3-(para-Methoxyphenyl)-1-propylmercaptyl(2S)-N-(α-toluenesulfonyl)-pyrrolidine-2-carboxylate (122)

A solution of 3-(*p*-methoxyphenyl)-1-mercaptyl pyrrolidine-2-carboxylate (567 mg; 2.30 mmol) and p-toluenesulfonyl chloride (425 mg; 2.23 mmol) in methylene chloride (5 mL) was stirred overnight at room temperature. Purification as described for Example 10 provided the compound of Example 12 (Compound 122, Table VIII) as a clear oil. ¹H NMR (300 MHz, CDCl₃): δ 1.67-1.94 (m, 6H); 2.40 (s, 3H); 2.61 (t, 2H, 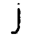 = 7.3); 2.84 (m, 2H, 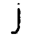 = 7.2); 3.22 (m, 1H); 3.52 (m, 1H); 3.76 (s, 3H); 4.32 (dd, 1H, J-2.9, 8.5); 6.79 (d, 2H, 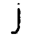 = 6.5); 7.07 (d, 2H, 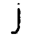 = 6.5); 7.29 (d, 2H, 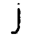 = 6.5); 7.74 (d, 2H, 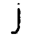 = 6.5).

### Example 13

### Synthesis of 1,5-Diphenyl-3-pentylmercaptyl N-(para-toluenesulfonyl)pipecolate (134)

### 3-Phenyl-1-propanal

Oxalyl chloride (2.90 g; 2.29 mmol) in methylene chloride (50 mL), cooled to -78°C, was treated with dimethylsulfoxide (3.4 mL) in 10 mL of methylene chloride. After stirring for 5 min, 3-phenyl-1-propanol (2.72 g; 20 mmol) in 20 mL of methylene chloride was added, and the resulting mixture was stirred at -78°C for 15 min, treated with 14 mL of triethylamine, stirred an additional 15 min, and poured into 100 mL of water. The layers were separated, the organic phase was dried and concentrated, and the crude residue was purified on a silica gel column, eluting with 10% ethyl acetate in hexane, to obtain 1.27 g (47%) of the aldehyde as a clear oil. ¹H NMR (300 MHz, CDCl₃): δ 2.80 (m, 2H); 2.98 (m, 2H); 7.27 (m, 5H) ; 9.81 (2, 1H).

### 1,5-Diphenyl-3-pentanol

A solution of 2-(bromoethyl)benzene (1.73 g; 9.33 mmol) in diethylether (10 mL) was added to a stirred slurry of magnesium turnings (250 mg; 10.18 mmol) in 5 mL of ether. The reaction was initiated with a heat gun, and after the addition was complete the mixture was heated on an oil bath for 30 min. 3-Phenyl-1-propanal (1.25 g; 9.33 mmol) was added in 10 mL of ether, and reflux was continued for 1 hour. The reaction was cooled and quenched with saturated ammonium chloride, extracted into 2x ethyl acetate, and the combined organic portions were dried and concentrated. Chromatographic purification on a silica gel column (10% ethyl acetate in hexane) delivered 1.42 g(63%) of the diphenyl alcohol. ¹H NMR (300 MHz, CDCl₃): δ 1.84 (m, 4H); 2.61-2.76 (m, 4H); 3.65 (m, 1H); 7.19-7.29 (m, 10H).

### 1,5-Diphenyl-3-bromopentane

To a solution of 1,5-diphenyl-3-pentanol (1.20 g (5 mmol) and carbon tetrabromide (1.67 g; 5 mmol) in methylene chloride (20 mL) was added triphenylphosphine (1.31 g; 5 mmol) portionwise, at 0°C. After stirring at room temperature for 18 hours, the mixture was concentrated, triturated with ether, and the solids removed by filtration. The filtrate was passed through a plug of silica gel, eluting with hexane:methylene chloride, 10:1, to give 1.35 g (90%) of the bromide as an oil which was used without further purification. ¹H NMR (300 MHz, CDCl₃): δ 2.11-2.18 (m, 4H); 2.73 (m, 2H); 2.86 (m, 2H); 3.95 (m, 1H); 7.16-7.30 (m, 10H).

### 1,5-Diphenyl-3-pentylmercaptan

Using the procedure described in Example 10 for the conversion of bromides to thiols, 1,5-diphenyl-3-bromopentane was converted to 1,5-diphenyl-3-pentylmercaptan in 35% overall yield. ¹H NMR (300 MHz, CDCl₃): δ 1.79 (m, 2H); 1.98 (m, 2H); 2.71 (m, 3H); 2.80 (m, 2H); 7.16-7.28 (m, 10H).

### 1,5-Diphenyl-3-pentylmercaptyl N-(tert-butyloxycarbonyl)pyrrolidine-2-carboxylate

A mixture of N-(*tert*-butyloxycarbonyl)-(*S*)-pipecolic acid (2.11 g; 9.29 mmol), 1,5-diphenyl-3-pentylmercaptan (2.58 g; 10.22 mmol), 1-(3-dimethylaminopropyl)-3-ethylcarbodiimide hydrochloride (1.96 g; 10.22 mmol) and 4-dimethylaminopyridine (catalytic) in dry methylene chloride (50 mL) was stirred overnight. the reaction mixture was diluted with methylene chloride (50 mL) and water (100 mL), and the layers were separated. The organic phase was washed with water (3 x 100 mL), dried over magnesium sulfate, and concentrated to provide 870 mg (20%) of the product as a thick oil, which was used without further purification.

### 1,5-Diphenyl-3-pentylmercaptyl pyrrolidine-2-carboxylate

A solution of 1,5-diphenyl-3-pentylmercaptyl N-(*tert*-butyloxycarbonyl)pyrrolidine-2-carboxylate (850 mg; 1.8 mmol) in methylene chloride (10 mL) and trifluoroacetic acid (1 mL) was stirred at room temperature for three hours. Saturated potassium carbonate was added until the pH was basic, and the reaction mixture was extracted with methylene chloride. The combined organic extracts were dried and concentrated to yield 480 mg (72%) of the free amine as a thick oil, which was used without further purification.

### 1,5-Diphenyl-3-pentylmercaptyl N-(para-toluenesulfonyl)pipecolate (134)

1,5-Diphenyl-3-pentylmercaptyl N-(*para*-toluenesulfonyl)pipecolate(18) was prepared from 1,5-diphenyl-3-pentylmercaptyl pyrrolidine-2-carboxylate and para-toluenesulfonyl chloride as described for Example 12, in 65% yield. ¹H NMR (CDCl₃, 300 MHz): δ 0.80 (m, 4H); 1.23-1.97 (m, 5H); 2.15 (d, 1H); 2.61-2.69 (m, 4H); 3.23 (m, 1H); 3.44 (dm, 1H); 4.27 (s, 2H); 4.53 (d, 1H, 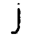 = 4.5); 5.06 (m, 1H); 7.16-7.34 (m, 15H).

### Example 14

### Synthesis of 3-phenyl-1-propyl (2S)-1-(3,3-dimethyl-1,2-dioxopentyl)-2-pyrrolidinecarboxylate (137)

### Methyl (2S)-1-(1,2-dioxo-2-methoxyethyl)-2-pyrrolidinecarboxylate

A solution of L-proline methyl ester hydrochloride (3.08 g; 18.60 mmol) in dry methylene chloride was cooled to 0°C and treated with triethylamine (3.92 g, 38.74 mmol; 2.1 eq). After stirring the formed slurry under a nitrogen atmosphere for 15 min, a solution of methyl oxalyl chloride (3.20 g; 26.12 mmol) in methylene chloride (45 mL) was added dropwise. The resulting mixture was stirred at 0°C for 1.5 hour. After filtering to remove solids, the organic phase was washed with water, dried over MgSO₄ and concentrated. The crude residue was purified on a silica gel column, eluting with 50% ethyl acetate in hexane, to obtain 3.52 g (88%) of the product as a reddish oil. Mixture of cis-trans amide rotamers; data for trans rotamer given. ¹H NMR (CDCl₃): d 1.93 (dm, 2H); 2.17 (m, 2H); 3.62 (m, 2H); 3.71 (s, 3H); 3.79, 3.84 (s, 3H total); 4.86 (dd, 1H, 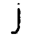 = 8.4, 3.3).

### Methyl (2S)-1-(1,2-dioxo-3,3-dimethylpentyl)-2-pyrrolidinecarboxylate

A solution of methyl (2*S*)-1-(1,2-dioxo-2-methoxyethyl)-2-pyrrolidinecarboxylate (2.35 g; 10.90 mmol) in 30 mL of tetrahydrofuran (THF) was cooled to -78°C and treated with 14.2 mL of a 1.0 M solution of 1,1-dimethylpropylmagnesium chloride in THF. After stirring the resulting homogeneous mixture at -78°C for three hours, the mixture was poured into saturated ammonium chloride (100 mL) and extracted into ethyl acetate. The organic phase was washed with water, dried, and concentrated, and the crude material obtained upon removal of the solvent was purified on a silica gel column, eluting with 25% ethyl acetate in hexane, to obtain 2.10 g (75%) of the oxamate as a colorless oil. ¹H NMR (CDCl₃): d 0.88 (t, 3H) ; 1.22, 1.26 (s, 3H each); 1.75 (dm, 2H); 1.87-2.10 (m, 3H); 2.23 (m, 1H) ; 3.54 (m, 2H) ; 3.76 (s, 3H) ; 4.52 (dm, 1H, 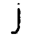 = 8.4, 3.4).

### Synthesis of (2S)-1-(1,2-dioxo-3,3-dimethylpentyl)-2-pyrrolidinecarboxylic acid

A mixture of methyl (2*S*)-1-(1,2-dioxo-3,3-dimethylpentyl)-2-pyrrolidinecarboxylate (2.10 g; 8.23 mmol), 1 N LiOH (15 mL), and methanol (50 mL) was stirred at 0°C for 30 minutes and at room temperature overnight. The mixture was acidified to pH 1 with 1 N HCl, diluted with water, and extracted into 100 mL of methylene chloride. The organic extract was washed with brine and concentrated to deliver 1.73 g (87%) of snow-white solid which did not require further purification. ¹H NMR (CDCl₃): d 0.87 (t, 3H); 1.22, 1.25 (s, 3H each); 1.77 (dm, 2H); 2.02 (m, 2H); 2.17 (m, 1H); 2.25 (m, 1H); 3.53 (dd, 2H, 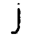 = 10.4, 7.3); 4.55 (dd, 1H, 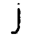 = 8.6, 4.1).

### 3-Phenyl-1-propyl (2S)-1-(3,3-dimethyl-1,2-dioxopentyl)-2-pyrrolidinecarboxylate (137)

A mixture of (2*S*)-1-(1,2-dioxo-3,3-dimethylpentyl)-2-pyrrolidine-carboxylic acid (600 mg; 2.49 mmol), 3-phenyl-1-propanol (508 mg; 3.73 mmol), dicyclohexylcarbodiimide (822 mg; 3.98 mmol), camphorsulfonic acid (190 mg; 0.8 mmol) and 4-dimethylaminopyridine (100 mg; 0.8 mmol) in methylene chloride (20 mL) was stirred overnight under a nitrogen atmosphere. The reaction mixture was filtered through Celite to remove solids and concentrated in vacuo, and the crude material was purified on a flash column (25% ethyl acetate in hexane) to obtain 720 mg (80%) of Example 14 as a colorless oil. ¹H NMR (CDCl₃): d 0.84 (t, 3H); 1.19 (s, 3H); 1.23 (s, 3H); 1.70 (dm, 2H); 1.98 (m, 5H); 2.22 (m, 1H); 2.64 (m, 2H); 3.47 (m, 2H) ; 4.14 (m, 2H); 4.51 (d, 1H); 7.16 (m, 3H); 7.26 (m, 2H).

### Example 15

The method of Example 14 was utilized to prepare the following illustrative compounds.
Compound 138: 3-phenyl-1-prop-2-(E)-enyl (2*S*)-1-(3,3-dimethyl-1,2-dioxopentyl)-2-pyrrolidinecarboxylate, 80%. ¹H NMR (360 Mhz, CDCl₃): d 0.86 (t, 3H); 1.21 (s, 3H); 1.25 (s, 3H); 1.54-2.10 (m, 5H); 2.10-2.37 (m, 1H); 3.52-3.55 (m, 2H); 4.56 (dd, 1H, 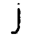 = 3.8, 8.9); 4.78-4.83 (m, 2H); 6.27 (m, 1H); 6.67 (dd, 1H, 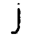 = 15.9); 7.13-7.50 (m; 5H).
Compound 139: 3-(3,4,5-trimethoxyphenyl)-1-propyl (2*S*)-1-(3,3-dimethyl-1,2-dioxopentyl)-2-pyrrolidinecarboxylate, 61%. ¹H NMR (CDCl₃): d 0.84 (t, 3H); 1.15 (s, 3H); 1.24 (s, 3H); 1.71 (dm, 2H); 1.98 (m, 5H); 2.24 (m, 1H); 2.63 (m, 2H); 3.51 (t, 2H); 3.79 (s, 3H); 3.83 (s, 3H); 4.14 (m, 2H); 4.52 (m, 1H); 6.36 (s, 2H).
Compound 140: 3-(3,4,5-trimethoxyphenyl)-1-prop-2-(E)-enyl (2*S*)-1-(3,3-dimethyl-1,2-dioxopentyl)-2-pyrrolidine carboxylate, 66%. ¹H NMR (CDCl₃): d 0.85 (t, 3H); 1.22 (s, 3H); 1.25 (s, 3H); 1.50-2.11 (m, 5H); 2.11-2.40 (m, 1H); 3.55 (m, 2H); 2.85 (s, 3H); 3.88 (s, 6H); 4.56 (dd, 1H); 4.81 (m, 2H); 6.22 (m, 1H); 6.58 (d, 1H, 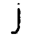 = 16); 6.63 (s, 2H).
Compound 141: 3-(4,5-methylenedioxyphenyl)-1-propyl (2*S*)-1-(3,3-dimethyl-1,2-dioxopentyl)-2-pyrrolidinecarboxylate, 82%. ¹H NMR (360 MHz, CDCl₃): d 0.86 (t, 3H); 1.22 (s, 3H); 1.25 (s, 3H); 1.60-2.10 (m, 5H); 3.36-3.79 (m, 2H); 4.53 (dd, 1H, 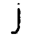 = 3.8, 8.6); 4.61-4.89 (m, 2H) ; 5.96 (s, 2H) ; 6.10 (m, 1H); 6.57 (dd, 1H, 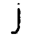 = 6.2, 15.8); 6.75 (d, 1H, 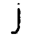 = 8.0); 6.83 (dd, 1H, 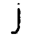 = 1.3, 8.0); 6.93 (s, 1H).
Compound 142: 3-(4,5-methylenedioxyphenyl)-1-prop-2-(E)-enyl (2*S*)-1-(3,3-dimethyl-1,2-dioxopentyl)-2-pyrrolidinecarboxylate, 82%. ¹H NMR (360 MHz, CDCl₃): d 0.86 (t, 3H); 1.22 (s, 3H); 1.25 (s, 3H); 1.60-2.10 (m, 5H); 2.10-2.39 '(m, 1H); 3.36-3.79 (m, 2H); 4.53 (dd, 1H, 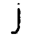 = 3.8, 8.6); 4.61-4.89 (m, 2H); 5.96 (s, 2H); 6.10 (m, 1H); 6.57 (dd, 1H, 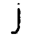 = 6.2, 15.8); 6.75 (d, 1H, 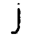 = 8.0); 6.83 (dd, 1H, 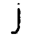 = 1.3, 8.0); 6. 93 (s, 1H).
Compound 144: 3-cyclohexyl-1-prop-2-(E)-enyl (2*S*)-1-(3,3-dimethyl-1,2-dioxopentyl)-2-pyrrolidinecarboxylate, 92%. ¹H NMR (360 MHz, CDCl₃): d 0.86 (t, 3H); 1.13-1.40 (m + 2 singlets, 9H total); 1.50-1.87 (m, 8H); 1.87-2.44 (m, 6H); 3.34-3.82 (m, 2H); 4.40-4.76 (m, 3H); 5.35-5.60 (m, 1H); 5.60-5.82 (dd, 1H, 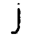 = 6.5, 16).
Compound 145: (1*R*)-1,3-Diphenyl-1-propyl (2*S*)-1-(3,3-dimethyl-1,2-dioxopentyl)-2-pyrrolidinecarboxylate, 90%. ¹H NMR (360 MHz, CDCl₃): d 0.85 (t, 3H); 1.20 (s, 3H); 1.23 (s, 3H); 1.49-2.39 (m, 7H); 2.46-2.86 (m, 2H); 3.25-3.80 (m, 2H); 4.42-4.82 (m, 1H); 5.82 (td, 1H, 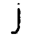 = 1.8, 6.7); 7.05-7.21 (m, 3H) ; 7.21-7.46 (m, 7H).
Compound 146: 3-phenyl-1-propyl (2*S*)-1-(1,2-dioxo-2-[2-furanyl])ethyl-2-pyrrolidinecarboxylate, 99%. ¹H NMR (300 MHz, CDCl₃): d 1.66-2.41 (m, 6H) ; 2.72 (t, 2H, 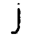 = 7.5); 3.75 (m, 2H); 4.21 (m, 2H); 4.61 (m, 1H); 6.58 (m, 1H); 7.16-7.29 (m, 5H); 7.73 (m, 2H).
Compound 147: 3-phenyl-1-propyl (2*S*)-1-(1,2-dioxo-2-[2-thienyl])ethyl-2-pyrrolidinecarboxylate, 81%. ¹H NMR (300 MHz, CDCl₃): d 1.88-2.41 (m, 6H); 2.72 (dm, 2H); 3.72 (m, 2H); 4.05 (m, 1H); 4.22 (m, 1H); 4.64 (m, 1H); 7.13-7.29 (m, 6H); 7.75 (dm, 1H); 8.05 (m, 1H).
Compound 149: 3-phenyl-1-propyl (2*S*)-1-(1,2-dioxo-2-phenyl)ethyl-2-pyrrolidinecarboxylate, 99%. ¹H NMR (300 MHz, CDCl₃): d 1.97-2.32 (m, 6H); 2.74 (t, 2H, 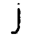 = 7.5) ; 3.57 (m, 2H) ; 4.24 (m, 2H); 4.67 (m, 1H); 6.95-7.28 (m, 5H); 7.51-7.64 (m, 3H); 8.03-8.09 (m, 2H).
Compound 150: 3-(2,5-dimethoxyphenyl)-1-propyl (2*S*)-1-(3,3-dimethyl-1,2-dioxopentyl)-2-pyrrolidinecarboxylate, 99%. ¹H NMR (300 MHz, CDCl₃): d 0.87 (t, 3H); 1.22 (s, 3H); 1.26 (s, 3H); 1.69 (m, 2H); 1.96 (m, 5H); 2.24 (m, 1H); 2.68 (m, 2H); 3.55 (m, 2H); 3.75 (s, 3H); 3.77 (s, 3H); 4.17 (m, 2H); 4.53 (d, 1H); 6. 72 (m, 3H).
Compound 151: 3- (2, 5-dimethoxyphenyl) -1-prop-2-(E)-enyl (2*S*)-1-(3,3-dimethyl-1,2-dioxopentyl)-2-pyrrolidine-carboxylate, 99%. ¹H NMR (300 MHz, CDCl₃) : d 0.87 (t, 3H); 1.22 (s, 3H) ; 1.26 (s, 3H); 1.67 (m, 2H); 1.78 (m, 1H); 2.07 (m, 2H); 2.26 (m, 1H); 3.52 (m, 2H); 3.78 (s, 3H); 3.80 (s, 3H); 4.54 (m, 1H); 4.81 (m, 2H); 6.29 (dt, 1H, 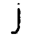 = 15.9); 6.98 (s, 1H).
Compound 152: 2-(3,4,5-trimethoxyphenyl)-1-ethyl (2*S*)-1-(3,3-dimethyl-1,2-dioxopentyl)-2-pyrrolidinecarboxylate, 97%. ¹H NMR (300 MHz, CDCl₃): d 0.84 (t, 3H); 1.15 (s, 3H); 1.24 (s, 3H); 1.71 (dm, 2H); 1.98 (m, 5H); 2.24 (m, 1H); 2.63 (m, 2H); 3.51 (t, 2H); 3.79 (s, 3H); 3.83 (s, 3H); 4.14 (m, 2H); 4.52 (m, 1H); 6.36 (s, 2H).
Compound 153: 3-(3-Pyridyl)-1-propyl (2*S*)-1-(3,3-dimethyl-1,2-dioxopentyl)-2-pyrrolidinecarboxylate, 80%. ¹H NMR (CDCl₃, 300 MHz): d 0.85 (t, 3H) ; 1.23, 1.26 (s, 3H each); 1.63-1.89 (m, 2H); 1.90-2.30 (m, 4H); 2.30-2.50 (m, 1H); 2.72 (t, 2H); 3.53 (m, 2H); 4.19 (m, 2H); 4.53 (m, 1H); 7.22 (m, 1H); 7.53 (dd, 1H); 8.45.
Compound 154: 3-(2-Pyridyl)-1-propyl (2*S*)-1-(3,3-dimethyl-1,2-dioxopentyl)-2-pyrrolidinecarboxylate, 88%. ¹H NMR (CDCl₃, 300 MHz): d 0.84 (t, 3H); 1.22, 1.27 (s, 3H each); 1.68.-2.32 (m, 8H); 2.88 (t, 2H, 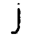 = 7.5); 3.52 (m, 2H); 4.20 (m, 2H); 4.51 (m, 1H); 7.09-7.19 (m, 2H); 7.59 (m, 1H); 8.53 (d, 1H, 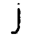 = 4.9).
Compound 155: 3-(4-Pyridyl)-1-propyl (2*S*)-1-(3,3-dimethyl-1,2-dioxopentyl)-2-pyrrolidinecarboxylate, 91%. ¹H NMR (CDCl₃, 300 MHz): d 6.92-6.80 (m, 4H); 6.28 (m, 1H); 5.25 (d, 1H, 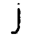 = 5.7); 4.12 (m, 1H); 4.08 (s, 3H); 3.79 (s, 3H); 3.30 (m, 2H); 2.33 (m, 1H); 1.85-1.22 (m, 7H); 1.25 (s, 3H); 1.23 (s, 3H); 0.89 (t, 3H, 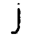 = 7.5).
Compound 156: 3-phenyl-1-propyl (2*S*)-1-(2-cyclohexyl-1,2-dioxoethyl)-2-pyrrolidinecarboxylate, 91%. ¹H NMR (CDCl₃, 300 MHz): d 1.09-1.33 (m, 5H); 1.62-2.33 (m, 12H); 2.69 (t, 2H, 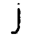 = 7.5); 3.15 (dm, 1H); 3.68 (m, 2H); 4.16 (m, 2H); 4.53, 4.84 (d, 1H total); 7.19 (m, 3H); 7.29 (m, 2H).
Compound 157: 3-phenyl-1-propyl (2*S*)-1-(2-*tert*-butyl-1,2-dioxoethyl)-2-pyrrolidinecarboxylate, 92%. ¹H NMR (CDCl₃, 300 MHz): d 1.29 (s, 9H); 1.94-2.03 (m, 5H); 2.21 (m, 1H); 2.69 (m, 2H); 3.50-3.52 (m, 2H); 4.16 (m, 2H); 4.53 (m, 1H); 7.19 (m, 3H); 7.30 (m, 2H).
Compound 158: 3-phenyl-1-propyl (2*S*)-1-(2-cyclohexylethyl-1,2-dioxoethyl)-2-pyrrolidinecarboxylate, 97%. ¹H NMR (CDCl₃, 300 MHz): d 0.88 (m, 2H); 1.16 (m, 4H) ; 1.43-1.51 (m, 2H); 1.67 (m, 5H); 1.94-2.01 (m, 6H); 2.66-2.87 (m, 4H); 3.62-3.77 (m, 2H); 4.15 (m, 2H); 4.86 (m, 1H); 7.17-7.32 (m, 5H).
Compound 159: 3-(3-pyridyl)-1-propyl (2*S*)-1-(2-cyclohexylethyl-1,2-dioxoethyl)-2-pyrrolidinecarboxylate, 70%. ¹H NMR (CDCl₃, 300 MHz): d 0.87 (m, 2H); 1.16 (m, 4H); 1.49 (m, 2H); 1.68 (m, 4H); 1.95-2.32 (m, 7H); 2.71 (m, 2H); 2.85 (m, 2H); 3.63-3.78 (m, 2H); 4.19 (m, 2H); 5.30 (m, 1H); 7.23 (m, 1H); 7.53 (m, 1H); 8.46 (m, 2H).
Compound 160: 3-(3-pyridyl)-1-propyl (2*S*)-1-(2-*tert*-butyl-1,2-dioxoethyl)-2-pyrrolidinecarboxylate, 83%. ¹H NMR (CDCl₃, 300 MHz): d 1.29 (s, 9H); 1.95-2.04 (m, 5H); 2.31 (m, 1H); 2.72 (t, 2H, 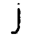 = 7.5); 3.52 (m, 2H); 4.18 (m, 2H); 4.52 (m, 1H); 7.19-7.25 (m, 1H); 7.53 (m, 1H); 8.46 (m, 2H).
Compound 161: 3,3-diphenyl-1-propyl (2*S*)-1-(3,3-dimethyl-1,2-dioxopentyl)-2-pyrrolidinecarboxylate, 99%. ¹H NMR (CDCl₃, 300 MHz): d 0.85 (t, 3H); 1.21, 1.26 (s, 3H each); 1.68-2.04 (m, 5H); 2.31 (m, 1H); 2.40 (m, 2H); 3.51 (m, 2H); 4.08 (m, 3H); 4.52 (m, 1H); 7.18-7.31 (m, 10H).
Compound 162: 3-(3-pyridyl)-1-propyl (2*S*)-1-(2-cyclohexyl-1,2-dioxoethyl)-2-pyrrolidinecarboxylate, 88%. ¹H NMR (CDCl₃, 300 MHz): d 1.24-1.28 (m, 5H); 1.88-2.35 (m, 11H); 2.72 (t, 2H, 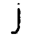 = 7.5); 3.00-3.33 (dm, 1H); 3.69 (m, 2H); 4.19 (m, 2H) ; 4.55 (m, 1H); 7.20-7.24 (m, 1H); 7.53 (m, 1H); 8.47 (m, 2H).
Compound 163: 3-(3-Pyridyl)-1-propyl (2*S*)-N-([2-thienyl] glyoxyl)pyrrolidinecarboxylate, 49%. ¹H NMR (CDCl₃, 300 MHz): d 1.81-2.39 (m, 6H) ; 2.72 (dm, 2H); 3.73 (m, 2H); 4.21 (m, 2H); 4.95 (m, 1H); 7.19 (m, 2H); 7.61 (m, 1H); 7.80 (d, 1H); 8.04 (d, 1H); 8.46 (m, 2H).
Compound 164: 3,3-Diphenyl-1-propyl (2*S*)-1-(3,3-dimethyl-1,2-dioxobutyl)-2-pyrrolidinecarboxylate, 99%. ¹H NMR (CDCl₃, 300 MHz): d 1.27 (s, 9H) ; 1.96 (m, 2H); 2.44 (m, 4H); 3.49 (m, 1H); 3.64 (m, 1H); 4.08 (m, 4H) ; 4.53 (dd, 1H); 7.24 (m, 10H).
Compound 165: 3,3-Diphenyl-1-propyl (2*S*)-1-cyclohexyl glyoxyl-2-pyrrolidinecarboxylate, 91%. ¹H NMR (CDCl₃, 300 MHz): d 1.32 (m, 6H); 1.54-2.41 (m, 10H); 3.20 (dm, 1H) ; 3.69 (m, 2H); 4.12 (m, 4H) ; 4.52 (d, 1H); 7.28 (m, 10H).
Compound 166: 3,3-Diphenyl-1-propyl (2*S*)-1-(2-thienyl) glyoxyl-2-pyrrolidinecarboxylate, 75%. ¹H NMR (CDCl₃, 300 MHz): d 2.04 (m, 3H); 2.26 (m, 2H); 2.48 (m, 1H); 3.70 (m, 2H); 3.82-4.18 (m, 3H total); 4.64 (m, 1H); 7.25 (m, 11H); 7.76 (dd, 1H); 8.03 (m, 1H).

### Example 16

General procedure for the synthesis of acrylic esters, exemplified for methyl (3,3,5-trimethoxy)-*trans*-cinnamate.

A solution of 3,4,5-trimethoxybenzaldehyde (5.0 g; 25.48 mmol) and methyl (triphenylphosphoranylidene) acetate (10.0 g; 29.91 mmol) in tetrahydrofuran (250 mL) was refluxed overnight. After cooling, the reaction mixture was diluted with 200 mL of ethyl acetate and washed with 2 x 200 mL of water, dried, and concentrated in vacuo. The crude residue was chromatographed on a silica gel column, eluting with 25% ethyl acetate in hexane, to obtain 5.63 g (88%) of the cinnamate as a white crystalline solid. ¹H NMR (300 Mhz; CDCl₃): d 3.78 (s, 3H) ; 3.85 (s, 6H); 6.32 (d, 1H, 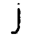 = 16) ; 6.72 (s, 2H); 7.59 (d, 1H, 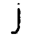 = 16).

### Example 17

General procedure for the synthesis of saturated alcohols from acrylic esters, exemplified for (3,4,5-trimethoxy) phenylpropanol.

A solution of methyl (3,3,5-trimethoxy)-*trans*-cinnamate (1.81 g; 7.17 mmol) in tetrahydrofuran (30 mL) was added in a dropwise manner to a solution of lithium aluminum hydride (14 mmol) in THF (35 mL), with stirring and under an argon atmosphere. After the addition was complete, the mixture was heated to 75°C for 4 hours. After cooling, it was quenched by the careful addition of 15 mL of 2 N NaOH followed by 50 mL of water. The resulting mixture was filtered through Celite to remove solids, and the filter cake was washed with ethyl acetate. The combined organic fractions were washed with water, dried, concentrated in vacuo, and purified on a silica gel column, eluting with ethyl acetate to obtain 0.86 g (53%) of the alcohol as a clear oil. ¹H NMR (300 Mhz; CDCl₃): d 1.23 (br, 1H); 1.87 (m, 2H); 2.61 (t, 2H, 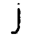 = 7.1); 3.66 (t, 2H); 3.80 (s, 3H); 3.83 (s, 6H); 6.40 (s, 2H).

### Example 18

General procedure for the synthesis of *trans*-allylic alcohols from acrylic esters, exemplified for (3,4,5-trimethoxy)phenylprop-2-(E)-enol.

A solution of methyl (3,3,5-trimethoxy)-*trans*-cinnamate (1.35 g; 5.35 mmol) in toluene (25 mL) was cooled to -10°C and treated with a solution of diisobutylaluminum hydride in toluene (11.25 mL of a 1.0 M solution; 11.25 mmol). The reaction mixture was stirred for 3 hours at 0°C and then quenched with 3 mL of methanol followed by 1 N HCl until the pH was 1. The reaction mixture was extracted into ethyl acetate and the organic phase was washed with water, dried and concentrated. Purification on a silica gel column eluting with 25% ethyl acetate in hexane furnished 0.96 g (80%) of a thick oil. ¹H NMR (360 Mhz; CDCl₃): d 3.85 (s, 3H); 3.87 (s, 6H); 4.32 (d, 2H, 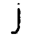 = 5.6); 6.29 (dt, 1H, 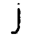 = 15.8, 5.7), 6.54 (d, 1H, 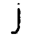 = 15.8); 6.61 (s, 2H).

### Example 19

### In Vivo Hair Generation Tests With C57 Black 6 Mice

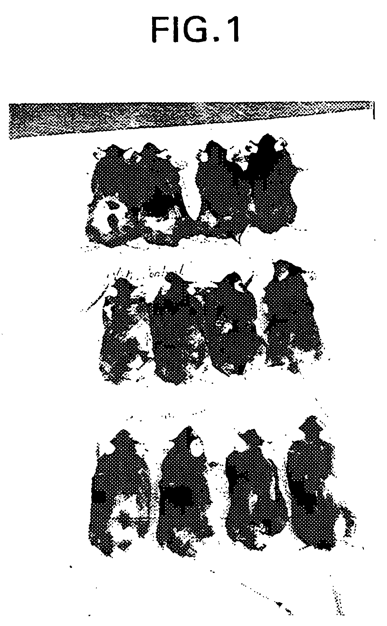

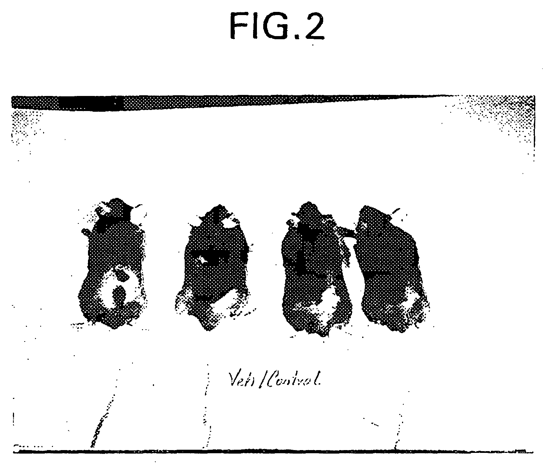

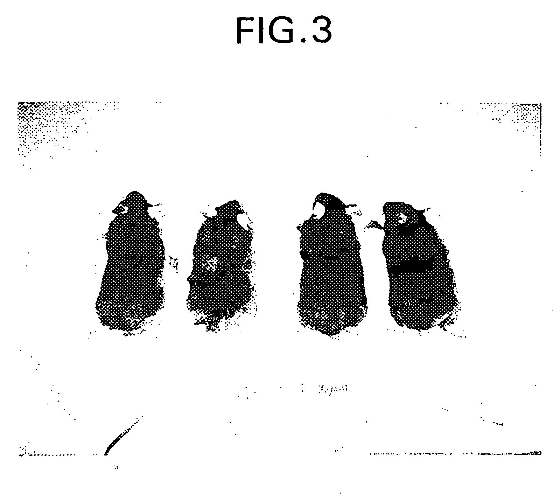

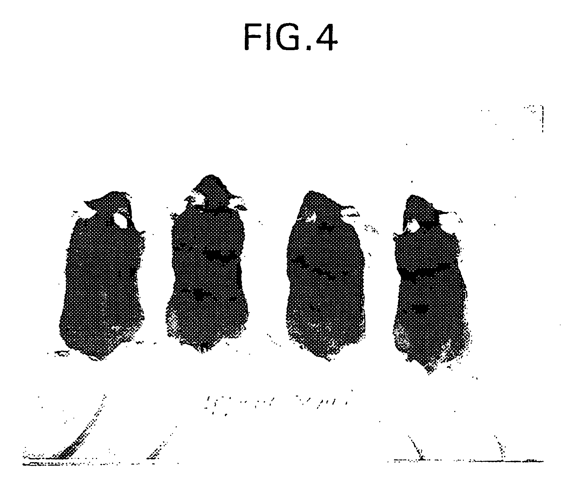

Experiment A: C57 black 6 mice were used to demonstrate the hair revitalizing properties of a non-immunosuppressive neuroimmunophilin FKBP ligand, GPI 1046. Referring now to FIGS. 1 and 2 of the drawings, C57 black 6 mice, approximately 7 weeks old, had an area of about 2 inches by 2 inches on their hindquarters shaved to remove all existing hair. Care was taken not to nick or cause abrasion to the underlaying dermal layers. The animals were in anagen growth phase, as indicated by the pinkish color of the skin. Referring now to FIGS. 2, 3 and 4, four animals per group were treated by topical administration with 20% propylene glycol vehicle (FIG. 2), 10 µM GPI 1046 (FIG. 3) or 30 µM GPI 1046 (FIG. 4) dissolved in the vehicle. The animals were treated with vehicle or GPI 1046 every 48 hours (3 applications total over the course of 5 days) and the hair growth was allowed to proceed for 6 weeks. Hair growth was quantitated by the percent of shaved area covered by new hair growth during this time period.

FIG. 2 shows that animals treated with vehicle exhibited only a small amount of hair growth in patches or tufts, with less than 3% of the shaved area covered with new growth. In contrast, FIG. 3 shows that animals treated with 10 µM GPI 1046 exhibited dramatic hair growth, covering greater than 90% of the shaved area in all animals. Further, FIG. 4 shows that mice treated with 30 µM GPI 1046 exhibited essentially complete hair regrowth and their shaved areas were indistinguishable from unshaven C57 black 6 mice.

Experiment B: C57 Black 6 mice were used to demonstrate the hair revitalizing properties of various low molecular weight, small molecule, non-immunosuppressive neuroimmunophilin FKBP ligands. C57 Black 6 mice, 55 to 75 days old, had an area of about 2 inches by 2 inches on their hindquarters shaved to remove all existing hair. Care was taken not to nick or cause abrasion to the underlying dermal layers. The animals were in anagen growth phase when shaved. Five animals per group were treated by topical administration with a vehicle, FK506, or one of the low molecular weight, small molecule, non-immunosuppressive neuroimmunophilin FKBP ligands (GPI 1605, GPI 1046, GPI 1312, GPI 1572, GPI 1389, GPI 1511, or GPI 1234) to the shaved area. The animals were treated three times per week, and hair growth was evaluated 14 days after initiation of treatment. Hair growth was quantitated by the percent of shaved area covered by new hair growth, as scored by a blinded observer, on a scale of 0 (no growth) to 5 (complete hair regrowth in shaved area).

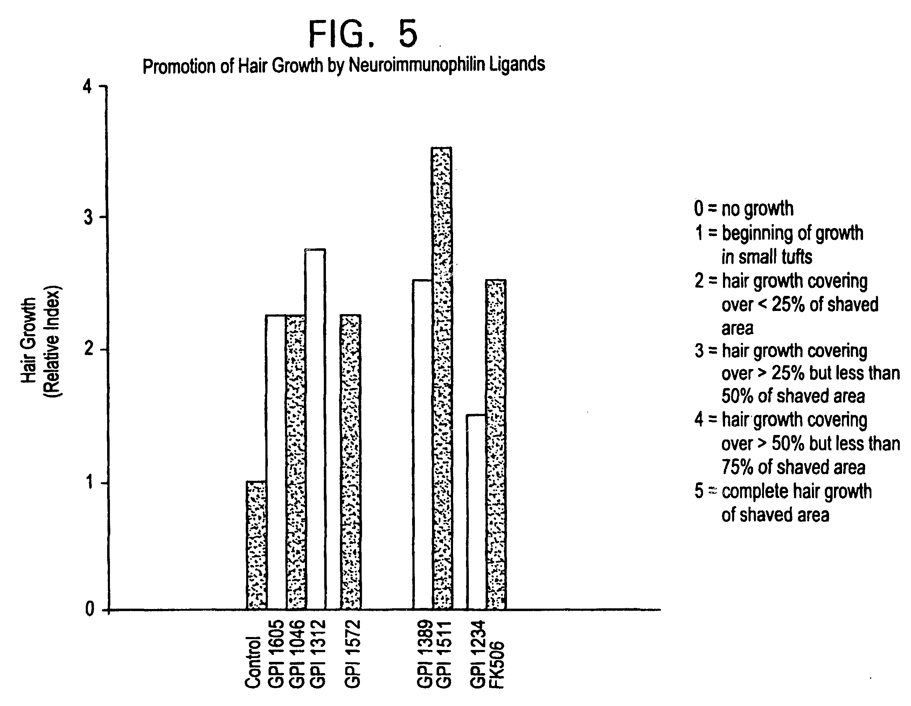

FIG. 5 shows that after 14 days, the animals treated with vehicle exhibited the beginning of hair growth in small tufts. By contrast, most of the animals treated with the low molecular weight, small molecule, non-immunosuppressive neuroimmunophilin FKBP ligands exhibited dramatic hair growth.

### Example 20

A lotion comprising the following composition may be prepared.

| | (%) |
|---|---|
| 95% Ethanol | 80.0 |
| a non-immunosuppressive neuroimmunophilin FKBP ligand | 10.0 |
| α-Tocopherol acetate | 0.01 |
| Ethylene oxide (40 mole) adducts of hardened castor oil | 0.5 |
| purified water | 9.0 |
| perfume and dye | q.s. |

Into 95% ethanol are added a non-immunosuppressive neuroimmunophilin FKBP ligand, α-tocopherol acetate, ethylene oxide (40 mole) adducts of hardened castor oil, perfume and a dye. The resulting mixture is stirred and dissolved, and purified water is added to the mixture to obtain a transparent liquid lotion.

5 ml of the lotion may be applied once or twice per day to a site having marked baldness or alopecia.

### Example 21

A lotion comprising the following composition shown may be prepared.

| | (%) |
|---|---|
| 95% Ethanol | 80.0 |
| a non-immunosuppressive neuroimmunophilin FKBP ligand | 0.005 |
| Hinokitol | 0.01 |
| Ethylene oxide (40 mole) adducts of hardened castor oil | 0.5 |
| Purified water | 19.0 |
| Perfume and dye | q.s. |

Into 95% ethanol are added a non-immunosuppressive neuroimmunophilin FKBP ligand, hinokitol, ethylene oxide (40 mole) adducts of hardened castor oil, perfume, and a dye. The resulting mixture is stirred, and purified water is added to the mixture to obtain a transparent liquid lotion.

The lotion may be applied by spraying once to 4 times per day to a site having marked baldness or alopecia.

### Example 22

An emulsion may be prepared from A phase and B phase having the following compositions.

| **(A phase)** | (%) |
|---|---|
| Whale wax | 0.5 |
| Cetanol | 2.0 |
| Petrolatum | 5.0 |
| Squalane | 10.0 |
| Polyoxyethylene (10 mole) monostearate | 2.0 |
| Sorbitan monooleate | 1.0 |
| a non-immunosuppressive neuroimmunophilin FKBP ligand | 0.01 |

| **(B phase)** | (%) |
|---|---|
| Glycerine | 10.0 |
| Purified water | 69.0 |
| Perfume, dye, and preservative | q.s. |

The A phase and the B phase are respectively heated and melted and maintained at 80°c. Both phases are then mixed and cooled under stirring to normal temperature to obtain an emulsion.

The emulsion may be applied by spraying once to four times per day to a site having marked baldness or alopecia.

### Example 23

A cream may be prepared from A phase and B phase having the following compositions.

| **(A Phase)** | (%) |
|---|---|
| Fluid paraffin | 5.0 |
| Cetostearyl alcohol | 5.5 |
| Petrolatum | 5.5 |
| Glycerine monostearate | 33.0 |
| Polyoxyethylene (20 mole) 2-octyldodecyl ether | 3.0 |
| Propylparaben | 0.3 |

| **(B Phase)** | (%) |
|---|---|
| a non-immunosuppressive neuroimmunophilin FKBP ligand | 0.8 |
| Glycerine | 7.0 |
| Dipropylene glycol | 20.0 |
| Polyethylene glycol 4000 | 5.0 |
| Sodium Hexametaphosphate | 0.005 |
| Purified water | 44.895 |

The A phase is heated and melted, and maintained at 70°c. The B phase is added into the A phase and the mixture is stirred to obtain an emulsion. The emulsion is then cooled to obtain a cream.

The cream may be applied once to 4 times per day to a site having marked baldness or alopecia.

### Example 24

A liquid comprising the following composition may be prepared.

| | (%) |
|---|---|
| Polyoxyethylene butyl ether | 20.0 |
| Ethanol | 50.0 |
| a non-immunosuppressive neuroimmunophilin FKBP ligand | 0.001 |
| Propylene glycol | 5.0 |
| Polyoxyethylene hardened castor oil derivative (ethylene oxide 80 mole adducts) | 0.4 |
| Perfume | q.s. |
| Purified water | q.s. |

Into ethanol are added polyoxypropylene butyl ether, propylene glycol, polyoxyethylene hardened castor oil, a non-immunosuppressive neuroimmunophilin FKBP ligand, and perfume. The resulting mixture is stirred, and purified water is added to the mixture to obtain a liquid.

The liquid may be applied once to 4 times per day to a site having marked baldness or alopecia.

### Example 25

A shampoo comprising the following composition may be prepared.

| | (%) |
|---|---|
| Sodium laurylsulfate | 5.0 |
| Triethanolamine laurylsulfate | 5.0 |
| Betaine lauryldimethylaminoacetate | 6.0 |
| Ethylene glycol distearate | 2.0 |
| Polyethylene glycol | 5.0 |
| a non-immunosuppressive neuroimmunophilin FKBP ligand | 5.0 |
| Ethanol | 2.0 |
| Perfume | 0.3 |
| Purified water | 69.7 |

Into 69.7 of purified water are added 5.0 g of sodium laurylsulfate, 5.0 g of triethanolamine laurylsulfate, 6.0 g of betaine lauryldimethylaminoacetate. Then a mixture obtained by adding 5.0 g of a non-immunosuppressive neuroimmunophilin FKBP ligand, 5.0 g of polyethylene glycol, and 2.0 g of ethylene glycol distearate to 2.0 g of ethanol, followed by stirring, and 0.3 g of perfume are successively added. The resulting mixture is heated and subsequently cooled to obtain a shampoo.

The shampoo may be used on the scalp once or twice per day.

### Example 26

A patient is suffering from alopecia senilis. A non-immunosuppressive neuroimmunophilin FKBP ligand or a pharmaceutical composition comprising the same may be administered to the patient. Increased hair growth is expected to occur following treatment.

### Example 27

A patient is suffering from male pattern alopecia. A non-immunosuppressive neuroimmunophilin FKBP ligand or a pharmaceutical composition comprising the same may be administered to the patient. Increased hair growth is expected to occur following treatment.

### Example 28

A patient is suffering from alopecia areata. A non-immunosuppressive neuroimmunophilin FKBP ligand or a pharmaceutical composition comprising the same may be administered to the patient. Increased hair growth is expected to occur following treatment.

### Example 29

A patient is suffering from hair loss caused by skin lesions. A non-immunosuppressive neuroimmunophilin FKBP ligand or a pharmaceutical composition comprising the same may be administered to the patient. Increased hair growth is expected to occur following treatment.

### Example 30

A patient is suffering from hair loss caused by tumors. A non-immunosuppressive neuroimmunophilin FKBP ligand or a pharmaceutical composition comprising the same may be administered to the patient. Increased hair growth is expected to occur following treatment.

### Example 31

A patient is suffering from hair loss caused by a systematic disorder, such as a nutritional disorder or an internal secretion disorder. A non-immunosuppressive neuroimmunophilin FKBP ligand or a pharmaceutical composition comprising the same may be administered to the patient. Increased hair growth is expected to occur following treatment.

### Example 32

A patient is suffering from hair loss caused by chemotherapy. A non-immunosuppressive neuroimmunophilin FKBP ligand or a pharmaceutical composition comprising the same may be administered to the patient. Increased hair growth is expected to occur following treatment.

### Example 33

A patient is suffering from hair loss caused by radiation. A non-immunosuppressive neuroimmunophilin FKBP ligand or a pharmaceutical composition comprising the same may be administered to the patient. Increased hair growth is expected to occur following treatment.

The invention being thus described, it will be obvious that the same may be varied in many ways. Such variations are not to be regarded as a departure from the spirit and scope of the invention and all such modifications are intended to be included within the scope of the following claims.

## Claims

1. Use of a non-immunosuppressive neuroimmunophilin FKBP ligand in the manufacture of a medicament for treating alopecia or promoting hair growth.

2. The use of Claim 1, wherein the neuroimmunophilin FKBP is FKBP-12.

## Patentansprüche

1. Verwendung eines nicht-immunosuppressiven Neuroimmunophilin-FKBP-Liganden bei der Herstellung eines Medikaments zur Behandlung von Alopezie oder zur Förderung von Haarwuchs.

2. Die Verwendung von Anspruch 1, wobei das Neuroimmunophilin-FKBP FKBP-12 ist.

## Revendications

1. Usage d'un ligand FKBP neuroimmunophilin non-immunosuppressif dans la fabrication d'un médicament pour traiter l'alopécie ou stimuler la croissance des cheveux.

2. L'usage de la revendication 1, le FKBP neuroimmunophilin étant FKBP-12.
